# NOUVEAU FASCICULE DE BREVET EUROPEEN

(11) **EP 1 366 176 B2**
(45) Date de publication et mention de la décision concernant l'opposition: **19.10.2011**
(45) Mention de la délivrance du brevet: 15.08.2007
(21) Numéro de dépôt: 02722336.1
(22) Date de dépôt: 11.03.2002
(51) Int. Cl.: C12N 15/70, C12N 15/31, C12N 1/21, C12N 9/10, C12N 15/54

(54) **GENES SYNTHETIQUES ET PLASMIDES BACTERIENS DEPOURVUS DE CPG**
CPG-FREIE SYNTHETISCHE GENE UND BAKTERIELLE PLASMIDE
SYNTHETIC GENES AND BACTERIAL PLASMIDS DEVOID OF CPG

(30) Priorité: 09.03.2001 FR 0103274
(43) Date de publication de la demande: 03.12.2003
(73) Titulaire: Cayla, 31405 Toulouse Cedex 4 (FR)
(72) Inventeur: DROCOURT, Daniel, F-31650 Saint-Orens de Gameville (FR); REYNES, Jean-Paul, F-31750 Escalquens (FR); TIRABY, Gérard, F-31400 Toulouse (FR)
(74) Mandataire: Bourout, Gaëlle
(86) Numéro de dépôt international: PCT/FR2002/000862
(87) Numéro de publication internationale: WO 2002/072846

(56) Documents cités:
- WO-A-00/14262
- WO-A-01/40478
- WO-A1-97/10343
- WO-A2-00/14262
- WO-A2-01/40478
- WO-A2-01/75092
- WO-A2-02/072846
- FR-A- 103 274
- ISABELLE HENRY ET AL.: "LagoZ et LagZ, deux gènes appauvris en dinucléotides CpG dérivés du gène LacZ pour l'étude des contrôles épigénétiques" C.R. ACAD. SCI. PARIS, vol. 322, 1999, pages 1061-1070, XP002185406
- THOMAS R. SKOPEK ET AL.: "Synthesis of a lacI gene analogue with reduced CpG content" MUTATION RESEARCH, vol. 349, 1996, pages 163-172, XP001041417
- THOMAS SKOPEK ET AL.: "Effect of target gene CpG content on spontaneous mutation in transgenic mice" MUTATION RESEARCH, vol. 400, 1998, pages 77-88, XP001030795
- Base de donnés EMBL; Numéro d'accès L37432; 10 Avril 1996 TIRABY G. ET AL.:"New suicide genes and new associations of pyrimidine nucleobase and nucleoside analogs with new suicide genes for a gene therapy of acquired diseases." XP002185408
- PRITAM SENGUPTA ET AL.: "Methylation in the initiation region of the first exon supresses collagen pro-alpha2(I) gene transcription" BIOCHIMICA ET BIOPHYSICA ACTA, vol. 1443, 1998, pages 75-89, XP002185407
- RICHARD S. HALE ET AL.: "Codon optimization of the gene encoding a domain from human type 1 neurofibromin protein results in a threefold improvement in expression level in Escherichia coli" PROTEIN EXPRESSION AND PURIFICATION, vol. 12, mars 1998 (1998-03), pages 185-188, XP001030791
- MARTIN HUG ET AL.: "Transcriptional repression by methylation: cooperativity between a CpG cluster in the promoter and remote CpG-rich regions" FEBS LETTERS, vol. 379, 1996, pages 251-254, XP002125021
- IGOR LEVCHENKO ET AL.: "Initiator protein pi can bind independently to two domains of the gamma origin core of plasmid R6K: the direct repeats and the A+T-rich segment" NUCLEIC ACIDS RESEARCH, vol. 24, no. 10, 1996, pages 1936-1942, XP002185270
- ARTHUR M. KRIEG ET AL.: 'CpG motifs in bacterial DNA trigger direct B-cell activation' NATURE vol. 06.04.95, pages 546 - 549
- ARTHUR M. KRIEG: 'Direct immunologic activities of CpG DNA and implications for gene therapy' THE JOURNAL OF GENE MEDICINE vol. 1999, pages 56 - 63
- HIROAKI HEMMI ET AL.: 'A toll-like receptor recognizes bacterial DNA' NATURE vol. 408, pages 740 - 745
- HIROAKI HEMMI ET AL.: 'Erratum' NATURE vol. 409, page 646
- NELSON S. YEW ET AL.: 'Contribution of plasmid DNA to inflammation in the lung after administration of cationic lipid:pDNA complexes' HUMAN GENE THERAPY vol. 10, pages 223 - 234
- NELSON S. YEW ET AL.: 'Reduced inflammatory response to plasmid DNA vectors by elimination and inhibition of immunostimulatory CpG motifs' MOLECULAR THERAPY vol. 1, pages 255 - 262
- NELSON S. YEW ET AL.: 'High and sustained transgene expression in vivo from plasmid vectors containing a hybrid ubiquitin promoter' MOLECULAR THERAPY vol. 4, pages 75 - 82
- NELSON S. YEW ET AL.: 'Erratum' MOLECULAR THERAPY vol. 4, page 280
- AVIGDOR SHAFFERMAN ET AL.: 'Structural properties of the beta origin of replication of plasmid R6K' THE JOURNAL OF BIOLOGICAL CHEMISTRY vol. 258, pages 4083 - 4090
- ROBERT C. TAIT ET AL.: 'A comparison of the origin of replication of pSa with R6K' MOL. GEN. GENET. vol. 192, pages 32 - 38
- B.R. PALMER ET AL.: 'The dam and dcm strains of Escherichia coli - a review' GENE vol. 143, pages 1 - 12
- S. ALLAMANE ET AL.: 'Bacterial DNA methylation and gene transfer efficiency' BIOCHEMICAL AND BIOPHYSICAL RESEARCH COMMUNICATIONS vol. 276, pages 1261 - 1264
- ISABELLE HENRY ET AL.: 'LagoZ et LagZ, deux gènes appauvris en dinucléotidesCpG dérivés du gène LacZ pour l'étude des contrôles épigénétiques' C.R. ACAD. SCHI PARIS vol. 322, pages 1061 - 1070, XP002185406
- THOMAS R. SKOPEK ET AL.: 'Synthesis of a lacl gene analogue with reduced CpG content' MUTATION RESEARCH vol. 349, pages 163 - 172, XP001041417
- RICHARD S. HALE ET AL.: 'Codon optimization of the gene encoding a domain from human type 1 neurofibrominprotein results in a threefold imporvement in expression level in Escherichia coli' PROTEIN EXPRESSION AND PURIFICATION vol. 12, pages 185 - 188, XP001030791
- IGOR LEVCHENKO ET AL.: 'Initiator protein pi can bind independently to two domains of the gamma origin core of plamid R6K: the direct repeats and the A+T-rich segment' NUCLEIC ACIDS RESEARCH vol. 24, no. 10, pages 1936 - 1942, XP002185270
- 'Abstracts 240 and 247' PEDIATRIC PUMONOLOGY, SUPPLEMENT 19
- BENJAMIN LEWIN: 'Genes VII' OXFORD UNIVERSITY PRESS 2000 pages 244 - 246
- BIOLOGICAL ABSTRACTS, Philadelphia, PA, US; BLAST RESULT: PCDNA3ZEO V. SEQ ID NO:11
- BIOLOGICAL ABSTRACTS, Philadelphia, PA, US; CLONING VECTOR PCDNA3ZEO 16.08.1995
- MAUREEN S. MAY ET AL.: 'Analysis of bacteriophage deoxyribonucleic acid sequences methylated by host- and R-factor-controlled enzymes' JOURNAL OF BACTERIOLOGY vol. 123, no. 2, pages 768 - 770
- 'NEW ENGLAND BIOLABS', 1988-1989 pages 143 - 145
- DAVID M. STALKER ET AL.: 'Plasmid R6K DNA replication. I. Complete nucleotide sequence of an autonomously replicating segment' JOURNAL OF MOLECULAR BIOLOGY vol. 161, pages 33 - 43
- DATABASE EMBL DATABASE 'Extract V00320'
- DATABASE SEQUENCE 'Alignments between V00320 and SEQ ID Nos: 12 and 13 of the patent'
- DATABASE GENBANK ACCESSION AF488695: 'Cloning vector pCpG-LacZdeltaCpG, complete (27.02.2002)'
- DATABASE GENBANK ACCESSION AF488695; VERSION AF488965.1: 'Cloning vector pCpG-LacZdeltaCpG, complete (01.09.2003)'
- DATABASE GENBANK ACCESSION AF488695: 'Cloning vector pCpG-LacZdeltaCpG, complete sequence (01.09.2003)'
- DATABASE GENBANK ACCESSION AF488696: 'Cloning vector pCpG-Neo deltaCpG (submitted 27.02.2002)'
- DATABASE GENBANK ACCESSION AF488696; VERSION AF488966.1.: 'Cloning vector pCpG-Neo deltaCpG, complete sequence (01.09.2003)'
- DATABASE GENBANK ACCESSION AF48869: 'Cloning vector pCpG-Neo deltaCpG, complete sequence (01.09.2003)'
- WILLIAM W. METCALF ET AL.: 'Use of the rep technique for allele replacement to construct new Escherichia coli hosts for maintenance of R6Ky origin plasmids at different copy numbers' GENE vol. 138, pages 1 - 7
- MANUBU INUZUKA ET AL.: 'A single amino acid alteration in the initiation protein is responsible for the DNA overproduction phenotype of copy number mutants of plasmid R6K' THE EMBO JOURNAL vol. 4, pages 2301 - 2307
- ROBERTO KOLTER ET AL.: 'Trans-complementation-dependent replication of a low molecular weight origin fragment from plasmid R6K' CELL vol. 15, pages 1199 - 1208
- ELLIOT EHRICH ET AL.: 'A family of cosmid vectors with multi-copy R6K replication origin' GENE vol. 57, pages 229 - 237
- DATABASE CLUSTALW V1.83: 'multiple sequence alignment'
- F. SOUBRIER ET AL.: 'pCOR: a new design for plasmid vectors for nonviral gene therapy' GENE THERAPY vol. 6, pages 1482 - 1488
- CALVIN B. HARLEY ET AL.: 'Analysis of E. coli promoter sequences' NUCLEIC ACIDS RESEARCH vol. 15, pages 2343 - 2361
- SHLOMIT LISSER ET AL.: 'Compilation of E. coli mRNA promoter sequences' NUCLEIC ACIDS RESEARCH vol. 21, pages 1507 - 1516
- DATABASE ALIGNMENT OF SEQ ID NOS: 12 AND 13
- MICHAEL J. MCEACHERN ET AL.: 'Mutations in direct repeat sequences and in a conserved sequence adjacent to the repeats result in a defective replication origin in plasmid R6K' PROC. NATL. ACAD. SCHI. USA vol. 82, pages 1480 - 1484
- DATABASE PMOD-LACZ 'A plasmid containing a synthetic b-galactosidase gene'
- DATABASE PMOD-LACZNLS 'A plasmid containing a synthetic b-galactosidase gene with a nuclear localization signal'
- DATABASE DESCRIPTION 'pMOD-Zeo v02'

## Description

### DOMAINE DE L'INVENTION

La présente demande est relative à des gènes synthétiques et à des plasmides entièrement dépourvus de CpG.

### ARRIERE-PLAN TECHNOLOGIQUE

Les plasmides sont des éléments génétiques essentiellement trouvés chez les bactéries, formés par une molécule d'acide désoxyribonucléique, le plus souvent circulaire dont la réplication est autonome et indépendante de celle de l'ADN génomique. Les plasmides naturels isolés à partir d'une très large variété de bactéries sont capables d'accomplir plusieurs fonctions cellulaires. La première, vitale pour tous les plasmides, est celle responsable de leur réplication, généralement réalisée en synchronie avec la réplication de l'ADN génomique et la division cellulaire. Outre la région nécessaire à la réplication du plasmide, tous les plasmides naturels portent des gènes qui codent pour des protéines dont la fonction reste le plus souvent non connue par absence d'investigation spécifique sur ces gènes. Le nombre de gènes présents sur un plasmide détermine la taille de ce plasmide, les plus petits plasmides naturels ne renfermant que deux à trois gènes. Les propriétés des plasmides ont très tôt attiré les chercheurs pour en faire des véhicules de transport et d'expression de gènes dans les cellules procaryotes comme eucaryotes. Les progrès très rapides observés dans les domaines de la biologie moléculaire des acides nucléiques et des protéines, ces deux dernières décennies, sont en partie attribuables à l'exploitation de plasmides recombinés construits à partir de fragments d'ADN naturel d'origine plasmidique ou autres ADN cellulaires, et même synthétisés chimiquement. Les quatre bases adénine (A), guanine (G), cytosine (C) et thymine (T) qui constituent l'acide désoxyribonucléique (ADN) se répartissent en 16 configurations dinucléotidiques à savoir CG, GC, TA, AT, CC, GG, TT, AA, TG, CA, AG, CT, AC, GT, GA et TC. L'analyse de la distribution qualitative des dinucléotides de l'ADN des milliers de plasmides dont les séquences sont connues révèle que les 16 dinucléotides sont toujours présents dans tous les plasmides naturels ou construits en laboratoire. Cependant l'analyse de la distribution quantitative des dinucléotides des plasmides montrent de fortes disparités qui sont, en partie seulement, fonction du pourcentage de chacune des quatre bases de l'ADN. En effet, la comparaison des fréquences observées de chacun des dinucléotides à celles des fréquences calculées sur la base d'une association aléatoire entre deux bases pour un plasmide donné peut mettre en évidence des écarts importants pour plusieurs dinucléotides dans le sens d'une sur-représentation ou au contraire une sous-représentation (Campbell A., Mrazek J. et Karlin S. (1999) Proc Natl Acad Sci U S A 96, 9184-9). Les écarts constatés dans la distribution de certains dinucléotides, pas toujours les mêmes, de plasmides naturels isolés de bactéries d'espèces phylogéniquement éloignées ont été expliqués par des différences de spécificité dans les mécanismes de réparation, recombinaison et réplication agissant sur les ADN cellulaires.

Les transferts de gènes *in vitro* dans des cellules en cultures et *in vivo* chez divers animaux sont des pratiques en grand développement dans le but d'une part de mieux connaître le fonctionnement cellulaire, et d'autre part afin d'appliquer ces techniques aux thérapies cellulaires et géniques. Aucun des vecteurs viraux et des vecteurs plasmidiques parmi la panoplie de vecteurs disponibles pour le transfert de gènes chez les animaux, n'a pris un avantage décisif sur les autres car chacun présente des avantages mais aussi des inconvénients. Il est cependant une application où l'ADN plasmidique nu ou complexé avec diverses substances pour faciliter le transport de l'ADN vers le noyau fait l'objet d'une intense activité de recherche, à savoir celui de l'ADN vaccinant. Le principe de l'ADN vaccinant repose sur les réponses immunitaires constatées chez des animaux de laboratoire traités par injection intramusculaire, intradermique ou inhalation avec de l'ADN plasmidique codant pour un peptide antigénique. Il est maintenant bien établi qu'une première conséquence de l'introduction d'un ADN plasmidique issu de la bactérie *E.coli* dans l'organisme d'un animal expérimental par les voies intraveineuse et musculaire est la production rapide de diverses cytokines par des cellules de garde du système immunitaire (Krieg A. M. et Kline J. N. (2000) Immunopharmacology 48, 303-305). Cette réponse est extrêmement spécifique de l'ADN bactérien puisque de l'ADN extrait de cellules animales ne provoque pas une telle induction de cytokines dans les mêmes conditions. Les mécanismes cellulaires impliqués dans cette réponse immunitaire sont loin d'être parfaitement connus. Cependant nous savons que la reconnaissance discriminatoire entre l'ADN bactérien et l'ADN d'origine animale se fait au niveau de différences structurales portant sur la méthylation de certaines cytosines de la molécule. En effet, l'ADN de mammifères est naturellement méthylé au niveau de la cytosine de tous les dinucléotides CG (écrit par la suite CpG) à l'exception de zones courtes de haute densité en CpG appelées îlots CpG présentes dans des régions fonctionnelles au niveau de certains promoteurs. L'ADN extrait d'*E.coli* ne présente pas ce type de méthylation par absence de l'activité enzymatique capable d'accomplir cette modification chez cette bactérie. Il est néanmoins possible de méthyler les CpGs de l'ADN plasmidique extrait d'*E.coli* en tube à essai par une enzyme appropriée. Dans ces conditions l'ADN méthylé *in vitro* perd une grande partie de son activité immunostimulante par rapport à l'ADN non méthylé témoin. La *souche E. coli K12* dont sont issues la quasi totalité des souches mutantes utilisées pour la production d'ADN plasmidiques renferme une activité enzymatique (DNA méthylase dcm (Palmer B. R. et Marinus M. G. (1994) Gene 143, 1-12) conduisant à la méthylation de cytosine se trouvant dans le contexte nucléique CC(A/T)GG. Tous les vecteurs plasmidiques de transfert de gène renferment cette séquence en nombre variable et de ce fait leur molécule ADN contient des cytosines méthylées qui ne se retrouvent pas dans l'ADN de mammifères. Cette forme de méthylation spécifique à *E.coli* introduit ainsi une autre différence au niveau des méthylations des cytosines entre l'ADN bactérien et celui des mammifères qui pourrait contribuer au pouvoir immunostimulant de l'ADN plasmidique.

La fréquence des CpG des ADN des primates et rongeurs est globalement très inférieure à celle attendue sur la base de la fréquence des cytosines et des guanines. Le déficit en CpG est dépendant pour un fragment d'ADN donné du rôle biologique de ce fragment, les régions intergéniques ne renferment qu'un cinquième de la fréquence attendue alors que les exons ont un déficit moins marqué et à l'autre extrême quelques promoteurs renfermant un ilôt CpG de grande taille présentent un pourcentage de CpG proche de celui attendu. L'analyse des données du séquençage des ADNc et des chromosomes humains révèle néammoins de larges hétérogénéités dans la fréquence de CpG pour des régions promotrices et des ADNc. Cette observation est illustrée par l'ADNc du gène humain codant pour l'interleukine 2 qui ne possède qu'un seul CpG. De même une partie du promoteur de ce gène renfermant la boîte TATA ne contient pas de CpG mais par contre la partie amont riche en sites de reconnaissance de facteurs de transcription renferme des CpG. Les régions en 3' des gènes formées par les 3' UTR (régions non traduites) et les séquences de polyadénylation et fin de transcription sont plutôt pauvres en CpG. Il n'est pas inhabituel de trouver chez l'homme des régions immédiatement en aval des gènes dépourvues de CpG. Cependant les données du séquençage humain disponible en fin de l'an 2000 n'ont pas permis de mettre en évidence une seule unité transcriptionnelle formée par les régions promotrices de la transcription, un gène avec ou sans intron et la région de polyadénylation qui soit entièrement dépourvue de CpG. La situation des CpG chez *E.coli* est toute autre que celle des cellules animales puisque la fréquence des CpG dans l'ADN génomique de cette bactérie est en léger excès par rapport à la fréquence calculée. Il en est de même pour les CpGs des plasmides naturels isolés à partir de souches hospitalières d*'E.coli.* Les plasmides recombinés résultant de manipulations génétiques, utilisés pour le transfert de gènes, présentent des variations dans leurs nombres de CpG qui dépendent de l'origine des fragments insérés dans le vecteur. Une analyse des séquences de plusieurs dizaines de plasmides recombinés *d'E.coli* pris de façon aléatoire dans la banque de données GenBank montrent que les plasmides les plus pauvres en CpG ont tout au plus un déficit de 50 % du nombre de leurs CpG.

Des essais de réduction de la teneur en CpG ont été menés pour le gène lac, mais n'ont pas abouti à la synthèse de gène fonctionnels entièrement dépourvus de CpG (Henry et al. 1999 C.R. ACAD. SCI. PARIS, vol. 322, page 1061-1070; Skopek et al. 1996 Mutation Research, vol. 349, page 163-172).

La présente invention fournit quant à elle des produits et méthodes pour synthétiser un ADN plasmidique chez *E.coli* qui est complètement dépourvu de CpG et dont les cytosines placées dans le contexte CC(A/T)GG ne sont pas méthylées. A la connaissance de la Demanderesse, il s'agit de la première description de tels produits qui présentent une telle structure tout en ayant conservé leur fonction.

### DESCRIPTION DETAILLEE DE L'INVENTION

La présente invention fournit des moyens permettant de produire des plasmides qui sont fonctionnels chez un organisme procaryote tel que *Escherichia coli,* et qui sont pourtant entièrement dépourvus de CpG. Elle fournit plus particulièrement des moyens permettant de produite des plasmides qui sont entièrement dépourvus de CpG et qui sont en outre exempts de méthylation au niveau de la cytosine dans le contexte nucléique CC(A/T)GG.
La présente demande est ainsi relative à des méthodes telles que décrites dans la revendication 1 pour la production de tels plasmides, ainsi qu'aux éléments constitutifs de ces plasmides, à savoir des gènes dépourvus de CpG qui peuvent être exprimés chez *E. coli,* des promoteurs dépourvus de CpG adaptés à l'expression desdits gènes, et des origines de réplication dépourvues de CpG adaptés à la transformation bactérienne desdits plasmides. La présente demande vise également les applications biotechnologiques et médicales de ces produits. Chacun de ces produits présente la caractéristique particulière d'être entièrement dépourvu de CpG, tout en ayant conservé sa fonctionnalité chez un procaryote tel que *E. coli.*
La présente demande décrit également une souche *E. coli* spécialement adaptée à la production des plasmides selon l'invention, cette souche présentant la caractéristique particulière de permettre la réplication stable de ces plasmides et du matériel génétique qu'ils transportent, sans altérer leur fonction, et sans pour autant induire de méthylation au niveau des sites CC(A/T)GG (souche comprenant un gène *dcm* inactivé).
Un des concepts communs liant les différents aspects de l'invention est donc de permettre de produire des plasmides qui sont entièrement dépourvus de CpG et qui ont malgré tout conservé leurs propriétés fonctionnelles chez un procaryote tel que *E. coli.*
A la connaissance de la Demanderesse, il s'agit de la première description de tels moyens.

La présente demande vise ainsi une méthode pour la production d'un plasmide qui est vecteur d'au moins un gène, et qui est entièrement dépourvu de CpG, caractérisée en ce que l'on construit un plasmide en assemblant par ligation enzymatique des fragments d'ADN tous dépourvus de CpG correspondant à une origine de réplication du plasmide et aux éléments constituant une unité transcriptionnelle pour ledit au moins un gène, et en ce que l'on transfère ce plasmide dans une souche d'*Escherichia coli* exprimant la protéine *pi* pour la réplication du plasmide et caractérisée en ce que l'origine de réplication du plasmide est l'origine R6K gamma modifiée par élimination des CpG..

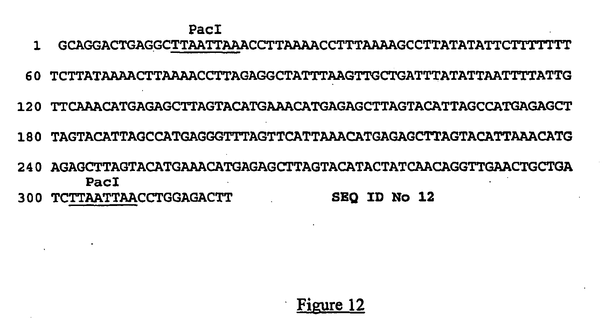

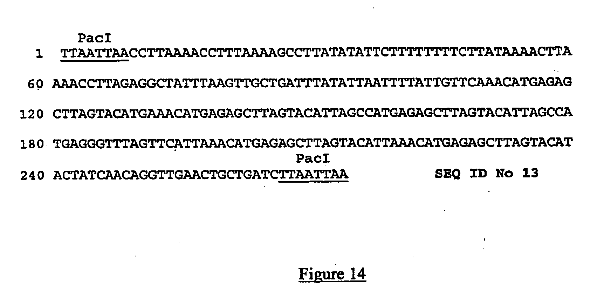

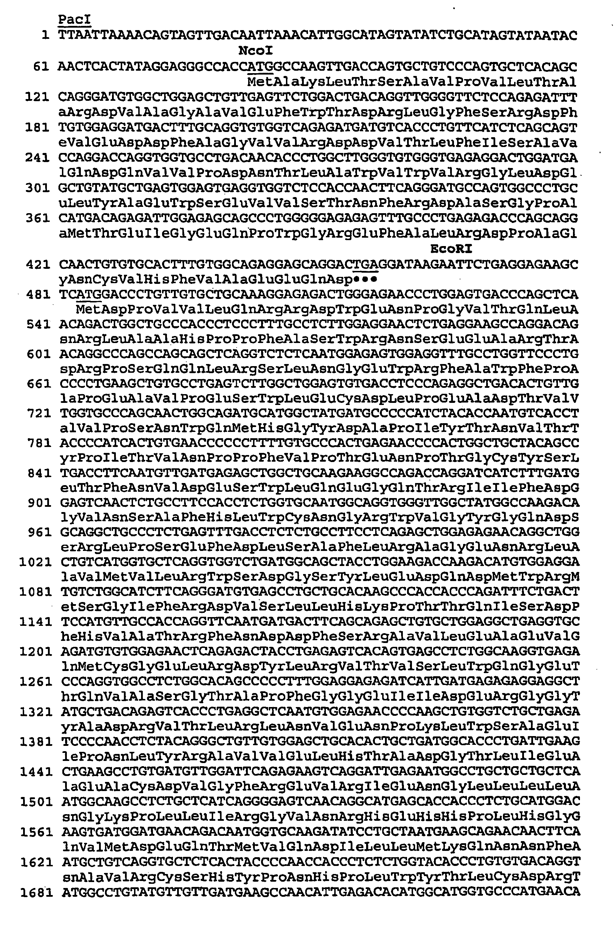

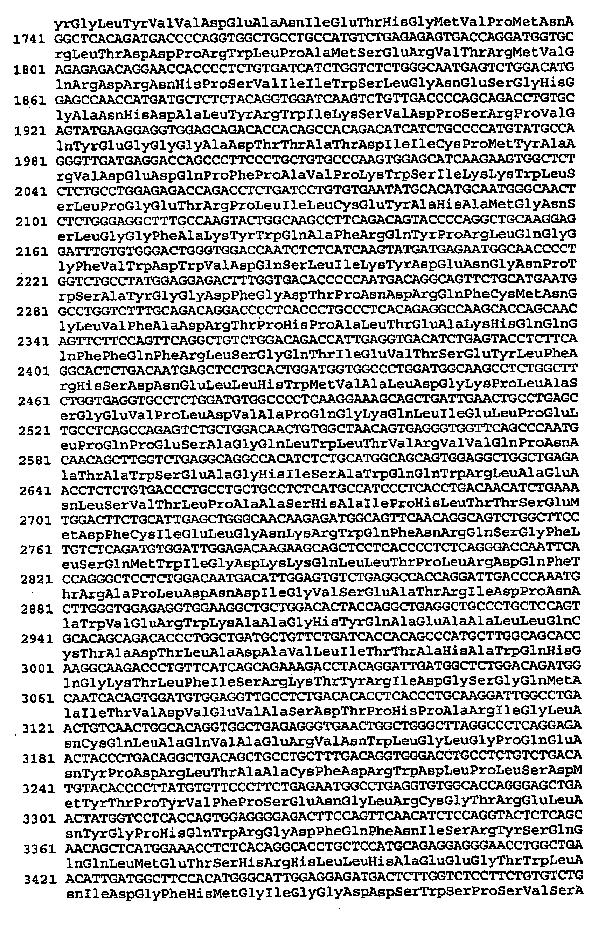

Les plasmides isolés à partir de souches sauvages de bactéries accomplissent généralement trois fonctions en rapport avec la réplication, à savoir initiation de la réplication de l'ADN, contrôle de la réplication et maintenance stable du plasmide au cours des divisions successives. Les plasmides construits en laboratoire ne présentent pas toujours l'ensemble de ces fonctions. Le nombre de copies des plasmides est par exemple bien souvent augmenté par rapport au plasmide parent dénotant que des éléments du contrôle de la réplication ont été modifiés. Le plasmide R6K renferme trois origines de réplication *alpha, gamma* et *beta,* liées sur un même fragment d'ADN (Filutowicz M. et Rakowski S. A. (1998) Gene 223, 195-204). Chacune des origines est activée par la protéine d'initiation *pi* spécifique de R6K, codée par le gène R6K *pir .* Les trois origines ont besoin, pour être fonctionnelles, d'une séquence de 277 pb, connue par l'homme du métier sous le nom de core (coeur), située au centre du fragment portant les trois origines, et aussi d'un fragment unique supplémentaire positionné en *cis* c'est-à-dire présent sur la même molécule d'ADN. Lorsque les séquences des origines *alpha* et *beta* sont délétées, l'origine *gamma* restante permet la duplication autonome du plasmide à la condition que le gène *pir* soit présent en *cis* sur le plasmide ou en *trans* sur le chromosome de la bactérie. Les inventeurs ont fait le choix de s'intéresser plus particulièrement à la plus petite des trois origines, à savoir l'origine *gamma,* qui présente l'avantage de contenir tous les éléments nécessaires pour une réplication contrôlée du plasmide, à savoir le core et une séquence adjacente activatrice. Le core est formé par une séquence de fixation de la protéine *pi* répétée 7 fois et d'une séquence riche en AT. La région activatrice renferme des sites de fixation de plusieurs protéines cellulaires de la bactérie requis pour un maintien stable du plasmide. Le nombre de copies des plasmides renfermant la seule origine *gamma* dépend de la protéine *pi,* des formes mutantes de *pi* conduisant à une forte augmentation du nombre de copies du plasmide ont été isolées et caractérisées. Comme présenté plus en détail dans les exemples ci-dessous, les inventeurs ont réussi à construire à partir de l'origine de réplication du plasmide R6K gamma des origines de réplication qui ne présentent plus aucun CpG, tout en ayant conservé leur fonctionnalité intacte. Il peut être noté que le choix particulier de R6K gamma comme matériel de départ, à savoir le choix d'un réplicon de petite taille qui ne présente qu'un nombre réduit de CpG, est un choix particulièrement pertinent, dans la mesure où lorsque l'on part d'autres plasmides tels que ceux de la série pUC, on ne parvient pas à obtenir de plasmides sans CpG qui restent fonctionnels : toutes les tentatives menées par les inventeurs pour reconstituer par voie chimique la séquence minimale pUC en remplaçant les cytosines des CpG par une guanine ou une adénine ont abouti à des fragment d'ADN ayant perdu toute activité fonctionnelle de réplication. Les plasmides qui comprennent une origine de réplication sans CpG selon l'invention ont quant à eux conservé leur capacité à se répliquer de manière stable à l'intérieur d'une cellule procaryote telle que *E. coli,* et *E. coli* K12 en particulier, dans la mesure où bien entendu on leur fournit la protéine pi nécessaire à l'activation de la réplication (*pir* sauvage ou *pir* muté tel que *pir*116 en *cis* ou en *trans*). Une origine de réplication pour plasmide selon l'invention est caractérisée en ce que sa séquence correspond à celle de l'origine de réplication R6K gamma dans laquelle chaque G des CpG de la région répétée du core a été remplacé par un A, un C ou un T, ou chaque C des CpG a été remplacé par un G, un A ou un T. Ont ainsi été obtenues différentes origines de réplication sans CpG, qui de manière surprenante, étaient toujours capables d'assurer les fonctions d'origine de réplication de plasmides chez E. coli, et qui plus est, étaient capables d'assurer ces fonctions pour des gènes et des unités transcriptionnelles qui eux-mêmes étaient dépourvus de CpG. Les exemples ci-dessous en donnent quelques illustrations (cf. origines R6K gamma M2A, R6K gamma M2C, R6K gamma M2T en exemples 7-10). La présente demande vise plus particulièrement toute origine de réplication dont la séquence comprend la séquence SEQ ID N°12 ou la séquence SEQ ID N°13 (Figures 12 et 14). Il a également été mis en évidence que la séquence de fixation de la protéine pi peut ne pas être répétée 7 fois, comme cela est observé chez l'origine R6K gamma standard avec CpG, mais qu'on en peut limiter le nombre à 5 ou 6, sans pour autant altérer les fonctions de l'origine de réplication. La présente demande vise ainsi toute origine de réplication selon l'invention comme ci-dessus définie, qui ne comprendrait que 5 ou 6 répétitions de la séquence de fixation de la protéine pi.
Grâce à ces origines de réplication fonctionnelles sans CpG, les inventeurs ont pu construire différents plasmides, qui, de manière remarquable, ont conservé leurs fonctions de vecteurs de transfection.

La création de plasmides d'*E.coli* dépourvus de CpG demande obligatoirement de disposer de gènes fonctionnels (exprimables chez un procaryote tel que *E. coli*) qui ne renferment pas de CpG. Ainsi, la sélection des bactéries transformées par un ADN plasmidique recombiné fait intervenir un gène dont la protéine confère un avantage dominant à la bactérie. Le plus souvent, le marqueur sélectif est apporté par un gène de résistance à un antibiotique actif sur la bactérie *E.coli.* L'analyse des séquences des gènes de résistance dans leur grande variété utilisés chez *E.coli* montre que sans aucune exception tous renferment des CpG, bien souvent en nombre très élevé pour les gènes de résistance trouvant leur origine chez les Streptomyces producteurs de l'antibiotique de sélection. De même, il est nécessaire de disposer de gènes rapporteurs qui soient sans CpG tout en restant fonctionnels.
Or, une analyse de plusieurs centaines de gènes chromosomiques et plasmidiques de la bactérie *E.coli* dont les séquences bien caractérisées sont disponibles dans plusieurs banques de données révèlent que tous les gènes sans exception dont la taille est supérieure à 250 pb sont constitués par les 16 dinucléotides.
La présente invention démontre qu'il est malgré tout possible de construire des gènes fonctionnels chez E. coli qui soient dépourvus de CpG. Les inventeurs ont en effet mis au point une méthode pour l'obtention de gènes dépourvus de CpG tout en étant exprimables chez *E. coli.* Cette méthode est basée sur la synthèse d'un enchaînement polynucléotidique en suivant l'enchaînement d'acides aminés d'une protéine qui peut être exprimée chez *E. coli,* en affectant à chaque acide aminé un codon nucléotidique choisi parmi ceux qui, selon le code génétique, et en tenant compte de la dégénérescence de ce code, correspondent à cet acide aminé, mais en éliminant de ce choix :
i. tous les codons contenant un CpG dans leur séquence : sont concernés les codons ACG (Thr), CCG (Pro), GCG (Ala), TCG (Ser), CGA (Arg), CGC (Arg), CGG (Arg) et CGT (Arg), et
ii. les codons finissant par un C quand le codon qui le suit directement commence par un G. Des exemples d'un gène ainsi obtenu comprennent le gène NéoΔCpG (SEQ ID NO : 316 ; *cf*: exemple 11).

Selon une variante de réalisation de l'invention, on éliminera aussi dudit choix les codons dont les fréquences sont faibles dans les protéines d'origine humaine : sont concernés les codons ATA (Ile), CTA (Leu), GTA (Val) et TTA (Leu). L'ensemble des codons possibles correspond donc alors, selon cette variante, à l'ensemble suivant : GCA (Ala), GCC (Ala), GCT (Ala), AGA (Arg), AGG (Arg), AAC (Asn), AAT (Asn), GAC (Asp), GAT (Asp), TGC (Cys), TGT (Cys), CAA (Gln), CAG (Gln), GAA (Glu), GAG (Glu), GGA (Gly), GGC (Gly), GGG (Gly), GGT (Gly), CAC (His), CAT (His), ATC (Ile), ATT (Ile), CTC (Leu), CTG (Leu), CTT (Leu), TTG (Leu), AAA (Lys), AAG (Lys), TTC (Phe), TTT (Phe), CCA (Pro), CCC (Pro), CCT (Pro), TCA (Ser), TCC (Ser), TCT (Ser), AGC (Ser), AGT (Ser), ACA (Thr), ACC (Thr), ACT (Thr), TAC (Tyr), TAT (Tyr), GTC (Val) GTG (Val), GTT (Val),
auquel il faut bien entendu appliquer la règle ii. ci-dessus. Des exemples d'un gène obtenu conformément à cette variante de réalisation comprennent notamment le gène LacZ ΔCpG (positions 3 à 3056 de SEQ ID NO :9 ; *cf.* exemple 5).

De manière préférentielle, ledit choix de codon se fera également de manière à éviter des structures non favorables pour l'ARN messager, comme la présence de séquences d'épissage, de séquences répétées directes ou inversées, des structures en épingle à cheveux ou de signaux de polyadénylation. Le nombre et la variété de taille des gènes synthétisés par cette méthode sont illustrés dans les exemples ci-dessous qui montrent qu'il est ainsi possible de concevoir la synthèse de gènes entièrement dépourvus de CpG qui n'en reste pas moins fonctionnels chez *E. coli.* Comme protéine de référence, on peut choisir toute protéine qui peut être exprimée par *E. coli,* par exemple une protéine codée par un gène de résistance à un antibiotique tel que les gènes de résistance à la zéocine^{®} (phléomycine), à l'hygromycine, à la blasticidine, à la puromycine, ou une protéine codée par un gène rapporteur tel que *lac*Z.

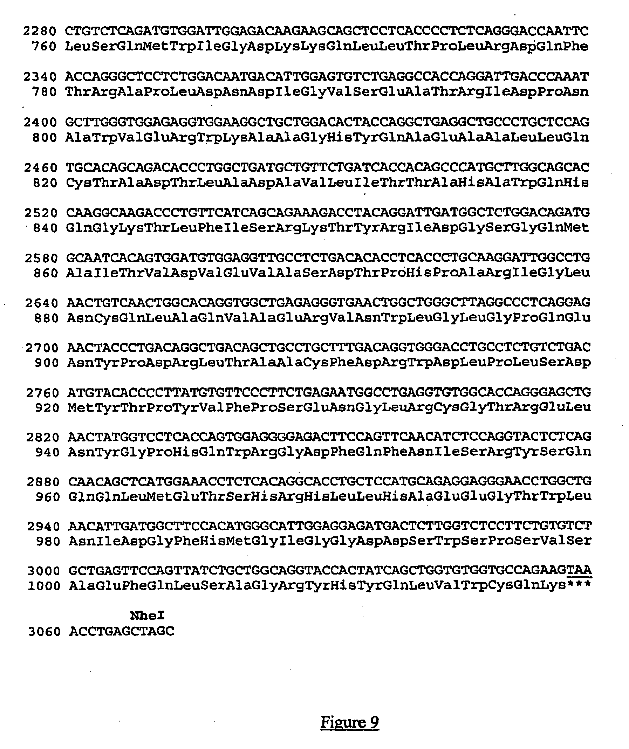

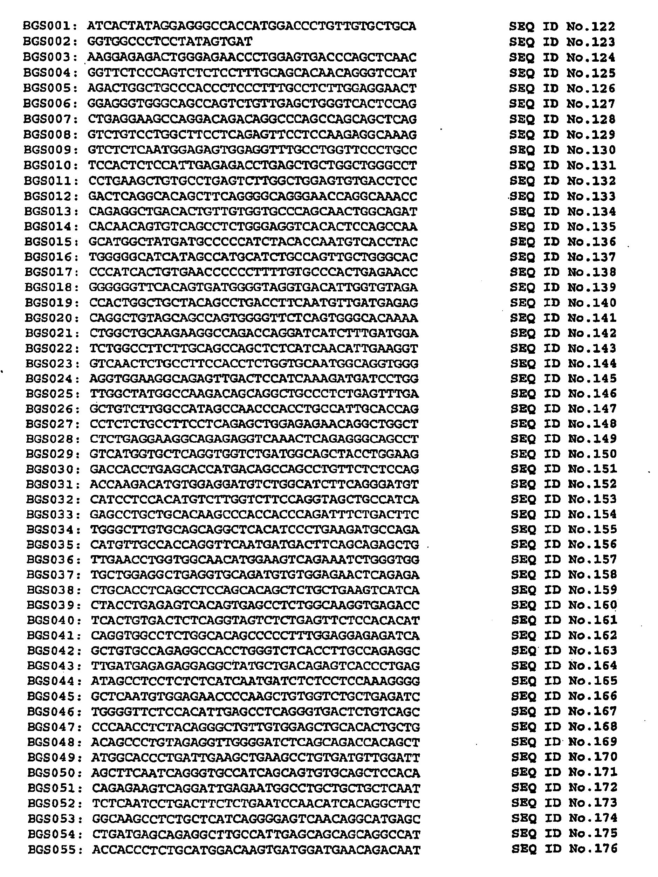

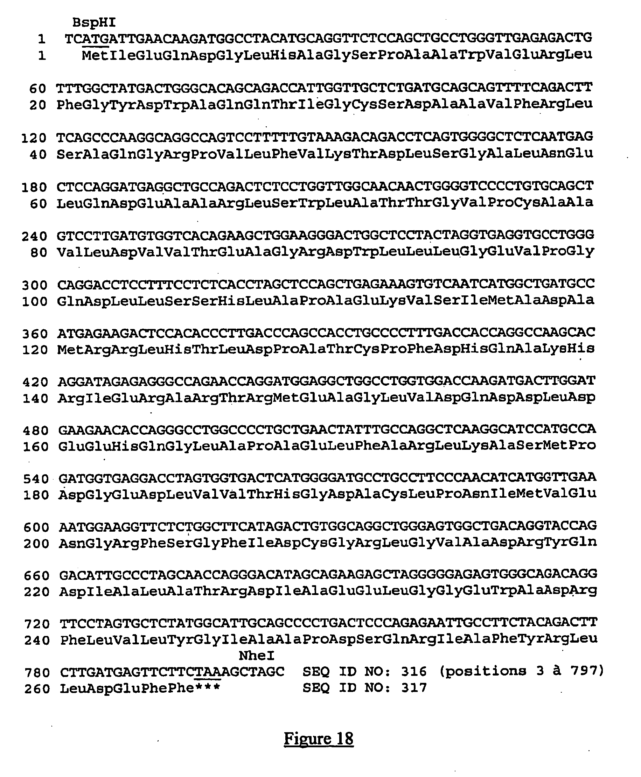

La présente demande décrit une telle méthode d'obtention de gènes dépourvus de CpG exprimables chez *E. coli,* ainsi que tout gène d'au moins 250 pb qui est susceptible d'être obtenu par cette méthode. Plus particulièrement, la présente demande vise :
- tout gène dont la séquence comprend la séquence SEQ ID N°1 de la position 3 à la position 374 (Figure 1), la séquence SEQ ID N°3 de la position 3 à la position 1025 (Figure 3), la séquence SEQ ID N°5 de la position 3 à la position 422 (Figure 5), la séquence SEQ ID N°7 de la position 3 à la position 599 (Figure 7), ainsi que toute utilisation de ces gènes en tant que marqueurs de sélection, et
- tout gène dont la séquence comprend la séquence SEQ ID N°9 de la position 3 à la position 3056 (*cf.* Figure 9), et tout gène dont la séquence comprend la séquence SEQ ID NO : 316 (positions 3 à 797 de la séquence ADN présentée en Figure 18 (codant pour SEQ ID NO : 317), ainsi que toute utilisation d'un tel gène en tant que gène rapporteur.

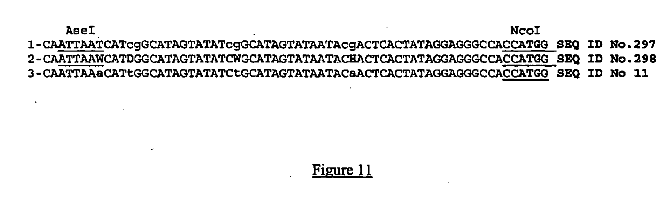

L'expression d'un gène plasmidique nécessite également de disposer de promoteurs adaptés à la cellule hébergeant le plasmide. La connaissance complète du génome d'*E.coli* depuis quelques années a facilité l'étude de différents éléments non codants présentant des fonctions particulières. Les résultats des travaux portant sur la nature des promoteurs d'*E.coli* sont continuellement mis à jour et rendus publics sur le site PromEC accessible par internet (http://bioinfo.md.huji.ac.il/marg/promec). Une analyse des 471 promoteurs bien définis de la base -75 à +25 par rapport au point +1 d'initiation de la transcription révèle que seulement 6 d'entre eux ne possèdent pas de CpG. L'addition de chacun des 6 promoteurs synthétisés chimiquement et placés en amont du gène lacZ codant pour la β-galactosidase d'*E*.*coli* s'est révélée négative pour la révélation de l'activité de ce gène rapporteur. Le manque de forte homologie avec les séquences consensus des boîtes canoniques -10 et -35 suggère que la force de ces promoteurs est faible et alternativement que ceux-ci pourraient être régulés par des conditions d'induction à définir pour chacun d'entre eux. Une analyse des promoteurs bien caractérisés d'*E.coli* a révélé qu'une dizaine seulement ne renfermaient pas de CpG au niveau des boîtes spécifiques de reconnaissance par la RNA polymérase. Une recherche bibliographique a en outre révélé que ces promoteurs étaient tous de nature inductible par divers stimuli, une situation parfois voulue mais le plus souvent délaissée pour un caractère constitutif de l'expression. Les inventeurs ont quant à eux réussi, par assemblage aléatoire PCR de fragments présentant des courtes séquences consensus dépourvues de CpG puisées parmi plusieurs promoteurs forts, à mettre au point des promoteurs inédits qui sont adaptés à l'expression de gènes sans CpG chez *E*. *coli,* et qui présentent l'avantage particulier d'être des promoteurs constitutifs très forts et d'être entièrement dépourvus de CpG. L'exemple 6 ci-dessous illustre la construction du promoteur inédit EM2K par cette technologie. La présente demande vise plus particulièrement tout promoteur dont la séquence comprend la séquence SEQ ID NO: 11 (*cf.* Figure 11).
Les terminateurs caractérisés de transcription chez *E.coli* sont formés par des séquences courtes dont plusieurs ne possèdent pas de CpG, et les inventeurs ont pu vérifier que de tels terminateurs assurent effectivement leur fonction lorsqu'ils sont associés chez *E. coli* à un promoteur et un gène sans CpG selon l'invention.
La présente demande vise ainsi toute unité transcriptionnelle qui comprend au moins un gène sans CpG selon l'invention, et au moins un promoteur sans CpG selon l'invention.
Une telle unité transcriptionnelle peut en outre comprendre au moins un terminateur sans CpG. L'invention fournit ainsi, pour la première fois, un ensemble nucléotidique qui est entièrement dépourvu de CpG, et qui peut néanmoins être exprimé chez E. *coli* en y assurant ses fonctions normales.

La présente demande vise ainsi tout plasmide qui comprend une origine de réplication selon l'invention. De tels plasmides peuvent en outre comprendre un gène sans CpG selon l'invention et/ou un promoteur sans CpG selon l'invention et/ou un terminateur de transcription sans CpG, ou une unité transcriptionnelle selon l'invention. Les plasmides selon l'invention présentent donc l'avantage de pouvoir ne présenter aucun CpG dans leur structure, tout en étant toujours capables d'assurer les fonctions de vecteurs d'expression. Des exemples de tels plasmides sont donnés dans les exemples ci-dessous.

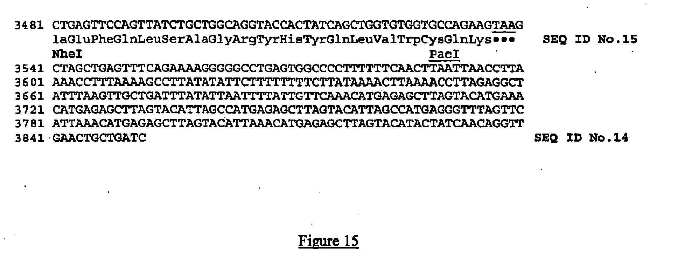

La présente demande vise plus particulièrement tout plasmide de SEQ ID NO: 14 (Figure 15).

Entre également dans le champ de la présente demande toute cellule transformée par au moins un élément sélectionné parmi le groupe constitué par les gènes sans CpG selon l'invention, les promoteurs sans CpG selon l'invention, les origines de réplication sans CpG selon l'invention, les plasmides sans CpG selon l'invention. Une telle cellule selon l'invention peut en outre comprendre un gène codant pour une protéine pi, tel que *pir* sauvage ou *pir* muté *pir*116. Avantageusement, une cellule transformée selon l'invention est une cellule *d'E. coli.*

Pour répliquer en un nombre suffisant de copies fonctionnelles un plasmide selon l'invention, l'homme du métier a à sa disposition de nombreuses bactéries, telles que par exemple les *E. coli* K12 qui sont classiquement utilisées à des fins de réplication plasmidique. Or, la souche originelle K12 d'*E.coli* possède une méthylase de l'ADN, qui introduit un groupement méthyl sur toutes les cytosines placées dans le contexte CC(A/T)GG des ADN génomique et plasmidique de la bactérie. L'ensemble des diverses souches de la lignée K12 possède cette activité du fait d'une méthylase codée par le gène *dcm* (Palmer B. R. et Marinus M. G. (1994) Gene 143, 1-12). Dans la mesure où la méthylation des ADN plasmidiques préparés à partir des souches *dcm*⁺ d'*E.coli* entraîne une modification de la molécule d'ADN non voulue pour le transfert de gènes en cellules eucaryotes, les inventeurs ont mis au point une souche qui permette à la fois le fonctionnement des plasmides avec l'origine R6K *gamma* sans CpG conformément à l'invention, et l'obtention d'ADN plasmidique dépourvu de méthylation sur les sites *dcm*. Pour cela, un nouveau gène a été construit par les inventeurs en délétant le gène *dcm* de la position +3 après l'ATG à la position -14 avant le TGA dans le gène *dcm* d'une souche *pir116 (cf.* exemple 10 ci-dessous).
Le gène *dcm* est situé dans une région chromosomique dont la séquence peut être obtenue via GenBank avec le numéro d'accession D90835 (cloneKohara #344 : 43,5-43,9 min).

Une délétion dans le gène (-) apporte l'avantage supplémentaire d'éviter toute réversion du gène vers la forme sauvage. Des souches mutantes *dcm* ont ainsi été produites par les inventeurs ; elles ne présentent aucun phénotype négatif qui pourrait altérer la croissance des bactéries ou modifier la qualité et la quantité de l'ADN plasmidique. Plus particulièrement, une souche optimisée d'*E. coli* a été construite par inactivation ciblée du gène *dcm* à partir d'une souche parente exprimant une protéine pi mutée en un site conduisant à l'augmentation du nombre de copies des plasmides sans CpG. Cette souche optimisée permet une production de qualité et en abondance des ADN plasmidiques objets de cette invention dépourvus de CpG et exempts de méthylation sur les cytosines des sites *dcm.* La présente demande vise donc toute cellule comprenant un gène codant pour la protéine pi, qui est transformée par le gène dcm délété selon l'invention, et toute méthode de réplication de plasmides qui comprend la transformation d'une telle cellule par un plasmide, et la culture de la cellule transformée dans des conditions adaptées à la réplication de ce plasmide.

La présente demande vise ainsi une méthode pour la production d'un plasmide entièrement dépourvu de CpG et exempt de méthylation au niveau de la cytosine dans le contexte nucléique CC(A/T)GG, caractérisée en ce que l'on produit un plasmide selon l'invention par réplication dans une souche d'*Escherichia coli* exprimant la protéine pi déficiente pour le système de méthylation *dcm.*

Entre également dans le champ de la présente demande tout kit de production de plasmides, qui comprend au moins une cellule selon l'invention. Ces kits sont particulièrement adaptés à la réplication de plasmides selon l'invention, afin d'éviter que ces plasmides dont le structure est dépourvue de CpG soit par ailleurs, lors de leur réplication, méthylés en CC(A/T)GG.

L'invention fournit ainsi un ensemble complet de moyens de transformation dépourvus de CpG : gènes sans CpG, promoteurs sans CpG, unités transcriptionnelles sans CpG, origines de réplication pour plasmide sans CpG, plasmides sans CpG, cellules spécialement adaptée à la réplication de plasmides sans méthylation de cytosines. Ces nouveaux moyens trouvent des applications directes pour la transformation génétique de cellules à visée biotechnologique ou médicale. De tels produits sont en effet exceptionnellement bien adaptés à la réalisation de compositions vaccinales à ADN destinées à l'homme ou l'animal.

L'invention est illustrée par les exemples suivants, dans lesquels il est fait référence aux figures :

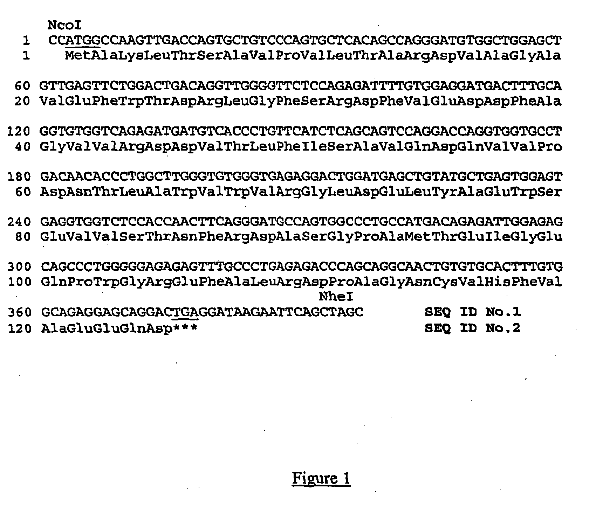

- Figure 1 : séquence du gène Sh ble ΔCpG (sans CpG),

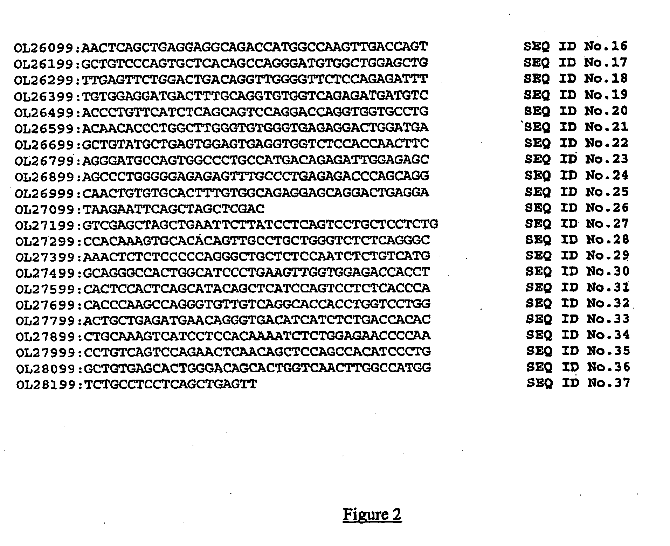

- Figure 2 : liste des oligonucléotides utilisés pour l'assemblage du gène Sh ble ΔCpG,

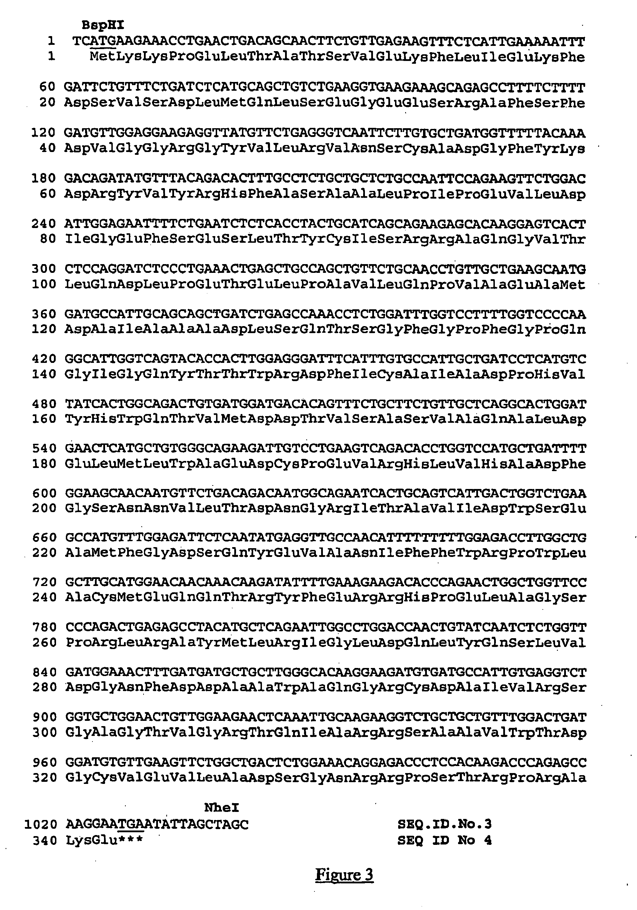

- Figure 3 : séquence du gène Hph ΔCpG,

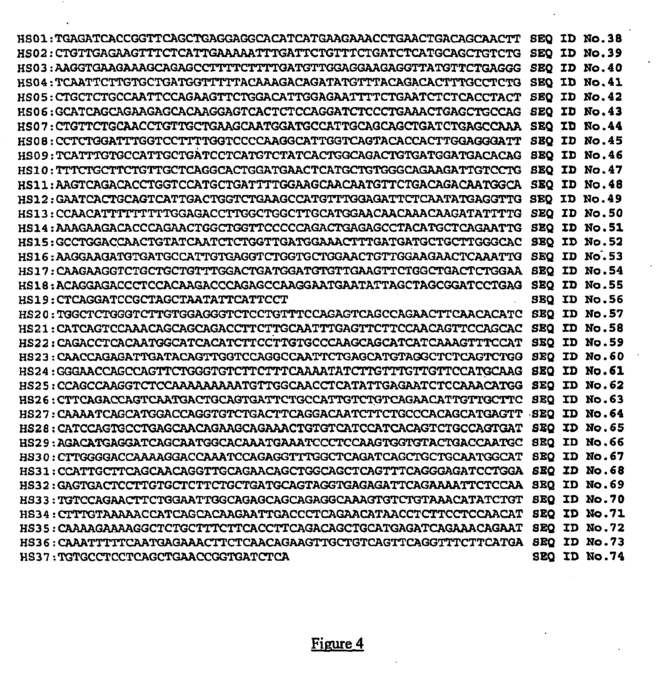

- Figure 4 : liste des oligonucléotides utilisés pour l'assemblage du gène Hph ΔCpG,

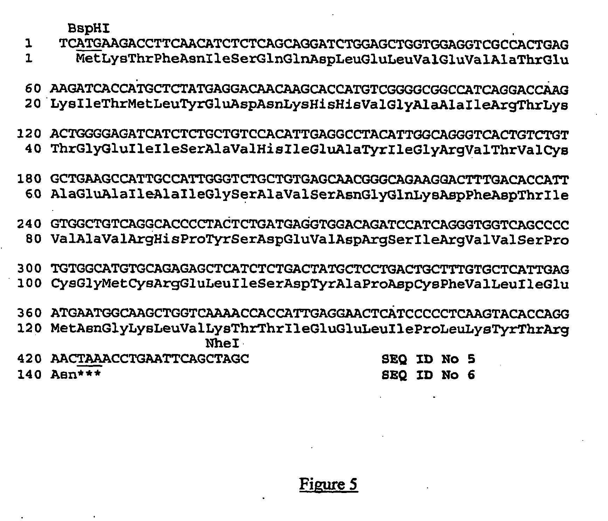

- Figure 5 : séquence du gène Bsr ΔCpG,

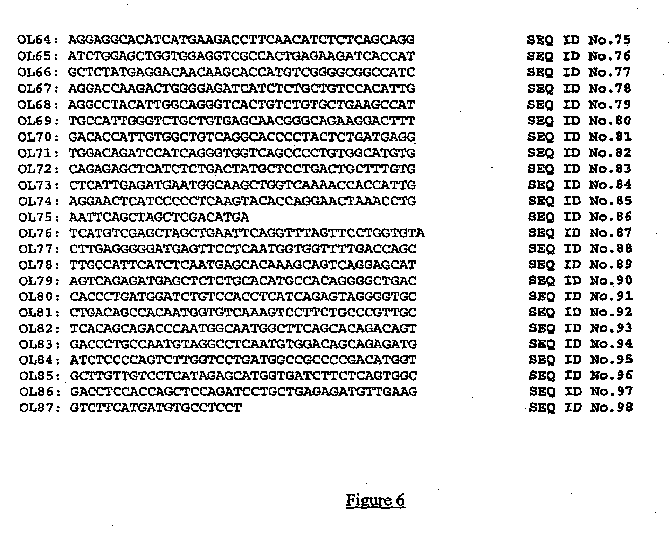

- Figure 6 : liste des oligonucléotides utilisés pour l'assemblage du gène Bsr ΔCpG,

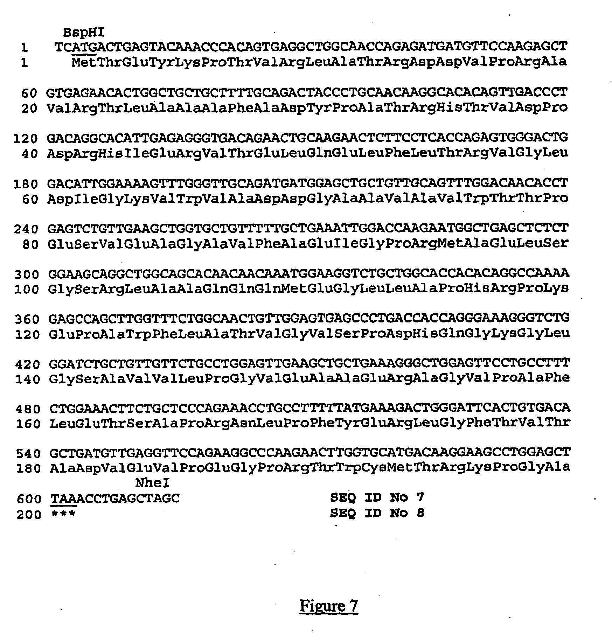

- Figure 7 : séquence du gène Pac ΔCpG,

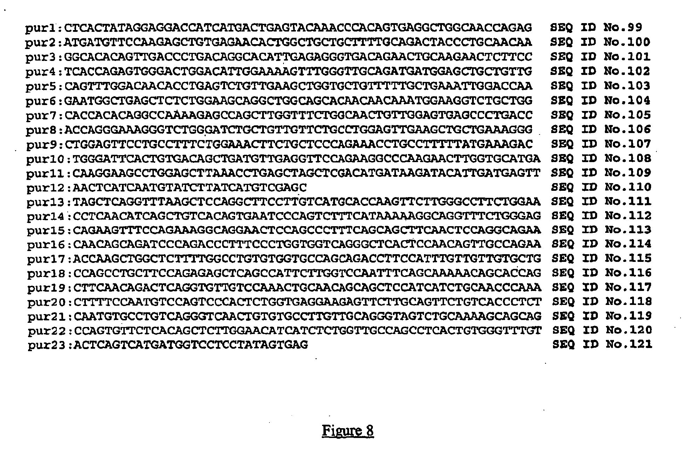

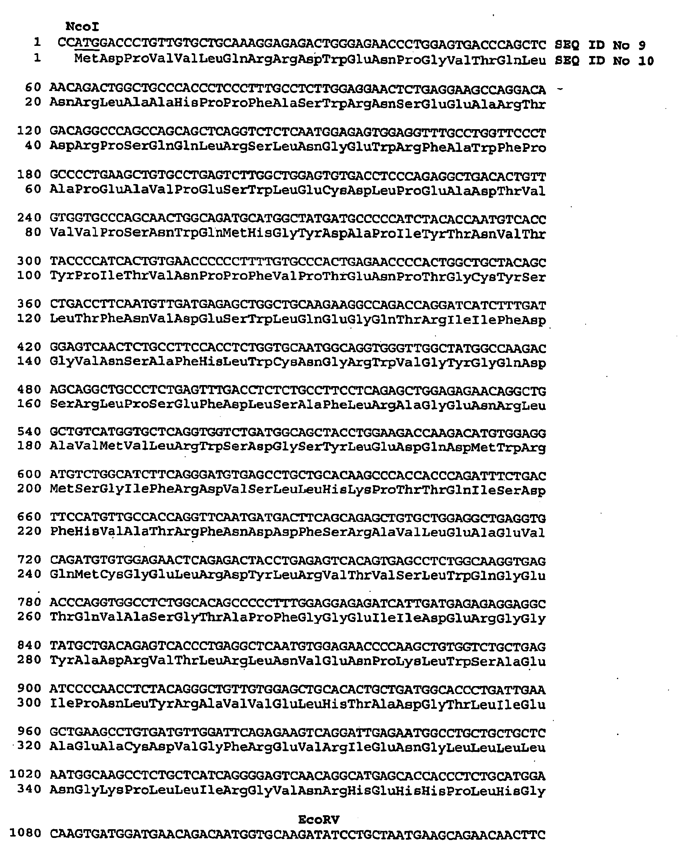

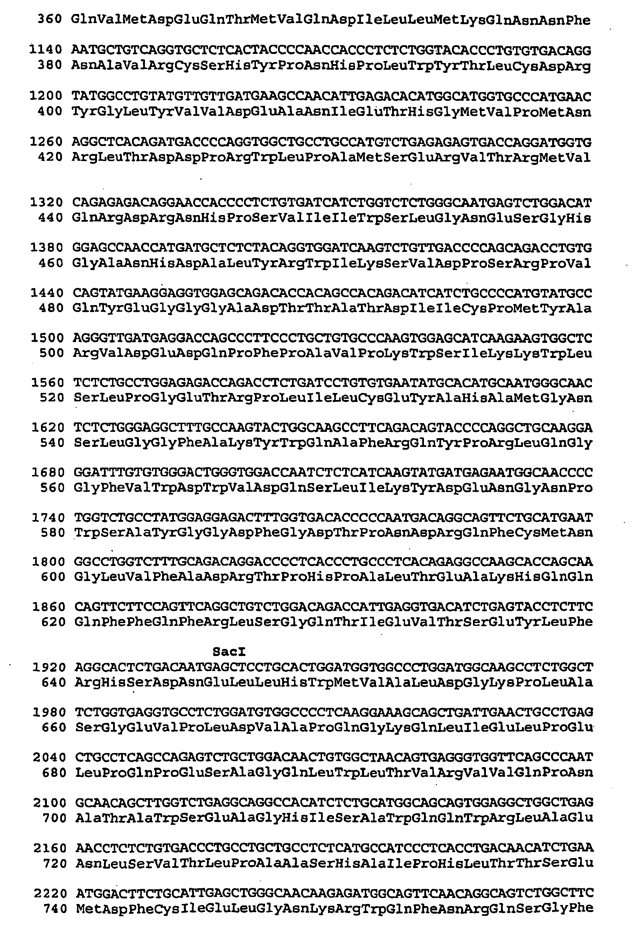

- Figure 8 : liste des oligonucléotides utilisés pour l'assemblage du gène Pac ΔCpG,
- Figure 9 : séquence du gène LacZ ΔCpG,

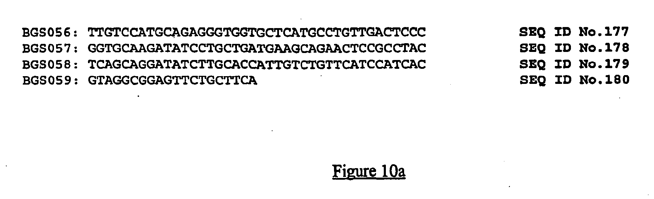

- Figure 10A : liste des oligonucléotides utilisés pour l'assemblage du premiers tiers du gène LacZ ΔCpG,

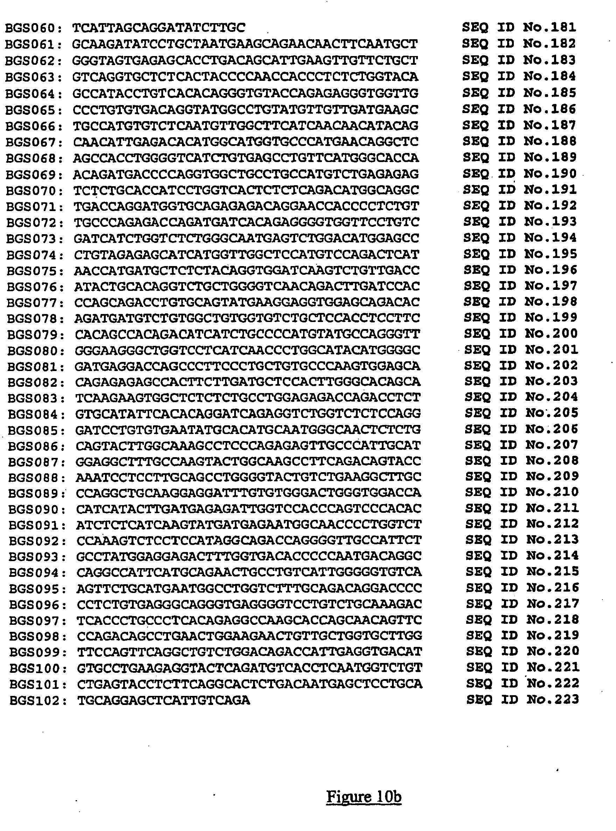

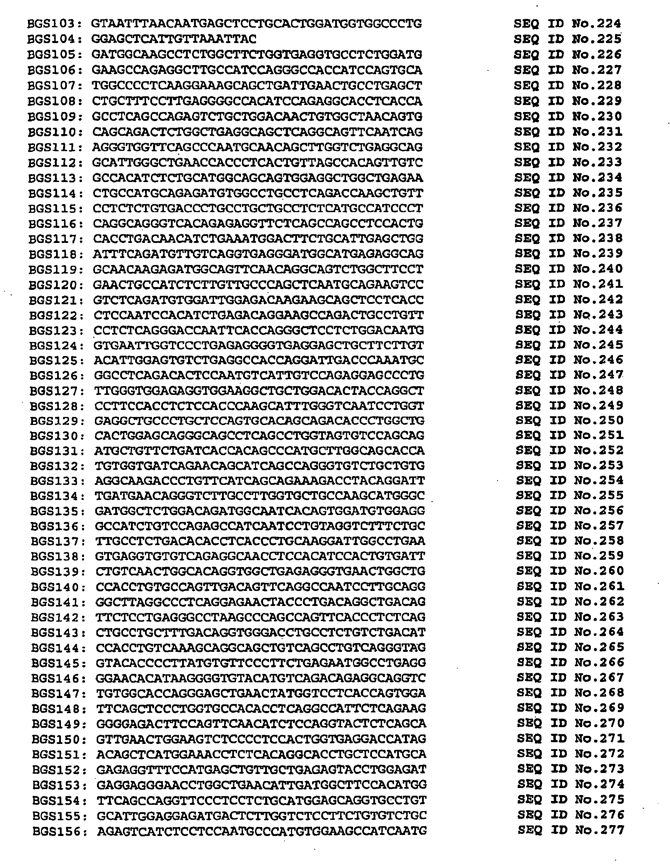

- Figure 10B : liste des oligonucléotides utilisés pour l'assemblage du deuxième tiers du gène LacZ ΔCpG,

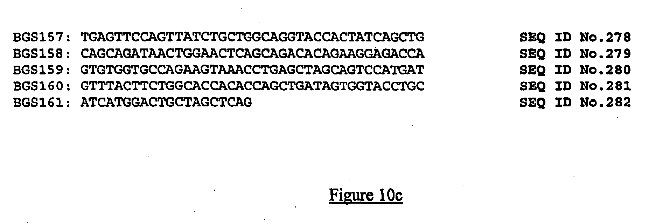

- Figure 10C : liste des oligonucléotides utilisés pour l'assemblage du troisième tiers du gène LacZ ΔCpG,
- Figure 11: séquence du promoteur EM7(1-), des oligonucléotides dégénérés (2-) utilisés pour construire le promoteur EM2K dépourvu de CpG (3-),
- Figure 12 : séquence de l'origine de réplication R6K gamma M2A,

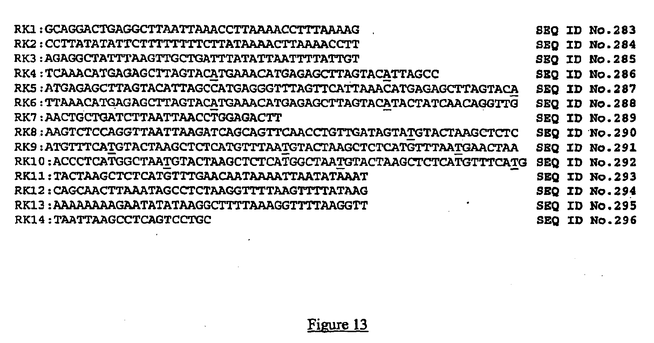

- Figure 13 : liste des oligonucléotides utilisés pour l'assemblage de l'origine de réplication R6K gamma M2A,
- Figure 14 : séquence de l'origine R6K gamma du plasmide pGTR6Kneoc9 délimitée par les sites PacI,
- Figure 15 : séquence du plasmide pSh-LacZΔCpG,

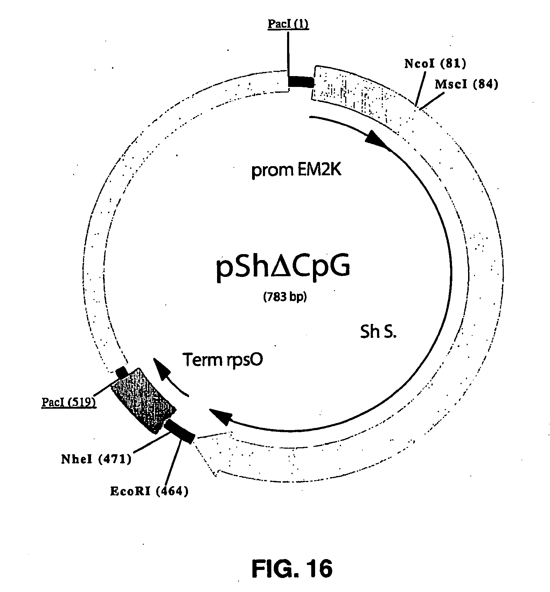

- Figure 16 : carte du plasmide pShΔCpG,

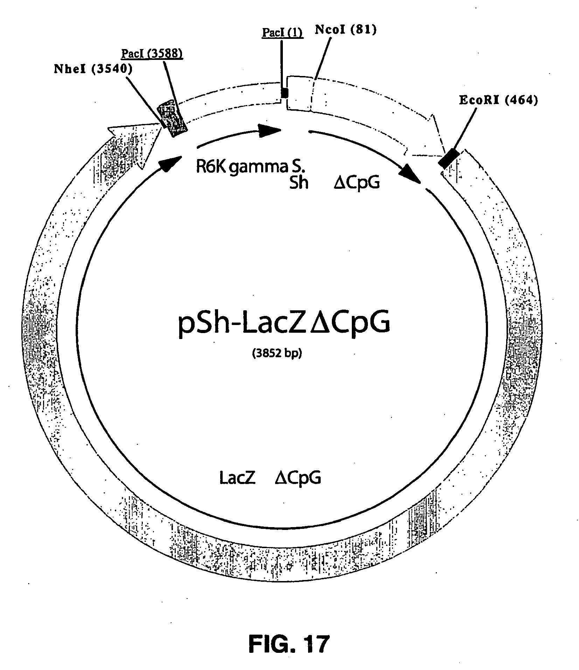

- Figure 17 : carte du plasmide pSh-LacZΔCpG,
- Figure 18 : séquence du gène NéoΔCpG (sans CpG) [position 3 à 797 de séquence ADN = SEQ ID NO : 316 ; séquence protéique = SEQ ID NO : 317],

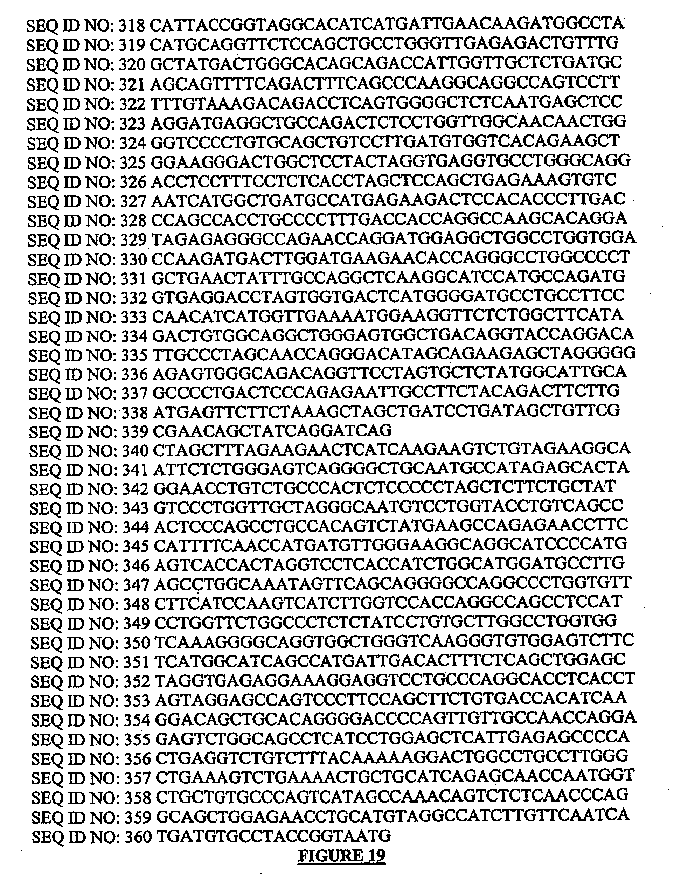

- Figure 19 : séquence des oligonucléotides SEQ ID NO : 318 à SEQ ID NO :X utilisés pour l'assemblage du gène NéoΔCpG.

### EXEMPLE 1 : Construction du gène Sh ble de résistance à la Zéocine dépourvu de CpG

Le gène Sh blé ΔCpG dont la séquence présentée en figure 1 (position 3 à 377 de SEQ ID NO:1) a été synthétisé à partir d'un assemblage d'oligonucléotides chevauchants (taille 20-40 pb) dont les séquences sont données dans la figure 2. La méthode d'assemblage se fait en trois étapes, la première consiste en la phosphorylation des oligonucléotides du brin codant, dans une deuxième étape l'ensemble des oligonucléotides des deux brins sont associés par hybridation et ligation et, dans la dernière étape, le gène est amplifié par PCR. Cette méthode a été utilisée avec succès pour la synthèse de tous les gènes synthétiques mentionnés dans les exemples 1, 2, 3, 4, 5. La méthode est détaillée pour le gène Sh ble ΔCpG:
Les 10 oligonucléotides de OL26199 à OL27099 (figure 2) correspondant au brin codant sont phosphorylés selon la procédure suivante: 1µl de chacun des oligonucléotides repris dans de l'eau à 250 µM sont mélangés dans un microtube contenant 15 µl d'eau pour amener la solution finale à une concentration de 100 picomoles par microlitre. On mélange ensuite 5 µl de cette solution à 10 µl de tampon de la polynucléotide-Kinase concentré 10 fois, 0,4 µl d'une solution d'ATP 50mM, 85 µl d'eau et 1 µl de l'enzyme (à 10 u/µl) et le tout est incubé 4 heures à 37°C (solution A).
Une solution des oligonucléotides du brin non codant est constituée en mélangeant 1 µl de chaque oligonucléotide (OL27199 à OL28199 ; cf. figure 2) et 1 µl de l'oligonucléotide OL26099 (figure 2) dans laquelle on ajoute 43 µl d'eau afin d'obtenir une solution finale à 54 picomoles par µl (solution B).
L'assemblage du gène est réalisé d'abord en mélangeant 10 µl de la solution A, 1 µl de la solution B, 6 µl d'une solution de KCI à 100 mM, 3 µl d'une solution de NP-40 à 0,5%, 4 µl d'une solution de MgCl2 à 50 mM, 3 µl d'une solution d'ATP à 10 mM et 7,5 µl de ligase Pfu (30 unités) puis le mélange est chauffé dans un thermocycleur programmable 3 minutes à 95°C puis 3 minutes à 80°C avant de subir 3 cycles d'une minute à 95°C, suivie d'un passage de 95°C à 70°C en 1 minute, puis d'un passage de 70°C à 55 °C en 1 heure et enfin 2 heures à 55°C. Puis le mélange des oligonucléotides assemblés est amplifié avec les amorces OL26099 et OL27199. Le produit d'amplification est purifié sur colonne Promega, digéré par les enzymes de restriction NcoI et NheI et cloné dans le plasmide pMOD1LacZ(wt) linéarisé par NcoI et NheI. Les séquences de l'ADN plasmidique de 2 clones résistants à la zéocine apparus après transformation de la souche d'*E.coli* GT100 (disponible auprès de Invivogen) par le mélange de la ligation entre le fragment vecteur et le fragment PCR, ont été trouvées conformes à la séquence désirée présentée en Figure 1. Ce gène synthétique placé sous la dépendance du promoteur bactérien EM7 (vecteur pMOD1Sh ΔCpG) confère une résistance à la zéocine identique à celle apportée par le même vecteur contenant le gène natif *Sh ble* avec la souche d'*E.coli* réceptrice GT100.

### EXEMPLE 2 : Construction du gène Hph de résistance à l'hygromycine dépourvu de CpG

Le gène synthétique Hph ΔCpG (séquence SEQ ID NO:3 présentée en Figure 3) a été construit selon la méthode décrite dans l'exemple 1. Les deux brins ont été synthétisés à l'aide d'oligonucléotides de 60 bases plus deux oligonucléotides de 30 bases avec une région chevauchante de 30 bases. L'assemblage des différents oligonucléotides présentés en Figure 4 a été réalisé par une PCR finale avec les oligonucléotides sens TTCAGCTGAGGAGGCACATC (SEQ ID NO: 299) et reverse CTCAGGATCCGCTAGCTAAT (SEQ ID NO: 300) selon les conditions expérimentales mentionnées dans l'exemple précédent.
Le fragment amplifié et purifié (1068 pb) a été ensuite digéré par les enzymes de restriction BspHI et NheI et cloné dans le vecteur pMOD2 LacZ(wt) dans lequel le site NcoI de pMOD1 est remplacé par le site BspHI. La sélection des clones d'*E.coli* contenant ce vecteur recombinant a été effectuée sur milieu FastMedia^{™} Hygro Agar (Cayla). La séquence SEQ ID NO: 3 de la figure 3 a été confirmée par séquençage sur les deux brins de l'ADN plasmidique de deux clones résistants à l'hygromycine. Ce gène synthétique placé sous la dépendance du promoteur bactérien EM7 (vecteur pMOD2 Hph ΔCpG) confère une résistance à l'hygromycine B au moins égale à celle apportée par le même vecteur contenant le gène natif Hph avec la souche d'*E.coli* réceptrice GT100.

### EXEMPLE 3 : Construction du gène Bsr de résistance à la blasticidine dépourvu de CpG.

Le gène Bsr ΔCpG dont la séquence est présentée en figure 5 (SEQ ID NO: 5) a été synthétisé à partir des oligonucléotides indiqués en 6 en suivant la méthode décrite dans l'exemple 1. Le mélange des oligonucléotides assemblés a été amplifié avec les amorces OL64 et OL76 (cf. Figure 6). Le fragment amplifié et purifié a été ensuite digéré par les enzymes de restriction BspHI et NheI et cloné dans le vecteur pMOD2 LacZ(wt) . La sélection des clones d'*E.coli* contenant ce vecteur recombinant a été effectuée sur milieu FastMedia^{™} Blasti Agar (Cayla). La séquence SEQ ID NO:5 de la figure 5 a été confirmée par séquençage sur les deux brins de l'ADN plasmidique de deux clones résistants à la blasticidine. Ce gène synthétique placé sous la dépendance du promoteur bactérien EM7 (vecteur pMOD2 Bsr ΔCpG) confère une résistance à la blasticidine identique à celle apportée par le même vecteur contenant le gène natif Bsr avec la souche d*'E.coli* réceptrice GT100.

### EXEMPLE 4 : Construction du gène Pac de résistance à la puromycine dépourvu de CpG

Le fragment BspHI-NheI (gène Pac ΔCpG ; SEQ ID NO: 7) dont la séquence est présentée en figure 7 a été synthétisé par un assemblage des oligonucléotides indiqués en Figure 8.
Le mélange des oligonucléotides assemblés a été amplifié avec les amorces sens pur24 (AGGACCATCATGACTGAG; SEQ ID NO: 301) et reverse pur25 (ATCATGTCGAGCTAGCTC ; SEQ ID NO: 302). Le fragment BspHI-NheI purifié a été cloné dans le plasmide pMOD2LacZ(wt) entre les sites BspHI et NheI. Les séquences de l'ADN plasmidique de 2 clones de la souche GT100 résistants à la puromycine apparus sur le milieu FastMedia^{™} puro Agar (Cayla) après transformation par le produit de la ligation entre le fragment vecteur et le fragment PCR, ont été trouvées conformes à la séquence désirée présentée en Figure 7. Le gène synthétique Pac ΔCpG placé sous la dépendance du promoteur bactérien EM7 (vecteur pMOD2 Pac ΔCpG) confère une résistance à la puromycine légèrement supérieure à celle apportée par le même vecteur contenant le gène natif *pac* avec la souche réceptrice GT100 d'*E.coli .*

### EXEMPLE 5 : Construction du gène LacZ dépourvu de CpG codant pour la β-galactosidase d'E. coli

Le gène synthétique LacZ ΔCpG (SEQ ID NO: 9 présentée en Figure 9) a été construit selon la méthode décrite dans les exemples précédents. Compte-tenu de la taille du gène à réaliser (plus de 3000 pb), la construction a été effectuée en trois parties distinctes en conservant les sites de restriction EcoRV et SacI aux mêmes sites que sur la séquence native du gène *lac*Z. Pour chaque partie, les deux brins ont été synthétisés à l'aide d'oligonucléotides de 40 bases plus deux oligonucléotides de 20 bases avec une région chevauchante de 20 bases.
La première région correspond au fragment NcoI-EcoRV (Partie I), la deuxième région correspond au fragment EcoRV-SacI (Partie II) et la troisième région correspond au fragment SacI-NheI. L'assemblage des différents oligonucléotides présentés dans les figures 10A (oligonucléotides utilisés pour l'assemblage de la partie I), 10B (oligonucléotides utilisés pour l'assemblage de la partie II), 10C (oligonucléotides utilisés pour l'assemblage de la partie III), a été réalisé par PCR selon les mêmes conditions expérimentales énoncées dans les exemples précédents. Le clonage progressif des trois parties du gène synthétique a été effectué dans le vecteur pMOD1 LacZ(wt). La fonctionnalité de chaque partie clonée ainsi que celle du gène synthétique complet présent sur le vecteur pMOD1 LacZ a été démontrée par la révélation de l'activité β-galactosidase sur milieu FastMedia^{™} Amp Xgal Agar (Cayla) des clones recombinants obtenus dans la souche MC1061ΔLac. Le gène synthétique complet LacZ ΔCpG mis sous la dépendance du promoteur,EM7 donne 30% de moins d'activité β-galactosidase (dosage luminométrique d'extraits protéiques de culture) par rapport à l'expression du gène natif LacZ dans le même environnement plasmidique.

### EXEMPLE 6: Construction d'un promoteur constitutif fort d'E.coli dépourvu de CpG

Le promoteur bactérien EM7 présent sur les vecteurs de type pMOD1 est un promoteur synthétique, constitutif et fort chez *E.coli.* Sa séquence qui contient 3 CpG (SEQ ID NO: 297 en Figure 11) a servi de référence pour élaborer un promoteur bactérien dépourvu de CpG. Nous avons réalisé des oligonucléotides "linker" dégénérés à 4 endroits (indiqués W,D,W et H sur la séquence SEQ ID NO:298 en Figure 11) et compatibles avec les sites de restriction AseI et NcoI. Ces différents oligos ont été hybridés et clonés dans le pMOD1 ShtΔCpG entre les sites de restriction AseI et NcoI du promoteur EM7. Après sélection des clones recombinants sur milieu FastMedia^{™}Zeo Agar et détermination de la séquence promotrice du clone le plus résistant à la zéocine, nous avons retenu le promoteur EM2K (séquence SEQ ID NO: 11 en Figure 11) comme promoteur bactérien dépourvu de CpG.

### EXEMPLE 7 : Synthèse des origines R6K gamma dépourvues de CpG

Le fragment d'ADN PacI contenant l'origine R6K gamma M2A (SEQ ID NO: 12 en Figure 12) a été synthétisé par PCR à partir de l'assemblage des oligonucléotides indiqués en Figure 13. L'assemblage du fragment R6K gamma M2A a été amplifié avec les amorces RK15 (GCAGGACTGAGGCTTAATTAAACCTTAAAAC ; SEQ ID NO: 303) et RK16 (AAGTCTCCAGGTTAATTAAGATCAGCAGTTC; SEQ ID NO: 304), et les fragments après digestion par l'enzyme PacI ont été clonés dans un plasmide (pGTCMVneo) contenant le gène de résistance à la kanamycine et l'origine de réplication pUC bornée par 2 sites PacI. De nombreux transformants de la souche GT97 (qui exprime la protéine pi) ont été analysés et n'ont été retenus que des clones contenant un plasmide à forte copie et conservé après plusieurs sous-cultures en absence de kanamycine. Il a été trouvé après séquençage que le fragment ori de la plupart de ces plasmides pouvait posséder un nombre plus réduit (5-6) de séquences répétées au lieu des 7 de l'origine naturelle du plasmide R6K. Une de ces nouvelles séquences de l'origine R6K gamma synthétique dépourvue de CpG est présentée en SEQ ID NO: 13 en Figure 14.
Deux autres versions de l'origine R6K gamma dans lesquelles le G de chaque CpG présent dans les séquences répétées (élément de 22 bp répété plusieurs fois dans la région de fixation de la protéine pi) a été remplacé par un C pour donner l'origine (R6K gamma M2C) ou un T pour donner l'origine (R6K gamma M2T) ont été synthétisées d'une manière analogue. La fonctionnalité de ces nouvelles origines R6K gamma dans lesquelles le G des CpG des séquences répétées est remplacé par un C ou par un T, ajouté à l'exemple de l'origine de la Figure 13 où le G est remplacé par un A démontre que les CpGs de ces séquences répétées n'ont pas de rôle dans la fonctionnalité de l'origine.

### EXEMPLE 8 : Assemblage de vecteurs plasmidiques entièrement dépourvus de CpG exprimant un gène de résistance chez E.coli

En premier lieu une cassette PacI-PacI contenant le promoteur bactérien EM2K, le gène de résistance à la Zéocine Sh ΔCpG suivi d'un terminateur bactérien sans CpG a été réalisé. Pour cela, des oligonucléotides "linker" contenant la séquence du terminateur *tl* de la région intergénique *rpsO-pnp* d'*E.coli* ont été hybridés et clonés entre les sites NheI et PacI du vecteur pMOD1 EM2K Sh ΔCpG :
Oligonucléotides "linker":
   rpsO-1(5'->3'):
   CTAGCTGAGTTTCAGAAAAGGGGGCCTGAGTGGCCCCTTTTTTCAACTTAAT SEQ ID NO: 305
   rpsO-2(5'->3'): TAAGTTGAAAAAAGGGGCCACTCAGGCCCCCTTTTCTGAAACTCAG SEQ ID NO: 306

Le vecteur recombinant obtenu (pMOD1 EM2K Sh ΔCpG Term) a été vérifié par séquençage au niveau de la séquence terminatrice qui ne contient pas naturellement de CpG. La cassette EM2K- Sh ΔCpG -Term contenue dans ce vecteur a été ensuite amplifiée par PCR pour la borner des deux côtés par des sites PacI à l'aide des amorces suivantes :
PACI-UP (5'->3'): ATCGTTAATTAAAACAGTAGTTGACAATTAAACATTGGC SEQ ID NO:307
PACI-DOWN(5'->3'): ATCGTTAATTAAGTTGAAAAAAGGGGCC SEQ ID NO: 308

Ce fragment amplifié a été ensuite purifié et coupé par PacI puis assemblé au fragment PacI contenant l'origine R6K gamma ΔCpG décrite dans l'exemple 7. Après transformation de ce mélange de ligation dans la souche GT97 (qui exprime la protéine pi) et sélection sur milieu FastMedia^{™}Zeo, l'analyse des clones recombinants obtenus a révélé deux orientations possibles du fragment PacI-PacI contenant l'origine R6K gamma ΔCpG. L'orientation retenue dans le pSh ΔCpG est représentée en Figure 16.

### EXEMPLE 9: Assemblage d'un vecteur plasmidique entièrement dépourvu de CpG exprimant le gène de résistance à la zéocine et le gène de la β-galactosidase chez E.coli.

Le vecteur pSh ΔCpG décrit dans l'exemple 8 (Figure 16) a été utilisé pour insérer le gène synthétique LacZ dépourvu de CpG entre les sites EcoRI et NheI. Pour cela des oligonucléotides "linker", compatibles EcoRI et NcoI, contenant une séquence consensus de site de fixation des ribosomes d'*E.coli* ont été hybridés et clonés avec le fragment LacZΔCpG NcoI-NheI du pMOD1 LacZΔCpG entre les sites EcoRI et NheI du vecteur pMOD1 EM2K ShΔCpG.

### Oligonucléotides "linker" utilisés:

rbs-1(5'->3'):AATTCTGAGGAGAAGCT SEQ ID NO: 309
rbs-2(5'->3'):CATGAGCTTCTCCTCAG SEQ ID NO: 310

La transformation de ce mélange de ligation dans la souche GT97 (qui exprime la protéine pi) et la sélection sur milieu FastMedia^{™}Zeo Xgal ont permis l'obtention des clones recombinants contenant le vecteur pSh-LacZΔCpG (Figures 15 et 17). Ce vecteur co-exprime, sous la dépendance du promoteur bactérien EM2K, en système d'opéron artificiel les gènes ShΔCpG et LacZΔCpG.

### EXEMPLE 10 : Obtention d'une souche d'E.coli exprimant la protéine mutante pi116 et portant une délétion dans le gène dcm.

Le gène *pir* codant pour la protéine pi indispensable à l'initiation de la réplication de l'origine R6K *gamma* ainsi que que le gène muté *pir116* conduisant à une augmentation du nombre de copies des plasmides R6K *gamma* ont été introduits sous une forme fonctionnelle dans diverses souches d'*E.coli* K12 par différents groupes. Des souches de ce type peuvent être obtenues auprès du *E.coli* Genetic Stock Center (http://cgsc.biology.yale.edu), et sont aussi commercialement disponibles auprès d'entreprises spécialisées dans la fourniture de matériel biologique pour la recherche. C'est le cas par exemple des souches pir1 (*pir116*) et pir2 (*pir sauvage*) proposées par la société Invitrogen dont les produits peuvent être achetés dans tous les pays européens. La souche GT 97 de lignée K12, de génotype Δ*lac169 hsdR514 endA1 recA1 codBA uidA (ΔMluI)::pir 116* (disponible auprès de InvivoGen), a été choisie pour sa rusticité, la constance des préparations d'ADN des plasmides R6K *gamma* et ses niveaux élevés de compétence parmi plusieurs souches K12 *pir* de génotype distinctif pour quelques gènes. L'introduction d'une délétion dans le gène *dcm* de la souche GT 97 a été réalisée de la façon suivante:
Deux régions d'ADN de 1,8 kb et 1,5 kb, bordant respectivement le codon d'initiation ATG (fragment A) et le codon stop TGA (fragment B) du gène *dcm* ont été amplifiés par PCR. Le fragment A a été amplifié avec le couple d'amorces OLdcmAF (TTTTGCGGCCGCTTGCTGCGCCAGCAACTAATAACG; SEQ ID NO: 311) et OLdcmAR (CCTTGGATCCTGGTAAACACGCACTGTCCGCCAATCGATTC ; SEQ ID NO: 312) et le fragment B a été amplifié avec le couple d'amorces OLdcmBF (TTTTGGATCCTCAGCAAGAGGCACAACATG; SEQ ID NO: 313) et OLdcmBR (TTTTCTCGAGAAACGGCAGCTCTGATACTTGCTTC ; SEQ ID NO: 314). Les sites de restriction pour les enzymes NotI (GCGGCCGC) BamHI (GGATCC) et XhoI (CTCGAG) ont été introduits dans les amorces afin d'associer le fragment A et le fragment B entre eux en formant un élément génétique borné par les sites NotI et XhoI. La région du gène *dcm* est ainsi reconstituée en créant une délétion qui s'étend de la position +3 après l'ATG à la position -14 avant le TGA. Cet élément génétique a été cloné dans pKO3 (Link A. J., Phillips D. et Church G. M. (1997) J Bacteriol 179, 6228-37), vecteur développé pour le remplacement d'allèle chez *Escherichia coli* à réplication thermosensible entre les sites NotI et SalI pour donner le plasmide désigné pKO3Δdcm. Ce plasmide a été co-transformé dans la souche GT97 avec un plasmide qui exprime la protéine RecA (pFL352). Un transformant contenant les deux plasmides a été cultivé à une température non permissive (42°C) en présence de chloramphénicol pour sélectionner des clones qui ont intégré par recombinaison homologue le pKO3Δdcm dans le chromosome bactérien. Un sous clone résistant au chloramphénicol à 42°C a ensuite été cultivé à 30°C sur un milieu contenant une forte concentration de saccharose (5%) pour contre-sélectionner les souches qui après un deuxième événement de recombinaison homologue ont échangé la région chromosomique du gène *dcm* avec le fragment homologue cloné dans le plasmide. La délétion introduite dans le clone retenu (GT106) a été vérifié par PCR avec le couple d'amorces OldcmAF et OldcmBR générant un fragment d'une taille inférieur à celle obtenue avec la souche parentale et par une PCR avec l'amorce OldcmBR et une amorce positionnée à l'extérieur de la région échangée (OldcmCF TTTTGCGGCCGCGTTGCGGTATTACCCTTGTC ; SEQ ID NO:315).
Le génotype *dcm* de la souche GT106 a été confirmé en introduisant dans celle- ci et dans GT106 un plasmide contenant un site de restriction de l'enzyme SexAI sujet à la méthylation dcm. Le plasmide purifié à partir de GT106 est clivé par SexAI alors qu'il est résistant à l'enzyme quand il est purifié à partir de GT97.
Cette dernière souche désignée GT106 présente les mêmes caractéristiques de croissance de la souche parentale GT97 et comme attendue aucune modification négative de la quantité des ADN des plasmides R6K *gamma* n'a été observée, seule la qualité des ADN estimée par l'absence de méthylation des cytosines des sites *dcm* a été améliorée. La souche GT106 sera disponible auprès de la société Invivogen à compter du jour du dépôt de cette demande de brevet.

### EXEMPLE 11- production du gène Néo de résistance à la néomycine dépourvu de CpG.

Le gène Néo ΔCpG dont la séquence est présentée en figure 18 (position 3 à 797 de séquence ADN présentée en Figure 18 = SEQ ID NO: 316 ; séquence protéique = SEQ ID NO : 317) a été synthétisé à partir d'un assemblage d'oligonucléotides chevauchants (taille 20-40 pb) dont les séquences sont données dans la figure 19. La méthode d'assemblage se fait en trois étapes, la première consiste en la phosphorylation des oligonucléotides du brin codant, dans une deuxième étape, l'ensemble des oligonucléotides des deux brins sont associés par hybridation et ligation et, dans la dernière étape, le gène est amplifié par PCR.
Les 20 oligonucléotides de SEQ ID NO: 319 à SEQ ID NO:338 (Figure 19) correspondant au brin codant sont phosphorylés selon la procédure suivante: 1µl de chacun des oligonucléotides repris dans de l'eau à 250 µM sont mélangés dans un microtube contenant 50 µl d'eau pour amener la solution finale à une concentration de 100 picomoles par microlitre. On mélange ensuite 5 µl de cette solution à 10 µl de tampon de la polynucléotide-Kinase concentré 10 fois, 0,4 µl d'une solution d'ATP 50 mM, 85 µl d'eau et 1 µl de l'enzyme (à 10 u/µl) et le tout est incubé 4 heures à 37°C puis 5 minutes à 95°C (solution A).
Une solution des oligonucléotides du brin non codant est constituée en mélangeant 1 µl de chaque oligonucléotide (SEQ ID NO:339 à SEQ ID NO:360; Figure 19) et 1 µl de l'oligonucléotide SEQ ID NO :318 (figure 19) dans laquelle on ajoute 106 µl d'eau afin d'obtenir une solution finale à 54 picomoles par µl (solution B).
L'assemblage du gène est réalisé d'abord en mélangeant 10 µl de la solution A, 1 µl de la solution B, 6 µl d'une solution de KCl à 100 mM, 3 µl d'une solution du tensio-actif NP-40 à 0,5%, 4 µl d'une solution de MgCl2 à 50 mM, 3 µl d'une solution d'ATP à 10 mM et 7,5 µl de ligase Pfu (30 unités) puis le mélange est chauffé dans un thermocycleur programmable 3 minutes à 95°C puis 3 minutes à 80°C avant de subir 3 cycles d'une minute à 95°C, suivi d'un passage de 95°C à 70°C en 1 minute, puis d'un passage de 70°C à 55°C en 1 heure et enfin 2 heures à 55°C. Puis le mélange des oligonucléotides assemblés est amplifié avec les amorces NO1 et NO22. Le produit d'amplification est purifié sur colonne Promega, digéré par les enzymes de restriction BspHI et NheI et cloné dans le plasmide pMOD2LacZ(wt) linéarisé par BspHI et NheI. Les séquences de l'ADN plasmidique de 2 clones résistants à la kanamycine, apparus après transformation de la souche d'*E. coli* GT100 (disponible auprès de Invivogen) par le mélange de la ligation entre le fragment vecteur et le fragment PCR, ont été trouvées conformes à la séquence présentée en Figure 18. Ce gène synthétique placé sous la dépendance du promoteur bactérien EM7 (vecteur pMOD2Néo ΔCpG) confère une résistance à la kanamycine identique à celle apportée par le même vecteur contenant le gène natif néo avec la souche *d'E. coli* réceptrice GT100. Le fragment néo BspHI-NheI du plasmide pMOD2Néo ΔCpG a été ensuite introduit dans le plasmide pSh ΔCpG de la figure 16 linéarisé par NcoI-NheI pour donner après ligation et transformation dans *E. coli* le plasmide pNéoΔCpG.

## Revendications

1. Méthode pour la production d'un plasmide qui est vecteur d'au moins un gène, et qui est entièrement dépourvu de CpG, **caractérisée en ce que** l'on construit un plasmide en assemblant par ligation enzymatique des fragments d'ADN tous dépourvus de CpG correspondant à une origine de réplication du plasmide et aux éléments constituant une unité transcriptionnelle pour ledit au moins un gène, et **en ce que** l'on transfère ce plasmide dans une souche d'*Escherichia coli* exprimant la protéine pi pour la réplication du plasmide et **caractérisée en ce que** l'origine de réplication du plasmide est l'origine R6K gamma modifiée par élimination des CpG.

2. Origine de réplication pour plasmide, **caractérisée en ce que** sa séquence correspond à celle de l'origine de réplication R6K gamma dans laquelle chaque G des CpG de la région répétée du core a été remplacé par un A, un C ou un T, ou chaque C des CpG a été remplacé par un G, un A ou un T.

3. Origine de réplication selon la revendication 2, **caractérisée en ce que** sa séquence comprend la séquence SEQ ID NO : 12 ou la séquence SEQ ID NO : 13.

4. Origine de réplication selon la revendication 2 ou 3, **caractérisée en ce que** la séquence de fixation de la protéine pi est répétée 5 ou 6 fois.

5. Méthode selon la revendication 1, **caractérisée en ce que** l'unité transcriptionnelle comprend un promoteur bactérien dépourvu de CpG.

6. Promoteur, **caractérisé en ce que** sa séquence comprend la séquence SEQ ID NO : 11.

7. Méthode selon la revendication 1, **caractérisée en ce que** l'unité transcriptionnelle comprend un terminateur de transcription bactérien dépourvu de CpG.

8. Méthode selon la revendication 1, **caractérisée en ce que** l'unité transcriptionnelle comprend un gène de résistance dépourvu de CpG.

9. Méthode selon la revendication 1, **caractérisée en ce que** l'unité transcriptionnelle comprend un gène rapporteur dépourvu de CpG.

10. Plasmide comprenant une origine de réplication selon l'une quelconque des revendications 2 à 4.

11. Plasmide selon la revendication 10, **caractérisé en ce qu'**il comprend en outre un gène choisi parmi le groupe constitué par :
a) un gène d'au moins 250 pb, susceptible d'être obtenu par une méthode de production d'un gène qui est dépourvu de CpG tout en étant exprimable chez *E. coli*, **caractérisée en ce que** l'on synthétise un enchaînement polynucléotidique en suivant l'enchaînement d'acides aminés d'une protéine qui peut être exprimée par *E. coli,* et en attribuant à chaque acide aminé un codon nucléotidique choisi parmi ceux qui, selon le code génétique, et en tenant compte de la dégénérescence de ce code, correspondent à cet acide aminé tout en éliminant de ce choix :
- les codons ACG (Thr), CCG (Pro), GCG (Ala), TCG (Ser), CGA (Arg), CGC (Arg), CGG (Arg), CGT (Arg) renfermant un CpG, et
- les codons finissant par un C quand le codon qui le suit directement commence par un G ;
b) un gène d'au moins 250 pb, susceptible d'être obtenu par une méthode de production d'un gène qui est dépourvu de CpG tout en étant exprimable chez *E. coli*, **caractérisée en ce que** l'on synthétise un enchaînement polynucléotidique en suivant l'enchaînement d'acides aminés d'une protéine qui peut être exprimée par *E. coli*, et en attribuant à chaque acide aminé un codon nucléotidique choisi parmi ceux qui, selon le code génétique, et en tenant compte de la dégénérescence de ce code, correspondent à cet acide aminé tout en éliminant de ce choix :
- les codons ACG (Thr), CCG (Pro), GCG (Ala), TCG (Ser), CGA (Arg), CGC (Arg), CGG (Arg), CGT (Arg) renfermant un CpG, et
- les codons finissant par un C quand le codon qui le suit directement commence par un G,
la méthode étant **caractérisée par** ce que l'on élimine en outre dudit choix les codons ATA (Ile), CTA (Leu), GTA (Val) et TTA (Leu) ;
c) un gène dont la séquence comprend la séquence SEQ ID NO : 1 de la position 3 à la position 374, la séquence SEQ ID NO : 3 de la position 3 à la position 1025, la séquence SEQ ID NO : 5 de la position 3 à la position 422, la séquence SEQ ID NO : 7 de la position 3 à la position 599 ;
d) un gène dont la séquence comprend la séquence SEQ ID NO : 9 de la position 3 à la position 3056;
e) un gène dont la séquence comprend la séquence SEQ ID NO : 316.

12. Plasmide selon la revendication 10 ou 11, **caractérisé en ce qu'**il comprend en outre un promoteur selon la revendication 6.

13. Plasmide selon l'une quelconque des revendications 10 à 12, **caractérisé en qu'**il comprend en outre un terminateur de transcription sans CpG.

14. Plasmide selon l'une quelconque des revendications 10 à 13 **caractérisé en qu'**il est entièrement dépourvu de CpG.

15. Plasmide de SEQ ID NO : 14.

16. Méthode pour la production d'un plasmide entièrement dépourvu de CpG et exempt de méthylation au niveau de la cytosine dans le contexte nucléique CC (A/T) GG, **caractérisée en ce que** l'on produit un plasmide selon l'une quelconque des revendications 10-15 par réplication dans une souche d'*Escherichia coli* exprimant la protéine pi déficiente pour le système de méthylation dcm.

17. Cellule d'*Escherichia coli* transformée par au moins un des plasmides selon l'une quelconque des revendications 10-15.

18. Cellule d'*Escherichia coli* transformée selon la revendication 17, **caractérisée en ce qu'**elle exprime un gène codant pour une protéine pi.

19. Cellule d'Escherichia coli transformée selon la revendication 18, **caractérisée en ce qu'**elle comprend en outre un gène dcm inactivé.

20. Kit de production de plasmides tels que définis dans l'une quelconque des revendications 10-15, **caractérisé en ce qu'**il comprend au moins une cellule selon l'une quelconque des revendications 17 à 19.

## Claims

1. A method for producing a plasmid which is a vector for at least one gene, and which is completely devoid of CpG, **characterized in that** a plasmid is constructed by assembling, by enzyme ligation, DNA fragments, all devoid of CpG, corresponding to an origin of replication of the plasmid and to the elements constituting a transcriptional unit for said at least one gene, and **in that** this plasmid is transferred into an *Escherichia coli* strain expressing the pi protein for replication of the plasmid, and **characterized in that** the origin of replication of the plasmid is the R6K gamma origin of replication modified by elimination of the CpGs.

2. An origin of replication for a plasmid, **characterized in that** its sequence corresponds to that of the R6K gamma origin of replication in which each G of the GpCs of the repeat region of the core has been replaced with an A, a C or a T, or each C of the CpGs has been replaced with a G, an A or a T.

3. The origin of replication as claimed in claim 2, **characterized in that** its sequence comprises the sequence SEQ ID No. 12 or the sequence SEQ ID No. 13.

4. The origin of replication as claimed in claim 2 or 3, **characterized in that** the pi protein-binding sequence is repeated 5 or 6 times.

5. The method as claimed in claim 1, **characterized in that** the transcriptional unit comprises a bacterial promoter devoid of CpG.

6. A promoter, **characterized in that** its sequence comprises the sequence SEQ ID No. 11.

7. The method as claimed in claim 1, **characterized in that** the transcriptional unit comprises a bacterial transcription terminator devoid of CpG.

8. The method as claimed in claim 1, **characterized in that** the transcriptional unit comprises a resistance gene devoid of CpG.

9. The method as claimed in claim 1, **characterized in that** the transcriptional unit comprises a reporter gene devoid of CpG.

10. A plasmid, comprising an origin of replication as claimed in any one of claims 2 to 4.

11. The plasmid as claimed in claim 10, **characterized in that** it also comprises a gene chosen among the group constituted by:
a) a gene of at least 250 pb, obtainable by a method for the production of a gene which is devoid of CpG while being expressed by *E. coli*, **characterized in that** a polynucleotide chain is synthesized by following the amino acid chain of a protein which can be expressed by *E. coli*, and assigning to each amino acid a nucleotide codon selected from those which, according to the genetic code, and taking into account the degeneracy of this code, correspond to this amino acid, while at the same time eliminating from this selection:
- the codons ACG (Thr), CCG (Pro), GCG (Ala), TCG (Ser), CGA (Arg), CGC (Arg), CGG (Arg), CGT (Arg) containing a CpG, and
- the codons which finish with a C when the codon which directly follows it begins with a G;
b) a gene of at least 250 pb, obtainable by a method for the production of a gene which is devoid of CpG while being expressed by *E. coli*, **characterized in that** a polynucleotide chain is synthesized by following the amino acid chain of a protein which can be expressed by *E. coli*, and assigning to each amino acid a nucleotide codon selected from those which, according to the genetic code, and taking into account the degeneracy of this code, correspond to this amino acid, while at the same time eliminating from this selection:
- the codons ACG (Thr), CCG (Pro), GCG (Ala), TCG (Ser), CGA (Arg), CGC (Arg), CGG (Arg), CGT (Arg) containing a CpG, and
- the codons which finish with a C when the codon which directly follows it begins with a G,
the method being **characterized in** which the codons ATA (Ile), CTA (Leu), GTA (Val) and TTA (Leu) are eliminated from said selection;
c) a gene which sequence comprises the sequence SEQ ID NO:1 from position 3 to position 374, the sequence SEQ ID NO:3 from position 3 to 1025, the sequence SEQ ID NO:5 from position 3 to position 422, the sequence SEQ ID NO:7 from position 3 to position 599;
d) a gene which sequence comprises the sequence SEQ ID NO:9 from position 3 to position 3056;
e) A gene which sequence comprises the sequence SEQ ID NO:316.

12. The plasmid as claimed in claim 10 or 11, **characterized in that** it also comprises a promoter as claimed in claim 6.

13. The plasmid as claimed in any one of claims 10 to 12, **characterized in that** it also comprises a CpG-free transcription terminator.

14. The plasmid as claimed in any one of claims 10 to 13, **characterized in that** it is completely devoid of CpG.

15. A plasmid of SEQ ID No:14.

16. A method for producing a plasmid completely devoid of CpG and free of methylation on cytosine in the nucleic acid context CC(A/T)GG, **characterized in that** a plasmid as claimd in any one of claims 10-15 is produced by replication in an *Escherichia coli* strain expressing the *pi* protein, which is deficient for the *dcm* methylation system.

17. An *Escherichia coli* cell transformed with at least one of the plasmids as claimed in any one of claims 10-15.

18. The transformed *Escherichia coli* cell as claimed in claim 17, **characterized in that** it expresses a gene encoding a pi protein.

19. The transformed *Escherichia coli* cell as claimed in claim 18, **characterized in that** it also comprises an inactivated *dcm* gene.

20. A kit for producing plasmids as defined in any one of clams 10-15, **characterized in that** it comprises at least one cell as claimed in any one of claims 17 to 19.

## Patentansprüche

1. Verfahren zur Herstellung eines Plasmids, das Vektor wenigstens eines Gens ist und das vollständig frei von CpG ist, **dadurch gekennzeichnet, dass** man ein Plasmid konstruiert, indem man durch enzymatische Ligation der DNA-Fragmente, die alle frei von CpG sind, die einem Replikationsursprung und den Elementen, die eine Transkriptionseinheit für das wenigstens eine Gen bilden, entsprechen, zusammenfügt und dass man dieses Plasmid in einen Stamm von Escherichia coli, der das Protein pi exprimiert, für die Replikation des Plasmids transferiert, und **dadurch gekennzeichnet, dass** der Replikationsursprung des Plasmids der Replikationsursprung R6K-gamma, der durch die Eliminierung der CpG modifiziert ist, ist.

2. Replikationsursprung für Plasmid, **dadurch gekennzeichnet, dass** seine Sequenz derjenigen des Replikationsursprungs R6K-gamma entspricht, in dem jedes G der CpG der wiederholten Region des Kerns durch ein A, ein C oder ein T ersetzt wurde, oder jedes C der CpG durch ein G, ein A oder ein T ersetzt wurde.

3. Replikationsursprung nach Anspruch 2, **dadurch gekennzeichnet, dass** seine Sequenz die Sequenz SEQ ID NO:12 oder die Sequeuenz SEQ ID NO:13 umfasst.

4. Replikationsursprung nach Anspruch 2 oder 3, **dadurch gekennzeichnet, dass** die Sequenz zur Fixierung des Proteins pi fünfmal oder sechsmal wiederholt ist.

5. Verfahren nach Anspruch 1, **dadurch gekennzeichnet, dass** die Transkriptionseinheit einen bakteriellen Promotor, der frei von CpG ist, umfasst.

6. Promotor, **dadurch gekennzeichnet, dass** seine Sequenz die SEQ ID NO:11 umfasst.

7. Verfahren nach Anspruch 1, **dadurch gekennzeichnet, dass** die Transkriptionseinheit einen bakteriellen Transkriptionsterminator, der frei von CpG ist, umfasst.

8. Verfahren nach Anspruch 1, **dadurch gekennzeichnet, dass** die Transkriptionseinheit ein Resistenzgen, das frei von CpG ist, umfasst.

9. Verfahren nach Anspruch 1, **dadurch gekennzeichnet, dass** die Transkriptionseinheit ein Reportergen, das frei von CpG ist, umfasst.

10. Plasmid, das einen Replikationsursprung nach einem der Ansprüche 2 bis 4 umfasst.

11. Plasmid nach Anspruch 10, **dadurch gekennzeichnet, dass** es außerdem ein Gen umfasst, ausgewählt aus der Gruppe, bestehend aus:
a) einem Gen aus wenigstens 250 bp, das durch ein Verfahren zur Herstellung eines Gens, das frei von GpG ist, obgleich es in E. coli exprimierbar ist, erhalten werden kann, **dadurch gekennzeichnet, dass** man eine Polynukleotidkette synthetisiert, indem man der Aminosäurekette eines Proteins folgt, das durch E. coli exprimiert werden kann, und indem man jeder Aminosäure einen Nukleotid-Codon zuordnet, ausgewählt unter denen, die entsprechend dem genetischen Code und unter Berücksichtigung der Degeneriertheit dieses Codes dieser Aminosäure entsprechen, indem man jedoch von dieser Wahl eliminiert:
- die Codons ACG (Thr), CCG (Pro), GCG (Ala), TCG (Ser), CGA (Arg), CGC (Arg), CGG (Arg), CGT (Arg), die ein CpG umfassen, und
- die Codons, die mit einem C enden, wenn das Codon, das direkt darauf folgt, mit einem G beginnt;
b) einem Gen mit wenigstens 250 bp, das durch ein Verfahren zur Herstellung eines Gens, das frei von CpG ist, jedoch in E. coli exprimierbar ist, erhalten werden kann, **dadurch gekennzeichnet, dass** man eine Polynukleotidkette synthetisiert, indem man der Aminosäurekette eines Proteins, das durch E. coli exprimiert werden kann, folgt und indem man jeder Aminosäure ein Nukleotid-Codon zuordnet, ausgewählt unter denen, die nach dem genetischen Code und unter Berücksichtigung der Degeneriertheit dieses Codes dieser Aminosäure entsprechen, indem man jedoch von dieser Wahl eliminiert:
- die Codons AGC (Thr), CCG (Pro), GCG (Ala), TCG (Ser), CGA (Arg), CGC (Arg), CGG (Arg), CGT (Arg), die ein CpG umfassen, und
- die Codons, die mit einem C enden, wenn das Codon, das direkt darauf folgt, mit einem G beginnt,
wobei das Verfahren **dadurch gekennzeichnet, ist, dass** man außerdem von der Wahl die Codons ATA (Ile), CTA (Leu), GTA (Val) und TTA (Leu) eliminiert;
c) einem Gen, dessen Sequenz die Sequenz SEQ ID NO:1 von der Position 3 bis zu der Position 374, die Sequenz SEQ ID NO:3 von der Position 3 bis zu der Position 1025, die Sequenz SEQ ID NO:5 von der Position 3 bis zu der Position 422, die Sequenz SEQ ID NO:7 von der Position 7 bis zu der Position 599 umfasst;
d) einem Gen, dessen Sequenz der Sequenz SEQ ID NO:9 von der Position 3 bis zu der Position 3056 entspricht; und
e) einem Gen, dessen Sequenz der Sequenz SEQ ID NO:316 entspricht.

12. Plasmid nach Anspruch 10 oder 11, **dadurch gekennzeichnet, dass** es außerdem einen Promotor nach Anspruch 7 umfasst.

13. Plasmid nach einem der Ansprüche 10 bis 12, **dadurch gekennzeichnet, dass** es außerdem einen Transkriptionsterminator ohne CpG umfasst.

14. Plasmid nach einem der Ansprüche 10 bis 13, **dadurch gekennzeichnet, dass** es vollständig frei von CpG ist.

15. Plasmid der SEQ ID NO:14.

16. Verfahren für die Herstellung eines Plasmids, das vollständig frei von CpG ist und ohne Methylierung auf der Ebene des Cytosins im Nukleinsäure-Kontext CC(A/T)GG ist, **dadurch gekennzeichnet, dass** man ein Plasmid nach einem der Ansprüche 10 bis 15 durch Replikation in einem Escherichia coli-Stamm produziert, der das Protein pi, das bezüglich des Methylierungssystems dcm defizient ist, exprimiert.

17. Escherichia coli-Zelle, transformiert durch wenigstens eines der Plasmide nach einem der Ansprüche 10 bis 15.

18. Transformierte Escherichia coli-Zelle nach Anspruch 17, **dadurch gekennzeichnet, dass** sie ein Gen exprimiert, das für ein Protein pi codiert.

19. Transformierte Escherichia coli-Zelle nach Anspruch 18, **dadurch gekennzeichnet, dass** sie außerdem ein inaktiviertes dcm-Gen umfasst.

20. Kit zur Herstellung von Plasmiden, wie sie in einem der Ansprüche 10 bis 15 definiert sind, **dadurch gekennzeichnet, dass** er wenigstens eine Zelle nach einem der Ansprüche 17 bis 19 umfasst.


### SEQUENCE LISTING

<110> CAYLA
<120> SYNTHETIC GENES AND BACTERIAL PLASMIDS DEVOID OF CpG
<130> FP - D. 0200862
<140> PCT/FR02/00862
   <141> 2002-03-11
<150> FR01/03274
   <151> 2001-03-09
<160> 360
<170> PatentIn version 3.1
<210> 1
   <211> 396
   <212> DNA
   <213> Artificial Sequence
<220>
   <223> CpG-Free shable
   <220>
   <221> CDS
   <222> (3)..(374)
   <223>
<400> 1
   cc atg gcc aag ttg acc agt gct gtc cca gtg ctc aca gcc agg gat      47
      Met Ala Lys Leu Thr Ser Ala Val Pro Val Leu Thr Ala Arg Asp
      1               5                   10                  15
   gtg gct gga gct gtt gag ttc tgg act gac agg ttg ggg ttc tcc aga      95
   Val Ala Gly Ala Val Glu Phe Trp Thr Asp Arg Leu Gly Phe Ser Arg
                   20                  25                  30
   gat ttt gtg gag gat gac ttt gca ggt gtg gtc aga gat gat gtc acc     143
   Asp Phe Val Glu Asp Asp Phe Ala Gly Val Val Arg Asp Asp Val Thr
               35                  40                  45
   ctg ttc atc tca gca gtc cag gac cag gtg gtg cct gac aac acc ctg     191
   Leu Phe Ile Ser Ala Val Gln Asp Gln Val Val Pro Asp Asn Thr Leu
           50                  55                  60
   gct tgg gtg tgg gtg aga gga ctg gat gag ctg tat gct gag tgg agt     239
   Ala Trp Val Trp Val Arg Gly Leu Asp Glu Leu Tyr Ala Glu Trp Ser
       65                  70                  75
   gag gtg gtc tcc acc aac ttc agg gat gcc agt ggc cct gcc atg aca     287
   Glu Val Val Ser Thr Asn Phe Arg Asp Ala Ser Gly Pro Ala Met Thr
   80                  85                  90                  95
   gag att gga gag cag ccc tgg ggg aga gag ttt gcc ctg aga gac cca     335
   Glu Ile Gly Glu Gln Pro Trp Gly Arg Glu Phe Ala Leu Arg Asp Pro
                   100                 105                 110
   gca ggc aac tgt gtg cac ttt gtg gca gag gag cag gac tgaggataag      384
   Ala Gly Asn Cys Val His Phe Val Ala Glu Glu Gln Asp
               115                 120
   aattcagcta gc                                                       396
<210> 2
   <211> 124
   <212> PRT
   <213> Artificial Sequence
<220>
   <223> CpG-Free sh ble
   <400> 2
   Met Ala Lys Leu Thr Ser Ala Val Pro Val Leu Thr Ala Arg Asp Val
   1               5                   10                  15
   Ala Gly Ala Val Glu Phe Trp Thr Asp Arg Leu Gly Phe Ser Arg Asp
               20                  25                  30
   Phe Val Glu Asp Asp Phe Ala Gly Val Val Arg Asp Asp Val Thr Leu
           35                  40                  45
   Phe Ile Ser Ala Val Gln Asp Gln Val Val Pro Asp Asn Thr Leu Ala
       50                  55                  60
   Trp Val Trp Val Arg Gly Leu Asp Glu Leu Tyr Ala Glu Trp Ser Glu
   65                  70                  75                  80
   Val Val Ser Thr Asn Phe Arg Asp Ala Ser Gly Pro Ala Met Thr Glu
                   85                  90                  95
   Ile Gly Glu Gln Pro Trp Gly Arg Glu Phe Ala Leu Arg Asp Pro Ala
               100                 105                 110
   Gly Asn Cys Val His Phe Val Ala Glu Glu Gln Asp
           115                 120
<210> 3
   <211> 1040
   <212> DNA
   <213> Artificial sequence
<220>
   <223> CpG-Free Hph
   <220>
   <221> CDS
   <222> (3)..(1025)
   <223>
<400> 3
   tc atg aag aaa cct gaa ctg aca gca act tct gtt gag aag ttt ctc     47
      Met Lys Lys Pro Glu Leu Thr Ala Thr Ser Val Glu Lys Phe Leu
      1               5                   10                  15
   att gaa aaa ttt gat tct gtt tct gat ctc atg cag ctg tct gaa ggt    95
   Ile Glu Lys Phe Asp Ser Val Ser Asp Leu Met Gln Leu Ser Glu Gly
                   20                  25                  30
   gaa gaa agc aga gcc ttt tct ttt gat gtt gga gga aga ggt tat gtt   143
   Glu Glu Ser Arg Ala Phe Ser Phe Asp Val Gly Gly Arg Gly Tyr Val
               35                  40                  45
   ctg agg gtc aat tct tgt gct gat ggt ttt tac aaa gac aga tat gtt   191
   Leu Arg Val Asn Ser Cys Ala Asp Gly Phe Tyr Lys Asp Arg Tyr Val
           50                  55                  60
   tac aga cac ttt gcc tct gct gct ctg cca att cca gaa gtt ctg gac   239
   Tyr Arg His Phe Ala Ser Ala Ala Leu Pro Ile Pro Glu Val Leu Asp
       65                  70                  75
   att gga gaa ttt tct gaa tct ctc acc tac tgc atc agc aga aga gca   287
   Ile Gly Glu Phe Ser Glu Ser Leu Thr Tyr Cys Ile Ser Arg Arg Ala
   80                  85                  90                  95
   caa gga gtc act ctc cag gat ctc cct gaa act gag ctg cca gct gtt   335
   Gln Gly Val Thr Leu Gln Asp Leu Pro Glu Thr Glu Leu Pro Ala Val
                   100                 105                 110
   ctg caa cct gtt gct gaa gca atg gat gcc att gca gca gct gat ctg   383
   Leu Gln Pro Val Ala Glu Ala Met Asp Ala Ile Ala Ala Ala Asp Leu
               115                 120                 125
   agc caa acc tct gga ttt ggt cct ttt ggt ccc caa ggc att ggt cag   431
   Ser Gln Thr Ser Gly Phe Gly Pro Phe Gly Pro Gln Gly Ile Gly Gln
           130                 135                 140
   tac acc act tgg agg gat ttc att tgt gcc att gct gat cct cat gtc      479
   Tyr Thr Thr Trp Arg Asp Phe Ile Cys Ala Ile Ala Asp Pro His Val
       145                 150                     155
   tat cac tgg cag act gtg atg gat gac aca gtt tct gct tct gtt gct      527
   Tyr His Trp Gln Thr Val Met Asp Asp Thr Val Ser Ala Ser Val Ala
   160                 165                     170                 175
   cag gca ctg gat gaa ctc atg ctg tgg gca gaa gat tgt cct gaa gtc      575
   Gln Ala Leu Asp Glu Leu Met Leu Trp Ala Glu Asp Cys Pro Glu Val
                   180                     185                 190
   aga cac ctg gtc cat gct gat ttt gga agc aac aat gtt ctg aca gac      623
   Arg His Leu Val His Ala Asp Phe Gly Ser Asn Asn Val Leu Thr Asp
               195                     200                 205
   aat ggc aga atc act gca gtc att gac tgg tct gaa gcc atg ttt gga      671
   Asn Gly Arg Ile Thr Ala Val Ile Asp Trp Ser Glu Ala Met Phe Gly
           210                 215                     220
   gat tct caa tat gag gtt gcc aac att ttt ttt tgg aga cct tgg ctg      719
   Asp Ser Gln Tyr Glu Val Ala Asn Ile Phe Phe Trp Arg Pro Trp Leu
       225                 230                     235
   gct tgc atg gaa caa caa aca aga tat ttt gaa aga aga cac cca gaa      767
   Ala Cys Met Glu Gln Gln Thr Arg Tyr Phe Glu Arg Arg His Pro Glu
   240                 245                     250                 255
   ctg gct ggt tcc ccc aga ctg aga gcc tac atg ctc aga att ggc ctg      815
   Leu Ala Gly Ser Pro Arg Leu Arg Ala Tyr Met Leu Arg Ile Gly Leu
                   260                     265                 270
   gac caa ctg tat caa tct ctg gtt gat gga aac ttt gat gat gct gct      863
   Asp Gln Leu Tyr Gln Ser Leu Val Asp Gly Asn Phe Asp Asp Ala Ala
               275                     280                 285
   tgg gca caa gga aga tgt gat gcc att gtg agg tct ggt gct gga act      911
   Trp Ala Gln Gly Arg Cys Asp Ala Ile Val Arg Ser Gly Ala Gly Thr
           290                 295                     300
   gtt gga aga act caa att gca aga agg tct gct gct gtt tgg act gat      959
   Val Gly Arg Thr Gln Ile Ala Arg Arg Ser Ala Ala Val Trp Thr Asp
       305                 310                     315
   gga tgt gtt gaa gtt ctg gct gac tct gga aac agg aga ccc tcc aca     1007
   Gly Cys Val Glu Val Leu Ala Asp Ser Gly Asn Arg Arg Pro Ser Thr
   320                 325                     330                 335
   aga ccc aga gcc aag gaa tgaatattag ctagc                            1040
   Arg Pro Arg Ala Lys Glu
                   340
<210> 4
   <211> 341
   <212> PRT
   <213> Artificial Sequence
<220>
   <223> CpG-Free Hph
<400> 4
   Met Lys Lys Pro Glu Leu Thr Ala Thr Ser Val Glu Lys Phe Leu Ile
   1               5                   10                  15
   Glu Lys Phe Asp Ser Val Ser Asp Leu Met Gln Leu Ser Glu Gly Glu
               20                  25                  30
   Glu Ser Arg Ala Phe Ser Phe Asp Val Gly Gly Arg Gly Tyr Val Leu
           35                  40                  45
   Arg Val Asn Ser Cys Ala Asp Gly Phe Tyr Lys Asp Arg Tyr Val Tyr
       50                  55                  60
   Arg His Phe Ala Ser Ala Ala Leu Pro Ile Pro Glu Val Leu Asp Ile
   65                  70                  75                  80
   Gly Glu Phe Ser Glu Ser Leu Thr Tyr Cys Ile Ser Arg Arg Ala Gln
                   85                  90                  95
   Gly Val Thr Leu Gln Asp Leu Pro Glu Thr Glu Leu Pro Ala Val Leu
               100                 105                 110
   Gln Pro Val Ala Glu Ala Met Asp Ala Ile Ala Ala Ala Asp Leu Ser
           115                 120                 125
   Gln Thr Ser Gly Phe Gly Pro Phe Gly Pro Gln Gly Ile Gly Gln Tyr
       130                 135                 140
   Thr Thr Trp Arg Asp Phe Ile Cys Ala Ile Ala Asp Pro His Val Tyr
   145                 150                 155                 160
   His Trp Gln Thr Val Met Asp Asp Thr Val Ser Ala Ser Val Ala Gln
                   165                 170                 175
   Ala Leu Asp Glu Leu Met Leu Trp Ala Glu Asp Cys Pro Glu Val Arg
               180                 185                 190
   His Leu Val His Ala Asp Phe Gly Ser Asn Asn Val Leu Thr Asp Asn
           195                 200                 205
   Gly Arg Ile Thr Ala Val Ile Asp Trp Ser Glu Ala Met Phe Gly Asp
       210                 215                 220
   Ser Gln Tyr Glu Val Ala Asn Ile Phe Phe Trp Arg Pro Trp Leu Ala
   225                 230                 235                 240
   Cys Met Glu Gln Gln Thr Arg Tyr Phe Glu Arg Arg His Pro Glu Leu
                   245                 250                 255
   Ala Gly Ser Pro Arg Leu Arg Ala Tyr Met Leu Arg Ile Gly Leu Asp
               260                 265                 270
   Gln Leu Tyr Gln Ser Leu Val Asp Gly Asn Phe Asp Asp Ala Ala Trp
           275                 280                 285
   Ala Gln Gly Arg Cys Asp Ala Ile Val Arg Ser Gly Ala Gly Thr Val
       290                 295                 300
   Gly Arg Thr Gln Ile Ala Arg Arg Ser Ala Ala Val Trp Thr Asp Gly
   305                 310                 315                 320
   Cys Val Glu Val Leu Ala Asp Ser Gly Asn Arg Arg Pro Ser Thr Arg
                   325                 330                 335
   Pro Arg Ala Lys Glu
               340
<210> 5
   <211> 442
   <212> DNA
   <213> Artificial Sequence
<220>
   <223> CpG-Free Bsr
   <220>
   <221> CDS
   <222> (3)..(422)
   <223>
<400> 5
   tc atg aag acc ttc aac atc tct cag cag gat ctg gag ctg gtg gag       47
      Met Lys Thr Phe Asn Ile Ser Gln Gln Asp Leu Glu Leu Val Glu
      1               5                   10                  15
   gtc gcc act gag aag atc acc atg ctc tat gag gac aac aag cac cat      95
   Val Ala Thr Glu Lys Ile Thr Met Leu Tyr Glu Asp Asn Lys His His
                   20                  25                  30
   gtc ggg gcg gcc atc agg acc aag act ggg gag atc atc tct gct gtc     143
   Val Gly Ala Ala Ile Arg Thr Lys Thr Gly Glu Ile Ile Ser Ala Val
               35                  40                  45
   cac att gag gcc tac att ggc agg gtc act gtc tgt gct gaa gcc att     191
   His Ile Glu Ala Tyr Ile Gly Arg Val Thr Val Cys Ala Glu Ala Ile
           50                  55                  60
   gcc att ggg tct gct gtg agc aac ggg cag aag gac ttt gac acc att     239
   Ala Ile Gly Ser Ala Val Ser Asn Gly Gln Lys Asp Phe Asp Thr Ile
       65                  70                  75
   gtg gct gtc agg cac ccc tac tct gat gag gtg gac aga tcc atc agg     287
   Val Ala Val Arg His Pro Tyr Ser Asp Glu Val Asp Arg Ser Ile Arg
   80 85 90 95
   gtg gtc agc ccc tgt ggc atg tgc aga gag ctc atc tct gac tat gct 335
   Val Val Ser Pro Cys Gly Met Cys Arg Glu Leu Ile Ser Asp Tyr Ala
   100 105 110
   cct gac tgc ttt gtg ctc att gag atg aat ggc aag ctg gtc aaa acc 383
   Pro Asp Cys Phe Val Leu Ile Glu Met Asn Gly Lys Leu Val Lys Thr
   115 120 125
   acc att gag gaa ctc atc ccc ctc aag tac acc agg aac taaacctgaa 432
   Thr Ile Glu Glu Leu Ile Pro Leu Lys Tyr Thr Arg Asn
   130 135 140
   ttcagctagc 442
<210> 6
   <211> 140
   <212> PRT
   <213> Artificial Sequence
<220>
   <223> cpG-Free Bsr
   <400> 6
   Met Lys Thr Phe Asn Ile Ser Gln Gln Asp Leu Glu Leu Val Glu Val
   1 5 10 15
   Ala Thr Glu Lys Ile Thr Met Leu Tyr Glu Asp Asn Lys His His Val
   20 25 30
   Gly Ala Ala Ile Arg Thr Lys Thr Gly Glu Ile Ile Ser Ala Val His
   35 40 45
   Ile Glu Ala Tyr Ile Gly Arg Val Thr Val Cys Ala Glu Ala Ile Ala
   50 55 60
   Ile Gly Ser Ala Val Ser Asn Gly Gln Lys Asp Phe Asp Thr Ile Val
   65 70 75 80
   Ala Val Arg His Pro Tyr Ser Asp Glu Val Asp Arg Ser Ile Arg Val
   85 90 95
   Val Ser Pro Cys Gly Met Cys Arg Glu Leu Ile Ser Asp Tyr Ala Pro
   100 105 110
   Asp Cys Phe Val Leu Ile Glu Met Asn Gly Lys Leu Val Lys Thr Thr
   115 120 125
   Ile Glu Glu Leu Ile Pro Leu Lys Tyr Thr Arg Asn
   130 135 140
<210> 7
   <211> 614
   <212> DNA
   <213> Artificial Sequence

<220>
   <223> CpG-Free Pac
   <220>
   <221> CDS
   <222> (3)..(599)
   <223>
<400> 7
   tc atg act gag tac aaa ccc aca gtg agg ctg gca acc aga gat gat      47
      Met Thr Glu Tyr Lys Pro Thr Val Arg Leu Ala Thr Arg Asp Asp
      1               5                   10                  15
   gtt cca aga gct gtg aga aca ctg gct gct gct ttt gca gac tac cct     95
   Val Pro Arg Ala Val Arg Thr Leu Ala Ala Ala Phe Ala Asp Tyr Pro
                   20                  25                  30
   gca aca agg cac aca gtt gac cct gac agg cac att gag agg gtg aca    143
   Ala Thr Arg His Thr Val Asp Pro Asp Arg His Ile Glu Arg Val Thr
               35                  40                  45
   gaa ctg caa gaa ctc ttc ctc acc aga gtg gga ctg gac att gga aaa    191
   Glu Leu Gln Glu Leu Phe Leu Thr Arg Val Gly Leu Asp Ile Gly Lys
           50                  55                  60
   gtt tgg gtt gca gat gat gga gct gct gtt gca gtt tgg aca aca cct    239
   Val Trp Val Ala Asp Asp Gly Ala Ala Val Ala Val Trp Thr Thr Pro
       65                  70                  75
   gag tct gtt gaa gct ggt gct gtt ttt gct gaa att gga cca aga atg    287
   Glu Ser Val Glu Ala Gly Ala Val Phe Ala Glu Ile Gly Pro Arg Met
   80                  85                  90                  95
   gct gag ctc tct gga agc agg ctg gca gca caa caa caa atg gaa ggt    335
   Ala Glu Leu Ser Gly Ser Arg Leu Ala Ala Gln Gln Gln Met Glu Gly
                   100                 105                 110
   ctg ctg gca cca cac agg cca aaa gag cca gct tgg ttt ctg gca act    383
   Leu Leu Ala Pro His Arg Pro Lys Glu Pro Ala Trp Phe Leu Ala Thr
               115                 120                 125
   gtt gga gtg agc cct gac cac cag gga aag ggt ctg gga tct gct gtt    431
   Val Gly Val Ser Pro Asp His Gln Gly Lys Gly Leu Gly Ser Ala Val
           130                 135                 140
   gtt ctg cct gga gtt gaa gct gct gaa agg gct gga gtt cct gcc ttt    479
   Val Leu Pro Gly Val Glu Ala Ala Glu Arg Ala Gly Val Pro Ala Phe
       145                 150                 155
   ctg gaa act tct gct ccc aga aac ctg cct ttt tat gaa aga ctg gga    527
   Leu Glu Thr Ser Ala Pro Arg Asn Leu Pro Phe Tyr Glu Arg Leu Gly
   160                 165                 170                 175
   ttc act gtg aca gct gat gtt gag gtt cca gaa ggc cca aga act tgg    575
   Phe Thr Val Thr Ala Asp Val Glu Val Pro Glu Gly Pro Arg Thr Trp
                   180                 185                 190
   tgc atg aca agg aag cct gga gct taaacctgag ctagc                   614
   Cys Met Thr Arg Lys Pro Gly Ala
               195
<210> 8
   <211> 199
   <212> PRT
   <213> Artificial Sequence
<220>
   <223> CpG-Free Pac
   <400> 8
   Met Thr Glu Tyr Lys Pro Thr Val Arg Leu Ala Thr Arg Asp Asp Val
   1 5 10 15
   Pro Arg Ala Val Arg Thr Leu Ala Ala Ala Phe Ala Asp Tyr Pro Ala
   20 25 30
   Thr Arg His Thr Val Asp Pro Asp Arg His Ile Glu Arg Val Thr Glu
   35 40 45
   Leu Gln Glu Leu Phe Leu Thr Arg Val Gly Leu Asp Ile Gly Lys Val
   50 55 60
   Trp Val Ala Asp Asp Gly Ala Ala Val Ala Val Trp Thr Thr Pro Glu
   65 70 75 80
   Ser Val Glu Ala Gly Ala Val Phe Ala Glu Ile Gly Pro Arg Met Ala
   85 90 95
   Glu Leu Ser Gly Ser Arg Leu Ala Ala Gln Gln Gln Met Glu Gly Leu
   100 105 110
   Leu Ala Pro His Arg Pro Lys Glu Pro Ala Trp Phe Leu Ala Thr Val
   115 120 125
   Gly Val Ser Pro Asp His Gln Gly Lys Gly Leu Gly Ser Ala Val Val
   130 135 140
   Leu Pro Gly Val Glu Ala Ala Glu Arg Ala Gly Val Pro Ala Phe Leu
   145 150 155 160
   Glu Thr Ser Ala Pro Arg Asn Leu Pro Phe Tyr Glu Arg Leu Gly Phe
   165 170 175
   Thr Val Thr Ala Asp Val Glu Val Pro Glu Gly Pro Arg Thr Trp Cys
   180 185 190
   Met Thr Arg Lys Pro Gly Ala
   195
<210> 9
   <211> 3071
   <212> DNA
   <213> Artificial Sequence
<220>
   <223> CpG-Free Lacz
   <220>
   <221> CDS
   <222> (3)..(3056)
   <223>
<400> 9
   cc atg gac cct gtt gtg ctg caa agg aga gac tgg gag aac cct gga       47
      Met Asp Pro Val Val Leu Gln Arg Arg Asp Trp Glu Asn Pro Gly
      1               5                   10                  15
   gtg acc cag ctc aac aga ctg gct gcc cac cct ccc ttt gcc tct tgg      95
   Val Thr Gln Leu Asn Arg Leu Ala Ala His Pro Pro Phe Ala Ser Trp
                   20                  25                  30
   agg aac tct gag gaa gcc agg aca gac agg ccc agc cag cag ctc agg     143
   Arg Asn Ser Glu Glu Ala Arg Thr Asp Arg Pro Ser Gln Gln Leu Arg
               35                  40                  45
   tct ctc aat gga gag tgg agg ttt gcc tgg ttc cct gcc cct gaa gct     191
   Ser Leu Asn Gly Glu Trp Arg Phe Ala Trp Phe Pro Ala Pro Glu Ala
           50                  55                  60
   gtg cct gag tct tgg ctg gag tgt gac ctc cca gag gct gac act gtt     239
   Val Pro Glu Ser Trp Leu Glu Cys Asp Leu Pro Glu Ala Asp Thr Val
       65                  70                  75
   gtg gtg ccc agc aac tgg cag atg cat ggc tat gat gcc ccc atc tac     287
   Val Val Pro Ser Asn Trp Gln Met His Gly Tyr Asp Ala Pro Ile Tyr
   80                  85                  90                  95
   acc aat gtc acc tac ccc atc act gtg aac ccc cct ttt gtg ccc act     335
   Thr Asn Val Thr Tyr Pro Ile Thr Val Asn Pro Pro Phe Val Pro Thr
                   100                 105                 110
   gag aac ccc act ggc tgc tac agc ctg acc ttc aat gtt gat gag agc     383
   Glu Asn Pro Thr Gly Cys Tyr Ser Leu Thr Phe Asn Val Asp Glu Ser
               115                 120                 125
   tgg ctg caa gaa ggc cag acc agg atc atc ttt gat gga gtc aac tct 431
   Trp Leu Gln Glu Gly Gln Thr Arg Ile Ile Phe Asp Gly Val Asn Ser
   130 135 140
   gcc ttc cac ctc tgg tgc aat ggc agg tgg gtt ggc tat ggc caa gac 479
   Ala Phe His Leu Trp Cys Asn Gly Arg Trp Val Gly Tyr Gly Gln Asp
   145 150 155
   agc agg ctg ccc tct gag ttt gac ctc tct gcc ttc ctc aga gct gga 527
   Ser Arg Leu Pro Ser Glu Phe Asp Leu Ser Ala Phe Leu Arg Ala Gly
   160 165 170 175
   gag aac agg ctg gct gtc atg gtg ctc agg tgg tct gat ggc agc tac 575
   Glu Asn Arg Leu Ala Val Met Val Leu Arg Trp Ser Asp Gly Ser Tyr
   180 185 190
   ctg gaa gac caa gac atg tgg agg atg tct ggc atc ttc agg gat gtg 623
   Leu Glu Asp Gln Asp Met Trp Arg Met Ser Gly Ile Phe Arg Asp Val
   195 200 205
   agc ctg ctg cac aag ccc acc acc cag att tct gac ttc cat gtt gcc 671
   Ser Leu Leu His Lys Pro Thr Thr Gln Ile Ser Asp Phe His Val Ala
   210 215 220
   acc agg ttc aat gat gac ttc agc aga gct gtg ctg gag gct gag gtg 719
   Thr Arg Phe Asn Asp Asp Phe Ser Arg Ala Val Leu Glu Ala Glu Val
   225 230 235
   cag atg tgt gga gaa ctc aga gac tac ctg aga gtc aca gtg agc ctc 767
   Gln Met Cys Gly Glu Leu Arg Asp Tyr Leu Arg Val Thr Val Ser Leu
   240 245 250 255
   tgg caa ggt gag acc cag gtg gcc tct ggc aca gcc ccc ttt gga gga 815
   Trp Gln Gly Glu Thr Gln Val Ala Ser Gly Thr Ala Pro Phe Gly Gly
   260 265 270
   gag atc att gat gag aga gga ggc tat gct gac aga gtc acc ctg agg 863
   Glu Ile Ile Asp Glu Arg Gly Gly Tyr Ala Asp Arg Val Thr Leu Arg
   275 280 285
   ctc aat gtg gag aac ccc aag ctg tgg tct gct gag atc ccc aac ctc 911
   Leu Asn Val Glu Asn Pro Lys Leu Trp Ser Ala Glu Ile Pro Asn Leu
   290 295 300
   tac agg gct gtt gtg gag ctg cac act gct gat ggc acc ctg att gaa 959
   Tyr Arg Ala Val Val Glu Leu His Thr Ala Asp Gly Thr Leu Ile Glu
   305 310 315
   gct gaa gcc tgt gat gtt gga ttc aga gaa gtc agg att gag aat ggc 1007
   Ala Glu Ala Cys Asp Val Gly Phe Arg Glu Val Arg Ile Glu Asn Gly
   320 325 330 335
   ctg ctg ctg ctc aat ggc aag cct ctg ctc atc agg gga gtc aac agg 1055
   Leu Leu Leu Leu Asn Gly Lys Pro Leu Leu Ile Arg Gly Val Asn Arg
   340 345 350
   cat gag cac cac cct ctg cat gga caa gtg atg gat gaa cag aca atg 1103
   His Glu His His Pro Leu His Gly Gln Val Met Asp Glu Gln Thr Met
   355 360 365
   gtg caa gat atc ctg cta atg aag cag aac aac ttc aat gct gtc agg 1151
   Val Gln Asp Ile Leu Leu Met Lys Gln Asn Asn Phe Asn Ala Val Arg
   370 375 380
   tgc tct cac tac ccc aac cac cct ctc tgg tac acc ctg tgt gac agg 1199
   Cys Ser His Tyr Pro Asn His Pro Leu Trp Tyr Thr Leu Cys Asp Arg
   385 390 395
   tat ggc ctg tat gtt gtt gat gaa gcc aac att gag aca cat ggc atg      1247
   Tyr Gly Leu Tyr Val Val Asp Glu Ala Asn Ile Glu Thr His Gly Met
   400                 405                 410                 415
   gtg ccc atg aac agg ctc aca gat gac ccc agg tgg ctg cct gcc atg      1295
   Val Pro Met Asn Arg Leu Thr Asp Asp Pro Arg Trp Leu Pro Ala Met
                   420                 425                 430
   tct gag aga gtg acc agg atg gtg cag aga gac agg aac cac ccc tct      1343
   Ser Glu Arg Val Thr Arg Met Val Gln Arg Asp Arg Asn His Pro Ser
               435                 440                 445
   gtg atc atc tgg tct ctg ggc aat gag tct gga cat gga gcc aac cat      1391
   Val Ile Ile Trp Ser Leu Gly Asn Glu Ser Gly His Gly Ala Asn His
           450                 455                 460
   gat gct ctc tac agg tgg atc aag tct gtt gac ccc agc aga cct gtg      1439
   Asp Ala Leu Tyr Arg Trp Ile Lys Ser Val Asp Pro Ser Arg Pro Val
       465                 470                 475
   cag tat gaa gga ggt gga gca gac acc aca gcc aca gac atc atc tgc      1487
   Gln Tyr Glu Gly Gly Gly Ala Asp Thr Thr Ala Thr Asp Ile Ile Cys
   480                 485                 490                 495
   ccc atg tat gcc agg gtt gat gag gac cag ccc ttc cct gct gtg ccc      1535
   Pro Met Tyr Ala Arg Val Asp Glu Asp Gln Pro Phe Pro Ala Val Pro
                   500                 505                 510
   aag tgg agc atc aag aag tgg ctc tct ctg cct gga gag acc aga cct      1583
   Lys Trp Ser Ile Lys Lys Trp Leu Ser Leu Pro Gly Glu Thr Arg Pro
               515                 520                 525
   ctg atc ctg tgt gaa tat gca cat gca atg ggc aac tct ctg gga ggc      1631
   Leu Ile Leu Cys Glu Tyr Ala His Ala Met Gly Asn Ser Leu Gly Gly
           530                 535                 540
   ttt gcc aag tac tgg caa gcc ttc aga cag tac ccc agg ctg caa gga      1679
   Phe Ala Lys Tyr Trp Gln Ala Phe Arg Gln Tyr Pro Arg Leu Gln Gly
       545                 550                 555
   gga ttt gtg tgg gac tgg gtg gac caa tct ctc atc aag tat gat gag      1727
   Gly Phe Val Trp Asp Trp Val Asp Gln Ser Leu Ile Lys Tyr Asp Glu
   560                 565                 570                 575
   aat ggc aac ccc tgg tct gcc tat gga gga gac ttt ggt gac acc ccc      1775
   Asn Gly Asn Pro Trp Ser Ala Tyr Gly Gly Asp Phe Gly Asp Thr Pro
                   580                 585                 590
   aat gac agg cag ttc tgc atg aat ggc ctg gtc ttt gca gac agg acc      1823
   Asn Asp Arg Gln Phe Cys Met Asn Gly Leu Val Phe Ala Asp Arg Thr
               595                 600                 605
   cct cac cct gcc ctc aca gag gcc aag cac cag caa cag ttc ttc cag      1871
   Pro His Pro Ala Leu Thr Glu Ala Lys His Gln Gln Gln Phe Phe Gln
           610                 615                 620
   ttc agg ctg tct gga cag acc att gag gtg aca tct gag tac ctc ttc      1919
   Phe Arg Leu Ser Gly Gln Thr Ile Glu Val Thr Ser Glu Tyr Leu Phe
       625                 630                 635
   agg cac tct gac aat gag ctc ctg cac tgg atg gtg gcc ctg gat ggc      1967
   Arg His Ser Asp Asn Glu Leu Leu His Trp Met Val Ala Leu Asp Gly
   640                 645                 650                 655
   aag cct ctg gct tct ggt gag gtg cct ctg gat gtg gcc cct caa gga      2015
   Lys Pro Leu Ala Ser Gly Glu Val Pro Leu Asp Val Ala Pro Gln Gly
                   660                 665                 670
   aag cag ctg att gaa ctg cct gag ctg cct cag cca gag tct gct gga     2063
   Lys Gln Leu Ile Glu Leu Pro Glu Leu Pro Gln Pro Glu Ser Ala Gly
               675                 680                 685
   caa ctg tgg cta aca gtg agg gtg gtt cag ccc aat gca aca gct tgg     2111
   Gln Leu Trp Leu Thr Val Arg Val Val Gln Pro Asn Ala Thr Ala Trp
           690                 695                 700
   tct gag gca ggc cac atc tct gca tgg cag cag tgg agg ctg gct gag     2159
   Ser Glu Ala Gly His Ile Ser Ala Trp Gln Gln Trp Arg Leu Ala Glu
       705                 710                 715
   aac ctc tct gtg acc ctg cct gct gcc tct cat gcc atc cct cac ctg     2207
   Asn Leu Ser Val Thr Leu Pro Ala Ala Ser His Ala Ile Pro His Leu
   720                 725                 730                 735
   aca aca tct gaa atg gac ttc tgc att gag ctg ggc aac aag aga tgg     2255
   Thr Thr Ser Glu Met Asp Phe Cys Ile Glu Leu Gly Asn Lys Arg Trp
                   740                 745                 750
   cag ttc aac agg cag tct ggc ttc ctg tct cag atg tgg att gga gac     2303
   Gln Phe Asn Arg Gln Ser Gly Phe Leu Ser Gln Met Trp Ile Gly Asp
               755                 760                 765
   aag aag cag ctc ctc acc cct ctc agg gac caa ttc acc agg gct cct     2351
   Lys Lys Gln Leu Leu Thr Pro Leu Arg Asp Gln Phe Thr Arg Ala Pro
           770                 775                 780
   ctg gac aat gac att gga gtg tct gag gcc acc agg att gac cca aat     2399
   Leu Asp Asn Asp Ile Gly Val Ser Glu Ala Thr Arg Ile Asp Pro Asn
       785                 790                 795
   gct tgg gtg gag agg tgg aag gct gct gga cac tac cag gct gag gct     2447
   Ala Trp Val Glu Arg Trp Lys Ala Ala Gly His Tyr Gln Ala Glu Ala
   800                 805                 810                 815
   gcc ctg ctc cag tgc aca gca gac acc ctg gct gat gct gtt ctg atc     2495
   Ala Leu Leu Gln Cys Thr Ala Asp Thr Leu Ala Asp Ala Val Leu Ile
                   820                 825                 830
   acc aca gcc cat gct tgg cag cac caa ggc aag acc ctg ttc atc agc     2543
   Thr Thr Ala His Ala Trp Gln His Gln Gly Lys Thr Leu Phe Ile Ser
               835                 840                 845
   aga aag acc tac agg att gat ggc tct gga cag atg gca atc aca gtg     2591
   Arg Lys Thr Tyr Arg Ile Asp Gly Ser Gly Gln Met Ala Ile Thr Val
           850                 855                 860
   gat gtg gag gtt gcc tct gac aca cct cac cct gca agg att ggc ctg     2639
   Asp Val Glu Val Ala Ser Asp Thr Pro His Pro Ala Arg Ile Gly Leu
       865                 870                 875
   aac tgt caa ctg gca cag gtg gct gag agg gtg aac tgg ctg ggc tta     2687
   Asn Cys Gln Leu Ala Gln Val Ala Glu Arg Val Asn Trp Leu Gly Leu
   880                 885                 890                 895
   ggc cct cag gag aac tac cct gac agg ctg aca gct gcc tgc ttt gac     2735
   Gly Pro Gln Glu Asn Tyr Pro Asp Arg Leu Thr Ala Ala Cys Phe Asp
                   900                 905                 910
   agg tgg gac ctg cct ctg tct gac atg tac acc cct tat gtg ttc cct     2783
   Arg Trp Asp Leu Pro Leu Ser Asp Met Tyr Thr Pro Tyr Val Phe Pro
               915                 920                 925
   tct gag aat ggc ctg agg tgt ggc acc agg gag ctg aac tat ggt cct     2831
   Ser Glu Asn Gly Leu Arg Cys Gly Thr Arg Glu Leu Asn Tyr Gly Pro
           930                 935                 940
   cac cag tgg agg gga gac ttc cag ttc aac atc tcc agg tac tct cag        2879
   His Gln Trp Arg Gly Asp Phe Gln Phe Asn Ile Ser Arg Tyr Ser Gln
       945                 950                     955
   caa cag ctc atg gaa acc tct cac agg cac ctg ctc cat gca gag gag        2927
   Gln Gln Leu Met Glu Thr Ser His Arg His Leu Leu His Ala Glu Glu
   960                 965                     970                 975
   gga acc tgg ctg aac att gat ggc ttc cac atg ggc att gga gga gat        2975
   Gly Thr Trp Leu Asn Ile Asp Gly Phe His Met Gly Ile Gly Gly Asp
                   980                 985                 990
   gac tct tgg tct cct tct gtg tct gct  gag ttc cag tta tct  gct ggc      3023
   Asp Ser Trp Ser Pro Ser Val Ser Ala  Glu Phe Gln Leu Ser  Ala Gly
               995                 1000                 1005
   agg tac cac  tat cag ctg gtg tgg  tgc cag aag taaacctgag ctagc         3071
   Arg Tyr His  Tyr Gln Leu Val Trp  Cys Gln Lys
           1010                 1015

<210> 10
   <211> 1018
   <212> PRT
   <213> Artificial Sequence
<220>
   <223> CpG-Free Lacz
   <400> 10
   Met Asp Pro Val Val Leu Gln Arg Arg Asp Trp Glu Asn Pro Gly Val
   1               5                   10                  15
   Thr Gln Leu Asn Arg Leu Ala Ala His Pro Pro Phe Ala Ser Trp Arg
               20                  25                  30
   Asn Ser Glu Glu Ala Arg Thr Asp Arg Pro Ser Gln Gln Leu Arg Ser
           35                  40                  45
   Leu Asn Gly Glu Trp Arg Phe Ala Trp Phe Pro Ala Pro Glu Ala Val
       50                  55                  60
   Pro Glu Ser Trp Leu Glu Cys Asp Leu Pro Glu Ala Asp Thr Val Val
   65                  70                  75                  80
   Val Pro Ser Asn Trp Gln Met His Gly Tyr Asp Ala Pro Ile Tyr Thr
                   85                  90                  95
   Asn Val Thr Tyr Pro Ile Thr Val Asn Pro Pro Phe Val Pro Thr Glu
               100                 105                 110
   Asn Pro Thr Gly Cys Tyr Ser Leu Thr Phe Asn Val Asp Glu Ser Trp
           115                 120                 125
   Leu Gln Glu Gly Gln Thr Arg Ile Ile Phe Asp Gly Val Asn Ser Ala
   130 135 140
   Phe His Leu Trp Cys Asn Gly Arg Trp Val Gly Tyr Gly Gln Asp Ser
   145 150 155 160
   Arg Leu Pro Ser Glu Phe Asp Leu Ser Ala Phe Leu Arg Ala Gly Glu
   165 170 175
   Asn Arg Leu Ala Val Met Val Leu Arg Trp Ser Asp Gly Ser Tyr Leu
   180 185 190
   Glu Asp Gln Asp Met Trp Arg Met Ser Gly Ile Phe Arg Asp Val Ser
   195 200 205
   Leu Leu His Lys Pro Thr Thr Gln Ile Ser Asp Phe His Val Ala Thr
   210 215 220
   Arg Phe Asn Asp Asp Phe Ser Arg Ala Val Leu Glu Ala Glu Val Gln
   225 230 235 240
   Met Cys Gly Glu Leu Arg Asp Tyr Leu Arg Val Thr Val Ser Leu Trp
   245 250 255
   Gln Gly Glu Thr Gln Val Ala Ser Gly Thr Ala Pro Phe Gly Gly Glu
   260 265 270
   Ile Ile Asp Glu Arg Gly Gly Tyr Ala Asp Arg Val Thr Leu Arg Leu
   275 280 285
   Asn Val Glu Asn Pro Lys Leu Trp Ser Ala Glu Ile Pro Asn Leu Tyr
   290 295 300
   Arg Ala Val Val Glu Leu His Thr Ala Asp Gly Thr Leu Ile Glu Ala
   305 310 315 320
   Glu Ala Cys Asp Val Gly Phe Arg Glu Val Arg Ile Glu Asn Gly Leu
   325 330 335
   Leu Leu Leu Asn Gly Lys Pro Leu Leu Ile Arg Gly Val Asn Arg His
   340 345 350
   Glu His His Pro Leu His Gly Gln Val Met Asp Glu Gln Thr Met Val
   355 360 365
   Gln Asp Ile Leu Leu Met Lys Gln Asn Asn Phe Asn Ala Val Arg Cys
   370 375 380
   Ser His Tyr Pro Asn His Pro Leu Trp Tyr Thr Leu Cys Asp Arg Tyr
   385 390 395 400
   Gly Leu Tyr Val Val Asp Glu Ala Asn Ile Glu Thr His Gly Met Val
   405 410 415
   Pro Met Asn Arg Leu Thr Asp Asp Pro Arg Trp Leu Pro Ala Met Ser
   420 425 430
   Glu Arg Val Thr Arg Met Val Gln Arg Asp Arg Asn His Pro Ser Val
   435 440 445
   Ile Ile Trp Ser Leu Gly Asn Glu Ser Gly His Gly Ala Asn His Asp
   450 455 460
   Ala Leu Tyr Arg Trp Ile Lys Ser Val Asp Pro Ser Arg Pro Val Gln
   465 470 475 480
   Tyr Glu Gly Gly Gly Ala Asp Thr Thr Ala Thr Asp Ile Ile Cys Pro
   485 490 495
   Met Tyr Ala Arg Val Asp Glu Asp Gln Pro Phe Pro Ala Val Pro Lys
   500 505 510
   Trp Ser Ile Lys Lys Trp Leu Ser Leu Pro Gly Glu Thr Arg Pro Leu
   515 520 525
   Ile Leu Cys Glu Tyr Ala His Ala Met Gly Asn Ser Leu Gly Gly Phe
   530 535 540
   Ala Lys Tyr Trp Gln Ala Phe Arg Gln Tyr Pro Arg Leu Gln Gly Gly
   545 550 555 560
   Phe Val Trp Asp Trp Val Asp Gln Ser Leu Ile Lys Tyr Asp Glu Asn
   565 570 575
   Gly Asn Pro Trp Ser Ala Tyr Gly Gly Asp Phe Gly Asp Thr Pro Asn
   580 585 590
   Asp Arg Gln Phe Cys Met Asn Gly Leu Val Phe Ala Asp Arg Thr Pro
   595 600 605
   His Pro Ala Leu Thr Glu Ala Lys His Gln Gln Gln Phe Phe Gln Phe
   610 615 620
   Arg Leu Ser Gly Gln Thr Ile Glu Val Thr Ser Glu Tyr Leu Phe Arg
   625 630 635 640
   His Ser Asp Asn Glu Leu Leu His Trp Met Val Ala Leu Asp Gly Lys
   645 650 655
   Pro Leu Ala Ser Gly Glu Val Pro Leu Asp Val Ala Pro Gln Gly Lys
   660 665 670
   Gln Leu Ile Glu Leu Pro Glu Leu Pro Gln Pro Glu Ser Ala Gly Gln
   675 680 685
   Leu Trp Leu Thr Val Arg Val Val Gln Pro Asn Ala Thr Ala Trp Ser
   690 695 700
   Glu Ala Gly His Ile Ser Ala Trp Gln Gln Trp Arg Leu Ala Glu Asn
   705 710 715 720
   Leu Ser Val Thr Leu Pro Ala Ala Ser His Ala Ile Pro His Leu Thr
   725 730 735
   Thr Ser Glu Met Asp Phe Cys Ile Glu Leu Gly Asn Lys Arg Trp Gln
   740 745 750
   Phe Asn Arg Gln Ser Gly Phe Leu Ser Gln Met Trp Ile Gly Asp Lys
   755 760 765
   Lys Gln Leu Leu Thr Pro Leu Arg Asp Gln Phe Thr Arg Ala Pro Leu
   770 775 780
   Asp Asn Asp Ile Gly Val Ser Glu Ala Thr Arg Ile Asp Pro Asn Ala
   785 790 795 800
   Trp Val Glu Arg Trp Lys Ala Ala Gly His Tyr Gln Ala Glu Ala Ala
   805 810 815
   Leu Leu Gln Cys Thr Ala Asp Thr Leu Ala Asp Ala Val Leu Ile Thr
   820 825 830
   Thr Ala His Ala Trp Gln His Gln Gly Lys Thr Leu Phe Ile Ser Arg
   835 840 845
   Lys Thr Tyr Arg Ile Asp Gly Ser Gly Gln Met Ala Ile Thr Val Asp
   850 855 860
   Val Glu Val Ala Ser Asp Thr Pro His Pro Ala Arg Ile Gly Leu Asn
   865 870 875 880
   Cys Gln Leu Ala Gln Val Ala Glu Arg Val Asn Trp Leu Gly Leu Gly
   885 890 895
   Pro Gln Glu Asn Tyr Pro Asp Arg Leu Thr Ala Ala Cys Phe Asp Arg
   900 905 910
   Trp Asp Leu Pro Leu Ser Asp Met Tyr Thr Pro Tyr Val Phe Pro Ser
   915 920 925
   Glu Asn Gly Leu Arg Cys Gly Thr Arg Glu Leu Asn Tyr Gly Pro His
   930 935 940
   Gln Trp Arg Gly Asp Phe Gln Phe Asn Ile Ser Arg Tyr Ser Gln Gln
   945 950 955 960
   Gln Leu Met Glu Thr Ser His Arg His Leu Leu His Ala Glu Glu Gly
   965 970 975
   Thr Trp Leu Asn Ile Asp Gly Phe His Met Gly Ile Gly Gly Asp Asp
   980 985 990
   Ser Trp Ser Pro Ser Val Ser Ala Glu Phe Gln Leu Ser Ala Gly Arg
   995 1000 1005
   Tyr His Tyr Gln Leu Val Trp Cys Gln Lys
   1010 1015
<210> 11
   <211> 66
   <212> DNA
   <213> Artificial Sequence
<220>
   <223> CpG-Free constitutive promotor
<400> 11
   caattaaaca ttggcatagt atatctgcat agtataatac aactcactat aggagggcca 60
   ccatgg 66
<210> 12
   <211> 320
   <212> DNA
   <213> Artificial Sequence
<220>
   <223> CpG-Free replication origin
<400> 12
   gcaggactga ggcttaatta aaccttaaaa cctttaaaag ccttatatat tctttttttt 60
   cttataaaac ttaaaacctt agaggctatt taagttgctg atttatatta attttattgt 120
   tcaaacatga gagcttagta catgaaacat gagagcttag tacattagcc atgagagctt 180
   agtacattag ccatgagggt ttagttcatt aaacatgaga gcttagtaca ttaaacatga 240
   gagcttagta catgaaacat gagagcttag tacatactat caacaggttg aactgctgat 300
   cttaattaac ctggagactt 320
<210> 13
   <211> 273
   <212> DNA
   <213> Artificial Sequence

<220>
   <223> CpG-Free replication origin
<400> 13
   ttaattaacc ttaaaacctt taaaagcctt atatattctt ttttttctta taaaacttaa      60
   aaccttagag gctatttaag ttgctgattt atattaattt tattgttcaa acatgagagc     120
   ttagtacatg aaacatgaga gcttagtaca ttagccatga gagcttagta cattagccat     180
   gagggtttag ttcattaaac atgagagctt agtacattaa acatgagagc ttagtacata     240
   ctatcaacag gttgaactgc tgatcttaat taa                                  273
<210> 14
   <211> 3852
   <212> DNA
   <213> Artificial Sequence
<220>
   <223> SEQ ID NO:14 = CpG-Free pSh-LacZ plasmid ; SEQ ID NO:15= SEQ ID N O:14 CDS from 83 to 454 (SEQ ID NO:2) + SEQ ID NO:14 CDS from 483 to 3536 (SEQ ID NO:10)
<220>
   <221> CDS
   <222> (83)..(454)
   <223>
<220>
   <221> CDS
   <222> (483)..(3536)
   <223>
<400> 14
   ttaattaaaa cagtagttga caattaaaca ttggcatagt atatctgcat agtataatac      60
   aactcactat aggagggcca cc atg gcc aag ttg acc agt gct gtc cca gtg     112
                           Met Ala Lys Leu Thr Ser Ala Val Pro Val
                           1               5                   10
   ctc aca gcc agg gat gtg gct gga gct gtt gag ttc tgg act gac agg      160
   Leu Thr Ala Arg Asp Val Ala Gly Ala Val Glu Phe Trp Thr Asp Arg
                   15                  20                  25
   ttg ggg ttc tcc aga gat ttt gtg gag gat gac ttt gca ggt gtg gtc      208
   Leu Gly Phe Ser Arg Asp Phe Val Glu Asp Asp Phe Ala Gly Val Val
               30                  35                  40
   aga gat gat gtc acc ctg ttc atc tca gca gtc cag gac cag gtg gtg      256
   Arg Asp Asp Val Thr Leu Phe Ile Ser Ala Val Gln Asp Gln Val Val
           45                  50                  55
   cct gac aac acc ctg gct tgg gtg tgg gtg aga gga ctg gat gag ctg      304
   Pro Asp Asn Thr Leu Ala Trp Val Trp Val Arg Gly Leu Asp Glu Leu
       60                  65                  70
   tat gct gag tgg agt gag gtg gtc tcc acc aac ttc agg gat gcc agt      352
   Tyr Ala Glu Trp Ser Glu Val Val Ser Thr Asn Phe Arg Asp Ala Ser
   75                  80                  85                  90
   ggc cct gcc atg aca gag att gga gag cag ccc tgg ggg aga gag ttt      400
   Gly Pro Ala Met Thr Glu Ile Gly Glu Gln Pro Trp Gly Arg Glu Phe
                   95                  100                 105
   gcc ctg aga gac cca gca ggc aac tgt gtg cac ttt gtg gca gag gag      448
   Ala Leu Arg Asp Pro Ala Gly Asn Cys Val His Phe Val Ala Glu Glu
               110                 115                 120
   cag gac tgaggataag aattctgagg agaagctc atg gac cct gtt gtg ctg       500
   Gln Asp                                Met Asp Pro Val Val Leu
                                          125                 130
   caa agg aga gac tgg gag aac cct gga gtg acc cag ctc aac aga ctg      548
   Gln Arg Arg Asp Trp Glu Asn Pro Gly Val Thr Gln Leu Asn Arg Leu
                   135                 140                 145
   gct gcc cac cct ccc ttt gcc tct tgg agg aac tct gag gaa gcc agg      596
   Ala Ala His Pro Pro Phe Ala Ser Trp Arg Asn Ser Glu Glu Ala Arg
               150                 155                 160
   aca gac agg ccc agc cag cag ctc agg tct ctc aat gga gag tgg agg      644
   Thr Asp Arg Pro Ser Gln Gln Leu Arg Ser Leu Asn Gly Glu Trp Arg
           165                 170                 175
   ttt gcc tgg ttc cct gcc cct gaa gct gtg cct gag tct tgg ctg gag      692
   Phe Ala Trp Phe Pro Ala Pro Glu Ala Val Pro Glu Ser Trp Leu Glu
       180                 185                 190
   tgt gac ctc cca gag gct gac act gtt gtg gtg ccc agc aac tgg cag      740
   Cys Asp Leu Pro Glu Ala Asp Thr Val Val Val Pro Ser Asn Trp Gln
   195                 200                 205                 210
   atg cat ggc tat gat gcc ccc atc tac acc aat gtc acc tac ccc atc      788
   Met His Gly Tyr Asp Ala Pro Ile Tyr Thr Asn Val Thr Tyr Pro Ile
                   215                 220                 225
   act gtg aac ccc cct ttt gtg ccc act gag aac ccc act ggc tgc tac      836
   Thr Val Asn Pro Pro Phe Val Pro Thr Glu Asn Pro Thr Gly Cys Tyr
               230                 235                 240
   agc ctg acc ttc aat gtt gat gag agc tgg ctg caa gaa ggc cag acc      884
   Ser Leu Thr Phe Asn Val Asp Glu Ser Trp Leu Gln Glu Gly Gln Thr
           245                 250                 255
   agg atc atc ttt gat gga gtc aac tct gcc ttc cac ctc tgg tgc aat      932
   Arg Ile Ile Phe Asp Gly Val Asn Ser Ala Phe His Leu Trp Cys Asn
       260                 265                 270
   ggc agg tgg gtt ggc tat ggc caa gac agc agg ctg ccc tct gag ttt      980
   Gly Arg Trp Val Gly Tyr Gly Gln Asp Ser Arg Leu Pro Ser Glu Phe
   275                 280                 285                 290
   gac ctc tct gcc ttc ctc aga gct gga gag aac agg ctg gct gtc atg     1028
   Asp Leu Ser Ala Phe Leu Arg Ala Gly Glu Asn Arg Leu Ala Val Met
                   295                 300                 305
   gtg ctc agg tgg tct gat ggc agc tac ctg gaa gac caa gac atg tgg     1076
   Val Leu Arg Trp Ser Asp Gly Ser Tyr Leu Glu Asp Gln Asp Met Trp
               310                 315                 320
   agg atg tct ggc atc ttc agg gat gtg agc ctg ctg cac aag ccc acc     1124
   Arg Met Ser Gly Ile Phe Arg Asp Val Ser Leu Leu His Lys Pro Thr
           325                 330                 335
   acc cag att tct gac ttc cat gtt gcc acc agg ttc aat gat gac ttc     1172
   Thr Gln Ile Ser Asp Phe His Val Ala Thr Arg Phe Asn Asp Asp Phe
       340                 345                 350
   agc aga gct gtg ctg gag gct gag gtg cag atg tgt gga gaa ctc aga     1220
   Ser Arg Ala Val Leu Glu Ala Glu Val Gln Met Cys Gly Glu Leu Arg
   355                 360                 365                 370
   gac tac ctg aga gtc aca gtg agc ctc tgg caa ggt gag acc cag gtg     1268
   Asp Tyr Leu Arg Val Thr Val Ser Leu Trp Gln Gly Glu Thr Gln Val
                   375                 380                 385
   gcc tct ggc aca gcc ccc ttt gga gga gag atc att gat gag aga gga     1316
   Ala Ser Gly Thr Ala Pro Phe Gly Gly Glu Ile Ile Asp Glu Arg Gly
               390                 395                 400
   ggc tat gct gac aga gtc acc ctg agg ctc aat gtg gag aac ccc aag     1364
   Gly Tyr Ala Asp Arg Val Thr Leu Arg Leu Asn Val Glu Asn Pro Lys
           405                 410                 415
   ctg tgg tct gct gag atc ccc aac ctc tac agg gct gtt gtg gag ctg     1412
   Leu Trp Ser Ala Glu Ile Pro Asn Leu Tyr Arg Ala Val Val Glu Leu
       420                 425                 430
   cac act gct gat ggc acc ctg att gaa gct gaa gcc tgt gat gtt gga     1460
   His Thr Ala Asp Gly Thr Leu Ile Glu Ala Glu Ala Cys Asp Val Gly
   435                 440                 445                 450
   ttc aga gaa gtc agg att gag aat ggc ctg ctg ctg ctc aat ggc aag     1508
   Phe Arg Glu Val Arg Ile Glu Asn Gly Leu Leu Leu Leu Asn Gly Lys
                   455                 460                 465
   cct ctg ctc atc agg gga gtc aac agg cat gag cac cac cct ctg cat     1556
   Pro Leu Leu Ile Arg Gly Val Asn Arg His Glu His His Pro Leu His
               470                 475                 480
   gga caa gtg atg gat gaa cag aca atg gtg caa gat atc ctg cta atg     1604
   Gly Gln Val Met Asp Glu Gln Thr Met Val Gln Asp Ile Leu Leu Met
           485                 490                 495
   aag cag aac aac ttc aat gct gtc agg tgc tct cac tac ccc aac cac     1652
   Lys Gln Asn Asn Phe Asn Ala Val Arg Cys Ser His Tyr Pro Asn His
       500                 505                 510
   cct ctc tgg tac acc ctg tgt gac agg tat ggc ctg tat gtt gtt gat     1700
   Pro Leu Trp Tyr Thr Leu Cys Asp Arg Tyr Gly Leu Tyr Val Val Asp
   515                 520                 525                 530
   gaa gcc aac att gag aca cat ggc atg gtg ccc atg aac agg ctc aca     1748
   Glu Ala Asn Ile Glu Thr His Gly Met Val Pro Met Asn Arg Leu Thr
                   535                 540                 545
   gat gac ccc agg tgg ctg cct gcc atg tct gag aga gtg acc agg atg      1796
   Asp Asp Pro Arg Trp Leu Pro Ala Met Ser Glu Arg Val Thr Arg Met
               550                 555                 560
   gtg cag aga gac agg aac cac ccc tct gtg atc atc tgg tct ctg ggc      1844
   Val Gln Arg Asp Arg Asn His Pro Ser Val Ile Ile Trp Ser Leu Gly
           565                 570                 575
   aat gag tct gga cat gga gcc aac cat gat gct ctc tac agg tgg atc      1892
   Asn Glu Ser Gly His Gly Ala Asn His Asp Ala Leu Tyr Arg Trp Ile
       580                 585                 590
   aag tct gtt gac ccc agc aga cct gtg cag tat gaa gga ggt gga gca      1940
   Lys Ser Val Asp Pro Ser Arg Pro Val Gln Tyr Glu Gly Gly Gly Ala
   595                 600                 605                 610
   gac acc aca gcc aca gac atc atc tgc ccc atg tat gcc agg gtt gat      1988
   Asp Thr Thr Ala Thr Asp Ile Ile Cys Pro Met Tyr Ala Arg Val Asp
                   615                 620                 625
   gag gac cag ccc ttc cct gct gtg ccc aag tgg agc atc aag aag tgg      2036
   Glu Asp Gln Pro Phe Pro Ala Val Pro Lys Trp Ser Ile Lys Lys Trp
               630                 635                 640
   ctc tct ctg cct gga gag acc aga cct ctg atc ctg tgt gaa tat gca      2084
   Leu Ser Leu Pro Gly Glu Thr Arg Pro Leu Ile Leu Cys Glu Tyr Ala
           645                 650                 655
   cat gca atg ggc aac tct ctg gga ggc ttt gcc aag tac tgg caa gcc      2132
   His Ala Met Gly Asn Ser Leu Gly Gly Phe Ala Lys Tyr Trp Gln Ala
       660                 665                 670
   ttc aga cag tac ccc agg ctg caa gga gga ttt gtg tgg gac tgg gtg      2180
   Phe Arg Gln Tyr Pro Arg Leu Gln Gly Gly Phe Val Trp Asp Trp Val
   675                 680                 685                 690
   gac caa tct ctc atc aag tat gat gag aat ggc aac ccc tgg tct gcc      2228
   Asp Gln Ser Leu Ile Lys Tyr Asp Glu Asn Gly Asn Pro Trp Ser Ala
                   695                 700                 705
   tat gga gga gac ttt ggt gac acc ccc aat gac agg cag ttc tgc atg      2276
   Tyr Gly Gly Asp Phe Gly Asp Thr Pro Asn Asp Arg Gln Phe Cys Met
               710                 715                 720
   aat ggc ctg gtc ttt gca gac agg acc cct cac cct gcc ctc aca gag      2324
   Asn Gly Leu Val Phe Ala Asp Arg Thr Pro His Pro Ala Leu Thr Glu
           725                 730                 735
   gcc aag cac cag caa cag ttc ttc cag ttc agg ctg tct gga cag acc      2372
   Ala Lys His Gln Gln Gln Phe Phe Gln Phe Arg Leu Ser Gly Gln Thr
       740                 745                 750
   att gag gtg aca tct gag tac ctc ttc agg cac tct gac aat gag ctc      2420
   Ile Glu Val Thr Ser Glu Tyr Leu Phe Arg His Ser Asp Asn Glu Leu
   755                 760                 765                 770
   ctg cac tgg atg gtg gcc ctg gat ggc aag cct ctg gct tct ggt gag      2468
   Leu His Trp Met Val Ala Leu Asp Gly Lys Pro Leu Ala Ser Gly Glu
                   775                 780                 785
   gtg cct ctg gat gtg gcc cct caa gga aag cag ctg att gaa ctg cct      2516
   Val Pro Leu Asp Val Ala Pro Gln Gly Lys Gln Leu Ile Glu Leu Pro
               790                 795                 800
   gag ctg cct cag cca gag tct gct gga caa ctg tgg cta aca gtg agg      2564
   Glu Leu Pro Gln Pro Glu Ser Ala Gly Gln Leu Trp Leu Thr Val Arg
   805 810 815
   gtg gtt cag ccc aat gca aca gct tgg tct gag gca ggc cac atc tct 2612
   Val Val Gln Pro Asn Ala Thr Ala Trp Ser Glu Ala Gly His Ile Ser
   820 825 830
   gca tgg cag cag tgg agg ctg gct gag aac ctc tct gtg acc ctg cct 2660
   Ala Trp Gln Gln Trp Arg Leu Ala Glu Asn Leu Ser Val Thr Leu Pro
   835 840 845 850
   gct gcc tct cat gcc atc cct cac ctg aca aca tct gaa atg gac ttc 2708
   Ala Ala Ser His Ala Ile Pro His Leu Thr Thr Ser Glu Met Asp Phe
   855 860 865
   tgc att gag ctg ggc aac aag aga tgg cag ttc aac agg cag tct ggc 2756
   Cys Ile Glu Leu Gly Asn Lys Arg Trp Gln Phe Asn Arg Gln Ser Gly
   870 875 880
   ttc ctg tct cag atg tgg att gga gac aag aag cag ctc ctc acc cct 2804
   Phe Leu Ser Gln Met Trp Ile Gly Asp Lys Lys Gln Leu Leu Thr Pro
   885 890 895
   ctc agg gac caa ttc acc agg gct cct ctg gac aat gac att gga gtg 2852
   Leu Arg Asp Gln Phe Thr Arg Ala Pro Leu Asp Asn Asp Ile Gly Val
   900 905 910
   tct gag gcc acc agg att gac cca aat gct tgg gtg gag agg tgg aag 2900
   Ser Glu Ala Thr Arg Ile Asp Pro Asn Ala Trp Val Glu Arg Trp Lys
   915 920 925 930
   gct gct gga cac tac cag gct gag gct gcc ctg ctc cag tgc aca gca 2948
   Ala Ala Gly His Tyr Gln Ala Glu Ala Ala Leu Leu Gln Cys Thr Ala
   935 940 945
   gac acc ctg gct gat gct gtt ctg atc acc aca gcc cat gct tgg cag 2996
   Asp Thr Leu Ala Asp Ala Val Leu Ile Thr Thr Ala His Ala Trp Gln
   950 955 960
   cac caa ggc aag acc ctg ttc atc agc aga aag acc tac agg att gat 3044
   His Gln Gly Lys Thr Leu Phe Ile Ser Arg Lys Thr Tyr Arg Ile Asp
   965 970 975
   ggc tct gga cag atg gca atc aca gtg gat gtg gag gtt gcc tct gac 3092
   Gly Ser Gly Gln Met Ala Ile Thr Val Asp Val Glu Val Ala Ser Asp
   980 985 990
   aca cct cac cct gca agg att ggc ctg aac tgt caa ctg gca cag 3137
   Thr Pro His Pro Ala Arg Ile Gly Leu Asn Cys Gln Leu Ala Gln
   995 1000 1005
   gtg gct gag agg gtg aac tgg ctg ggc tta ggc cct cag gag aac 3182
   Val Ala Glu Arg Val Asn Trp Leu Gly Leu Gly Pro Gln Glu Asn
   1010 1015 1020
   tac cct gac agg ctg aca gct gcc tgc ttt gac agg tgg gac ctg 3227
   Tyr Pro Asp Arg Leu Thr Ala Ala Cys Phe Asp Arg Trp Asp Leu
   1025 1030 1035
   cct ctg tct gac atg tac acc cct tat gtg ttc cct tct gag aat 3272
   Pro Leu Ser Asp Met Tyr Thr Pro Tyr Val Phe Pro Ser Glu Asn
   1040 1045 1050
   ggc ctg agg tgt ggc acc agg gag ctg aac tat ggt cct cac cag 3317
   Gly Leu Arg Cys Gly Thr Arg Glu Leu Asn Tyr Gly Pro His Gln
   1055 1060 1065
   tgg agg gga gac ttc cag ttc aac atc tcc agg tac tct cag caa 3362
   Trp  Arg Gly Asp Phe Gln  Phe Asn Ile Ser Arg  Tyr Ser Gln Gln
   1070                 1075                 1080
   cag  ctc atg gaa acc tct  cac agg cac ctg ctc  cat gca gag gag      3407
   Gln  Leu Met Glu Thr Ser  His Arg His Leu Leu  His Ala Glu Glu
   1085                 1090                 1095
   gga  acc tgg ctg aac att  gat ggc ttc cac atg  ggc att gga gga      3452
   Gly  Thr Trp Leu Asn Ile  Asp Gly Phe His Met  Gly Ile Gly Gly
   1100                 1105                 1110
   gat  gac tct tgg tct cct  tct gtg tct gct gag  ttc cag tta tct      3497
   Asp  Asp Ser Trp Ser Pro  Ser Val Ser Ala Glu  Phe Gln Leu Ser
   1115                 1120                 1125
   gct  ggc agg tac cac tat  cag ctg gtg tgg tgc  cag aag taagctagct   3546
   Ala  Gly Arg Tyr His Tyr  Gln Leu Val Trp Cys  Gln Lys
   1130                 1135                 1140
   gagtttcaga aaagggggcc tgagtggccc cttttttcaa cttaattaac cttaaaacct   3606
   ttaaaagcct tatatattct tttttttctt ataaaactta aaaccttaga ggctatttaa   3666
   gttgctgatt tatattaatt ttattgttca aacatgagag cttagtacat gaaacatgag   3726
   agcttagtac attagccatg agagcttagt acattagcca tgagggttta gttcattaaa   3786
   catgagagct tagtacatta aacatgagag cttagtacat actatcaaca ggttgaactg   3846
   ctgatc                                                              3852
<210> 15
   <211> 1142
   <212> PRT
   <213> Artificial Sequence
<220>
   <223> SEQ ID NO:14 = CpG-Free psh-Lacz plasmid ; SEQ ID NO:15= SEQ ID N o:14 CDS from 83 to 454 (SEQ ID NO:2) + SEQ ID NO:14 CDS from 483 to 3536 (SEQ ID NO:10)
<400> 15
   Met Ala Lys Leu Thr Ser Ala Val Pro Val Leu Thr Ala Arg Asp Val
   1               5                   10                  15
   Ala Gly Ala Val Glu Phe Trp Thr Asp Arg Leu Gly Phe Ser Arg Asp
               20                  25                  30
   Phe Val Glu Asp Asp Phe Ala Gly Val Val Arg Asp Asp Val Thr Leu
           35                  40                  45
   Phe Ile Ser Ala Val Gln Asp Gln Val Val Pro Asp Asn Thr Leu Ala
       50                  55                  60
   Trp Val Trp Val Arg Gly Leu Asp Glu Leu Tyr Ala Glu Trp Ser Glu
   65                  70                  75                  80
   Val Val Ser Thr Asn Phe Arg Asp Ala Ser Gly Pro Ala Met Thr Glu
   85 90 95
   Ile Gly Glu Gln Pro Trp Gly Arg Glu Phe Ala Leu Arg Asp Pro Ala
   100 105 110
   Gly Asn Cys Val His Phe Val Ala Glu Glu Gln Asp Met Asp Pro Val
   115 120 125
   Val Leu Gln Arg Arg Asp Trp Glu Asn Pro Gly Val Thr Gln Leu Asn
   130 135 140
   Arg Leu Ala Ala His Pro Pro Phe Ala Ser Trp Arg Asn Ser Glu Glu
   145 150 155 160
   Ala Arg Thr Asp Arg Pro Ser Gln Gln Leu Arg Ser Leu Asn Gly Glu
   165 170 175
   Trp Arg Phe Ala Trp Phe Pro Ala Pro Glu Ala Val Pro Glu Ser Trp
   180 185 190
   Leu Glu Cys Asp Leu Pro Glu Ala Asp Thr Val Val Val Pro Ser Asn
   195 200 205
   Trp Gln Met His Gly Tyr Asp Ala Pro Ile Tyr Thr Asn Val Thr Tyr
   210 215 220
   Pro Ile Thr Val Asn Pro Pro Phe Val Pro Thr Glu Asn Pro Thr Gly
   225 230 235 240
   Cys Tyr Ser Leu Thr Phe Asn Val Asp Glu Ser Trp Leu Gln Glu Gly
   245 250 255
   Gln Thr Arg Ile Ile Phe Asp Gly Val Asn Ser Ala Phe His Leu Trp
   260 265 270
   Cys Asn Gly Arg Trp Val Gly Tyr Gly Gln Asp Ser Arg Leu Pro Ser
   275 280 285
   Glu Phe Asp Leu Ser Ala Phe Leu Arg Ala Gly Glu Asn Arg Leu Ala
   290 295 300
   Val Met Val Leu Arg Trp Ser Asp Gly Ser Tyr Leu Glu Asp Gln Asp
   305 310 315 320
   Met Trp Arg Met Ser Gly Ile Phe Arg Asp Val Ser Leu Leu His Lys
   325 330 335
   Pro Thr Thr Gln Ile Ser Asp Phe His Val Ala Thr Arg Phe Asn Asp
   340 345 350
   Asp Phe Ser Arg Ala Val Leu Glu Ala Glu Val Gln Met Cys Gly Glu
   355 360 365
   Leu Arg Asp Tyr Leu Arg Val Thr Val Ser Leu Trp Gln Gly Glu Thr
   370 375 380
   Gln Val Ala Ser Gly Thr Ala Pro Phe Gly Gly Glu Ile Ile Asp Glu
   385 390 395 400
   Arg Gly Gly Tyr Ala Asp Arg Val Thr Leu Arg Leu Asn Val Glu Asn
   405 410 415
   Pro Lys Leu Trp Ser Ala Glu Ile Pro Asn Leu Tyr Arg Ala Val Val
   420 425 430
   Glu Leu His Thr Ala Asp Gly Thr Leu Ile Glu Ala Glu Ala Cys Asp
   435 440 445
   Val Gly Phe Arg Glu Val Arg Ile Glu Asn Gly Leu Leu Leu Leu Asn
   450 455 460
   Gly Lys Pro Leu Leu Ile Arg Gly Val Asn Arg His Glu His His Pro
   465 470 475 480
   Leu His Gly Gln Val Met Asp Glu Gln Thr Met Val Gln Asp Ile Leu
   485 490 495
   Leu Met Lys Gln Asn Asn Phe Asn Ala Val Arg Cys Ser His Tyr Pro
   500 505 510
   Asn His Pro Leu Trp Tyr Thr Leu Cys Asp Arg Tyr Gly Leu Tyr Val
   515 520 525
   Val Asp Glu Ala Asn Ile Glu Thr His Gly Met Val Pro Met Asn Arg
   530 535 540
   Leu Thr Asp Asp Pro Arg Trp Leu Pro Ala Met Ser Glu Arg Val Thr
   545 550 555 560
   Arg Met Val Gln Arg Asp Arg Asn His Pro Ser Val Ile Ile Trp Ser
   565 570 575
   Leu Gly Asn Glu Ser Gly His Gly Ala Asn His Asp Ala Leu Tyr Arg
   580 585 590
   Trp Ile Lys Ser Val Asp Pro Ser Arg Pro Val Gln Tyr Glu Gly Gly
   595 600 605
   Gly Ala Asp Thr Thr Ala Thr Asp Ile Ile Cys Pro Met Tyr Ala Arg
   610 615 620
   Val Asp Glu Asp Gln Pro Phe Pro Ala Val Pro Lys Trp Ser Ile Lys
   625 630 635 640
   Lys Trp Leu Ser Leu Pro Gly Glu Thr Arg Pro Leu Ile Leu Cys Glu
   645 650 655
   Tyr Ala His Ala Met Gly Asn Ser Leu Gly Gly Phe Ala Lys Tyr Trp
   660 665 670
   Gln Ala Phe Arg Gln Tyr Pro Arg Leu Gln Gly Gly Phe Val Trp Asp
   675 680 685
   Trp Val Asp Gln Ser Leu Ile Lys Tyr Asp Glu Asn Gly Asn Pro Trp
   690 695 700
   Ser Ala Tyr Gly Gly Asp Phe Gly Asp Thr Pro Asn Asp Arg Gln Phe
   705 710 715 720
   Cys Met Asn Gly Leu Val Phe Ala Asp Arg Thr Pro His Pro Ala Leu
   725 730 735
   Thr Glu Ala Lys His Gln Gln Gln Phe Phe Gln Phe Arg Leu Ser Gly
   740 745 750
   Gln Thr Ile Glu Val Thr Ser Glu Tyr Leu Phe Arg His Ser Asp Asn
   755 760 765
   Glu Leu Leu His Trp Met Val Ala Leu Asp Gly Lys Pro Leu Ala Ser
   770 775 780
   Gly Glu Val Pro Leu Asp Val Ala Pro Gln Gly Lys Gln Leu Ile Glu
   785 790 795 800
   Leu Pro Glu Leu Pro Gln Pro Glu Ser Ala Gly Gln Leu Trp Leu Thr
   805 810 815
   Val Arg Val Val Gln Pro Asn Ala Thr Ala Trp Ser Glu Ala Gly His
   820 825 830
   Ile Ser Ala Trp Gln Gln Trp Arg Leu Ala Glu Asn Leu Ser Val Thr
   835 840 845
   Leu Pro Ala Ala Ser His Ala Ile Pro His Leu Thr Thr Ser Glu Met
   850 855 860
   Asp Phe Cys Ile Glu Leu Gly Asn Lys Arg Trp Gln Phe Asn Arg Gln
   865 870 875 880
   Ser Gly Phe Leu Ser Gln Met Trp Ile Gly Asp Lys Lys Gln Leu Leu
   885 890 895
   Thr Pro Leu Arg Asp Gln Phe Thr Arg Ala Pro Leu Asp Asn Asp Ile
   900 905 910
   Gly Val Ser Glu Ala Thr Arg Ile Asp Pro Asn Ala Trp Val Glu Arg
   915 920 925
   Trp Lys Ala Ala Gly His Tyr Gln Ala Glu Ala Ala Leu Leu Gln Cys
   930 935 940
   Thr Ala Asp Thr Leu Ala Asp Ala Val Leu Ile Thr Thr Ala His Ala
   945 950 955 960
   Trp Gln His Gln Gly Lys Thr Leu Phe Ile Ser Arg Lys Thr Tyr Arg
   965 970 975
   Ile Asp Gly Ser Gly Gln Met Ala Ile Thr Val Asp Val Glu Val Ala
   980 985 990
   Ser Asp Thr Pro His Pro Ala Arg Ile Gly Leu Asn Cys Gln Leu Ala
   995 1000 1005
   Gln Val Ala Glu Arg Val Asn Trp Leu Gly Leu Gly Pro Gln Glu
   1010 1015 1020
   Asn Tyr Pro Asp Arg Leu Thr Ala Ala Cys Phe Asp Arg Trp Asp
   1025 1030 1035
   Leu Pro Leu Ser Asp Met Tyr Thr Pro Tyr Val Phe Pro Ser Glu
   1040 1045 1050
   Asn Gly Leu Arg Cys Gly Thr Arg Glu Leu Asn Tyr Gly Pro His
   1055 1060 1065
   Gln Trp Arg Gly Asp Phe Gln Phe Asn Ile Ser Arg Tyr Ser Gln
   1070 1075 1080
   Gln Gln Leu Met Glu Thr Ser His Arg His Leu Leu His Ala Glu
   1085 1090 1095
   Glu Gly Thr Trp Leu Asn Ile Asp Gly Phe His Met Gly Ile Gly
   1100 1105 1110
   Gly Asp Asp Ser Trp Ser Pro Ser Val Ser Ala Glu Phe Gln Leu
   1115 1120 1125
   Ser Ala Gly Arg Tyr His Tyr Gln Leu Val Trp Cys Gln Lys
   1130 1135 1140
<210> 16
   <211> 40
   <212> DNA
   <213> Artificial Sequence

<220>
   <223> assembling oligo for CpG-Free sh ble
<400> 16
   aactcagctg aggaggcaga ccatggccaa gttgaccagt 40
<210> 17
   <211> 40
   <212> DNA
   <213> Artificial Sequence
<220>
   <223> assembling oligo for CpG-Free sh ble
<400> 17
   gctgtcccag tgctcacagc cagggatgtg gctggagctg 40
<210> 18
   <211> 40
   <212> DNA
   <213> Artificial Sequence
<220>
   <223> assembling oligo for CpG-Free sh ble
<400> 18
   ttgagttctg gactgacagg ttggggttct ccagagattt 40
<210> 19
   <211> 40
   <212> DNA
   <213> Artificial Sequence
<220>
   <223> assembling oligo for CpG-Free Sh ble
<400> 19
   tgtggaggat gactttgcag gtgtggtcag agatgatgtc 40
<210> 20
   <211> 40
   <212> DNA
   <213> Artificial Sequence
<220>
   <223> assembling oligo for CpG-Free sh ble
<400> 20
   accctgttca tctcagcagt ccaggaccag gtggtgcctg 40
<210> 21
   <211> 40
   <212> DNA
   <213> Artificial Sequence
<220>
   <223> assembling oligo for CpG-Free sh ble
<400> 21
   acaacaccct ggcttgggtg tgggtgagag gactggatga 40
<210> 22
   <211> 40
   <212> DNA
   <213> Artificial Sequence
<220>
   <223> assembling oligo for CpG-Free sh ble
<400> 22
   gctgtatgct gagtggagtg aggtggtctc caccaacttc 40
<210> 23
   <211> 40
   <212> DNA
   <213> Artificial Sequence
<220>
   <223> assembling oligo for CpG-Free Sh ble
<400> 23
   agggatgcca gtggccctgc catgacagag attggagagc 40
<210> 24
   <211> 40
   <212> DNA
   <213> Artificial Sequence
<220>
   <223> assembling oligo for CPG-Free sh ble
<400> 24
   agccctgggg gagagagttt gccctgagag acccagcagg 40
<210> 25
   <211> 40
   <212> DNA
   <213> Artificial Sequence
<220>
   <223> assembling oligo for CpG-Free sh ble
<400> 25
   caactgtgtg cactttgtgg cagaggagca ggactgagga 40
<210> 26
   <211> 21
   <212> DNA
   <213> Artificial Sequence
<220>
   <223> assembling oligo for CpG-Free sh ble

<400> 26
   taagaattca gctagctcga c 21
<210> 2-7
   <211> 41
   <212> DNA
   <213> Artificial Sequence
<220>
   <223> assembling oligo for CpG-Free sh ble
<400> 27
   gtcgagctag ctgaattctt atcctcagtc ctgctcctct g 41
<210> 28
   <211> 40
   <212> DNA
   <213> Artificial Sequence
<220>
   <223> assembling oligo for CpG-Free Sh ble
<400> 28
   ccacaaagtg cacacagttg cctgctgggt ctctcagggc 40
<210> 29
   <211> 40
   <212> DNA
   <213> Artificial Sequence
<220>
   <223> assembling oligo for CpG-Free sh ble
<400> 29
   aaactctctc ccccagggct gctctccaat ctctgtcatg 40
<210> 30
   <211> 40
   <212> DNA
   <213> Artificial Sequence
<220>
   <223> assembling oligo for CpG-Free sh ble
<400> 30
   gcagggccac tggcatccct gaagttggtg gagaccacct 40
<210> 31
   <211> 40
   <212> DNA
   <213> Artificial Sequence
<220>
   <223> assembling oligo for CpG-Free sh ble
<400> 31
   cactccactc agcatacagc tcatccagtc ctctcaccca 40

<210> 32
   <211> 40
   <212> DNA
   <213> Artificial Sequence
<220>
   <223> assembling oligo for CpG-Free sh ble
<400> 32
   cacccaagcc agggtgttgt caggcaccac ctggtcctgg 40
<210> 33
   <211> 40
   <212> DNA
   <213> Artificial Sequence
<220>
   <223> assembling oligo for CpG-Free Sh ble
<400> 33
   actgctgaga tgaacagggt gacatcatct ctgaccacac 40
<210> 34
   <211> 40
   <212> DNA
   <213> Artificial Sequence
<220>
   <223> assembling oligo for CpG-Free sh ble
<400> 34
   ctgcaaagtc atcctccaca aaatctctgg agaaccccaa 40
<210> 35
   <211> 40
   <212> DNA
   <213> Artificial Sequence
<220>
   <223> assembling oligo for CpG-Free sh ble
<400> 35
   cctgtcagtc cagaactcaa cagctccagc cacatccctg 40
<210> 36
   <211> 60
   <212> DNA
   <213> Artificial Sequence
<220>
   <223> assembling oligo for CpG-Free sh ble
<400> 36
   gctgtgagca ctgggacagc actggtcaac ttggccatgg 40
<210> 37
   <211> 20
   <212> DNA
   <213> Artificial Sequence
<220>
   <223> assembling oligo for CpG-Free sh ble
<400> 37
   tctgcctcct cagctgagtt 20
<210> 38
   <211> 60
   <212> DNA
   <213> Artificial Sequence
<220>
   <223> assembling oligo for CpG-Free Hph
<400> 38
   tgagatcacc ggttcagctg aggaggcaca tcatgaagaa acctgaactg acagcaactt 60
<210> 39
   <211> 60
   <212> DNA
   <213> Artificial Sequence
<220>
   <223> assembling oligo for CpG-Free Hph
<400> 39
   ctgttgagaa gtttctcatt gaaaaatttg attctgtttc tgatctcatg cagctgtctg 60
<210> 40
   <211> 60
   <212> DNA
   <213> Artificial Sequence
<220>
   <223> assembling oligo for CpG-Free Hph

<400> 40
   aaggtgaaga aagcagagcc ttttcttttg atgttggagg aagaggttat gttctgaggg 60
<210> 41
   <211> 60
   <212> DNA
   <213> Artificial Sequence
<220>
   <223> assembling oligo for CpG-Free Hph
<400> 41
   tcaattcttg tgctgatggt ttttacaaag acagatatgt ttacagacac tttgcctctg 60
<210> 42
   <211> 60
   <212> DNA
   <213> Artificial Sequence
<220>
   <223> assembling oligo for CpG-Free Hph
<400> 42
   ctgctctgcc aattccagaa gttctggaca ttggagaatt ttctgaatct ctcacctact 60
<210> 43
   <211> 60
   <212> DNA
   <213> Artificial Sequence
<220>
   <223> assembling oligo for CpG-Free Hph
<400> 43
   gcatcagcag aagagcacaa ggagtcactc tccaggatct ccctgaaact gagctgccag 60
<210> 44
   <211> 60
   <212> DNA
   <213> Artificial Sequence
<220>
   <223> assembling oligo for CpG-Free Hph
<400> 44
   ctgttctgca acctgttgct gaagcaatgg atgccattgc agcagctgat ctgagccaaa 60
<210> 45
   <211> 60
   <212> DNA
   <213> Artificial Sequence
<220>
   <223> assembling oligo for CpG-Free Hph
<400> 45
   cctctggatt tggtcctttt ggtccccaag gcattggtca gtacaccact tggagggatt 60
<210> 46
   <211> 60
   <212> DNA
   <213> Artificial Sequence
<220>
   <223> assembling oligo for CpG-Free Hph
<400> 46
   tcatttgtgc cattgctgat cctcatgtct atcactggca gactgtgatg gatgacacag 60
<210> 47
   <211> 60
   <212> DNA
   <213> Artificial Sequence
<220>
   <223> assembling oligo for CpG-Free Hph
<400> 47
   tttctgcttc tgttgctcag gcactggatg aactcatgct gtgggcagaa gattgtcctg 60
<210> 48
   <211> 60
   <212> DNA
   <213> Artificial Sequence

<220>
   <223> assembling oligo for CpG-Free Hph
<400> 48
   aagtcagaca cctggtccat gctgattttg gaagcaacaa tgttctgaca gacaatggca 60
<210> 49
   <211> 60
   <212> DNA
   <213> Artificial Sequence
<220>
   <223> assembling oligo for CpG-Free Hph
<400> 49
   gaatcactgc agtcattgac tggtctgaag ccatgtttgg agattctcaa tatgaggttg 60
<210> 50
   <211> 60
   <212> DNA
   <213> Artificial Sequence
<220>
   <223> assembling oligo for CpG-Free Hph
<400> 50
   ccaacatttt tttttggaga ccttggctgg cttgcatgga acaacaaaca agatattttg 60
<210> 51
   <211> 60
   <212> DNA
   <213> Artificial Sequence
<220>
   <223> assembling oligo for CpG-Free Hph
<400> 51
   aaagaagaca cccagaactg gctggttccc ccagactgag agcctacatg ctcagaattg 60
<210> 52
   <211> 60
   <212> DNA
   <213> Artificial Sequence
<220>
   <223> assembling oligo for CpG-Free Hph
<400> 52
   gcctggacca actgtatcaa tctctggttg atggaaactt tgatgatgct gcttgggcac 60
<210> 53
   <211> 60
   <212> DNA
   <213> Artificial Sequence
<220>
   <223> assembling oligo for CpG-Free Hph
<400> 53
   aaggaagatg tgatgccatt gtgaggtctg gtgctggaac tgttggaaga actcaaattg 60
<210> 54
   <211> 60
   <212> DNA
   <213> Artificial Sequence
<220>
   <223> assembling oligo for CpG-Free Hph
<400> 54
   caagaaggtc tgctgctgtt tggactgatg gatgtgttga agttctggct gactctggaa 60
<210> 55
   <211> 60
   <212> DNA
   <213> Artificial Sequence

<220>
   <223> assembling oligo for CpG-Free Hph
<400> 55
   acaggagacc ctccacaaga cccagagcca aggaatgaat attagctagc ggatcctgag 60
<210> 56
   <211> 30
   <212> DNA
   <213> Artificial Sequence
<220>
   <223> assembling oligo for CpG-Free Hph
<400> 56
   ctcaggatcc gctagctaat attcattcct 30
<210> 57
   <211> 60
   <212> DNA
   <213> Artificial Sequence
<220>
   <223> assembling oligo for CpG-Free Hph
<400> 57
   tggctctggg tcttgtggag ggtctcctgt ttccagagtc agccagaact tcaacacatc 60
<210> 58
   <211> 60
   <212> DNA
   <213> Artificial Sequence
<220>
   <223> assembling oligo for CpG-Free Hph
<400> 58
   catcagtcca aacagcagca gaccttcttg caatttgagt tcttccaaca gttccagcac 60
<210> 59
   <211> 60
   <212> DNA
   <213> Artificial Sequence
<220>
   <223> assembling oligo for CpG-Free Hph
<400> 59
   cagacctcac aatggcatca catcttcctt gtgcccaagc agcatcatca aagtttccat 60
<210> 60
   <211> 60
   <212> DNA
   <213> Artificial Sequence
<220>
   <223> assembling oligo for CpG-Free Hph
<400> 60
   caaccagaga ttgatacagt tggtccaggc caattctgag catgtaggct ctcagtctgg 60
<210> 61
   <211> 60
   <212> DNA
   <213> Artificial Sequence
<220>
   <223> assembling oligo for CpG-Free Hph
<400> 61
   gggaaccagc cagttctggg tgtcttcttt caaaatatct tgtttgttgt tccatgcaag 60
<210> 62
   <211> 60
   <212> DNA
   <213> Artificial Sequence
<220>
   <223> assembling oligo for CpG-Free Hph
<400> 62
   ccagccaagg tctccaaaaa aaaatgttgg caacctcata ttgagaatct ccaaacatgg 60

<210> 63
   <211> 60
   <212> DNA
   <213> Artificial Sequence
<220>
   <223> assembling oligo for CpG-Free Hph
<400> 63
   cttcagacca gtcaatgact gcagtgattc tgccattgtc tgtcagaaca ttgttgcttc 60
<210> 64
   <211> 60
   <212> DNA
   <213> Artificial Sequence
<220>
   <223> assembling oligo for CpG-Free Hph
<400> 64
   caaaatcagc atggaccagg tgtctgactt caggacaatc ttctgcccac agcatgagtt 60
<210> 65
   <211> 60
   <212> DNA
   <213> Artificial Sequence
<220>
   <223> assembling oligo for CpG-Free Hph
<400> 65
   catccagtgc ctgagcaaca gaagcagaaa ctgtgtcatc catcacagtc tgccagtgat 60
<210> 66
   <211> 60
   <212> DNA
   <213> Artificial Sequence
<220>
   <223> assembling oligo for CpG-Free Hph
<400> 66
   agacatgagg atcagcaatg gcacaaatga aatccctcca agtggtgtac tgaccaatgc 60
<210> 67
   <211> 60
   <212> DNA
   <213> Artificial Sequence
<220>
   <223> assembling oligo for CpG-Free Hph
<400> 67
   cttggggacc aaaaggacca aatccagagg tttggctcag atcagctgct gcaatggcat 60
<210> 68
   <211> 60
   <212> DNA
   <213> Artificial Sequence
<220>
   <223> assembling oligo for CpG-Free Hph
<400> 68
   ccattgcttc agcaacaggt tgcagaacag ctggcagctc agtttcaggg agatcctgga 60
<210> 69
   <211> 60
   <212> DNA
   <213> Artificial Sequence
<220>
   <223> assembling oligo for cpG-Free Hph
<400> 69
   gagtgactcc ttgtgctctt ctgctgatgc agtaggtgag agattcagaa aattctccaa 60
<210> 70
   <211> 60
   <212> DNA
   <213> Artificial Sequence
<220>
   <223> assembling oligo for CpG-Free Hph
<400> 70
   tgtccagaac ttctggaatt ggcagagcag cagaggcaaa gtgtctgtaa acatatctgt 60

<210> 71
   <211> 60
   <212> DNA
   <213> Artificial Sequence
<220>
   <223> assembling oligo for CpG-Free Hph
<400> 71
   ctttgtaaaa accatcagca caagaattga ccctcagaac ataacctctt cctccaacat 60
<210> 72
   <211> 60
   <212> DNA
   <213> Artificial Sequence
<220>
   <223> assembling oligo for CpG-Free Hph
<400> 72
   caaaagaaaa ggctctgctt tcttcacctt cagacagctg catgagatca gaaacagaat 60
<210> 73
   <211> 60
   <212> DNA
   <213> Artificial Sequence
<220>
   <223> assembling oligo for CpG-Free Hph
<400> 73
   caaatttttc aatgagaaac ttctcaacag aagttgctgt cagttcaggt ttcttcatga 60
<210> 74
   <211> 30
   <212> DNA
   <213> Artificial Sequence
<220>
   <223> assembling oligo for CpG-Free Hph
<400> 74
   tgtgcctcct cagctgaacc ggtgatctca 30
<210> 75
   <211> 40
   <212> DNA
   <213> Artificial Sequence
<220>
   <223> assembling oligo for CpG-Free Bsr
<400> 75
   aggaggcaca tcatgaagac cttcaacatc tctcagcagg 40
<210> 76
   <211> 40
   <212> DNA
   <213> Artificial Sequence
<220>
   <223> assembling oligo for CpG-Free Bsr
<400> 76
   atctggagct ggtggaggtc gccactgaga agatcaccat 40
<210> 77
   <211> 40
   <212> DNA
   <213> Artificial Sequence
<220>
   <223> assembling oligo for CpG-Free Bsr
<400> 77
   gctctatgag gacaacaagc accatgtcgg ggcggccatc 40
<210> 78
   <211> 40
   <212> DNA
   <213> Artificial Sequence
<220>
   <223> assembling oligo for cpG-Free Bsr
<400> 78
   aggaccaaga ctggggagat catctctgct gtccacattg 40
<210> 79
   <211> 40
   <212> DNA
   <213> Artificial Sequence
<220>
   <223> assembling oligo for CpG-Free Bsr
<400> 79
   aggcctacat tggcagggtc actgtctgtg ctgaagccat 40
<210> 80
   <211> 40
   <212> DNA
   <213> Artificial Sequence
<220>
   <223> assembling oligo for CpG-Free Bsr
<400> 80
   tgccattggg tctgctgtga gcaacgggca gaaggacttt 40

<210> 81
   <211> 40
   <212> DNA
   <213> Artificial Sequence
<220>
   <223> assembling oligo for CpG-Free Bsr
<400> 81
   gacaccattg tggctgtcag gcacccctac tctgatgagg 40
<210> 82
   <211> 40
   <212> DNA
   <213> Artificial Sequence
<220>
   <223> assembling oligo for CpG-Free Bsr
<400> 82
   tggacagatc catcagggtg gtcagcccct gtggcatgtg 40
<210> 83
   <211> 40
   <212> DNA
   <213> Artificial Sequence
<220>
   <223> assembling oligo for CpG-Free Bsr
<400> 83
   cagagagctc atctctgact atgctcctga ctgctttgtg 40
<210> 84
   <211> 40
   <212> DNA
   <213> Artificial Sequence
<220>
   <223> assembling oligo for CpG-Free Bsr
<400> 84
   ctcattgaga tgaatggcaa gctggtcaaa accaccattg 40
<210> 85
   <211> 40
   <212> DNA
   <213> Artificial Sequence
<220>
   <223> assembling oligo for CpG-Free Bsr
<400> 85
   aggaactcat ccccctcaag tacaccagga actaaacctg 40
<210> 86
   <211> 21
   <212> DNA
   <213> Artificial Sequence
<220>
   <223> assembling oligo for CpG-Free Bsr
<400> 86
   aattcagcta gctcgacatg a 21
<210> 87
   <211> 41
   <212> DNA
   <213> Artificial Sequence
<220>
   <223> assembling oligo for CpG-Free Bsr
<400> 87
   tcatgtcgag ctagctgaat tcaggtttag ttcctggtgt a 41
<210> 88
   <211> 40
   <212> DNA
   <213> Artificial Sequence
<220>
   <223> assembling oligo for CpG-Free Bsr
<400> 88
   cttgaggggg atgagttcct caatggtggt tttgaccagc 40
<210> 89
   <211> 40
   <212> DNA
   <213> Artificial Sequence
<220>
   <223> assembling oligo for CpG-Free Bsr
<400> 89
   ttgccattca tctcaatgag cacaaagcag tcaggagcat 40
<210> 90
   <211> 40
   <212> DNA
   <213> Artificial Sequence

<220>
   <223> assembling oligo for CpG-Free Bsr
<400> 90
   agtcagagat gagctctctg cacatgccac aggggctgac 40
<210> 91
   <211> 40
   <212> DNA
   <213> Artificial Sequence
<220>
   <223> assembling oligo for CpG-Free Bsr
<400> 91
   caccctgatg gatctgtcca cctcatcaga gtaggggtgc 40
<210> 92
   <211> 40
   <212> DNA
   <213> Artificial Sequence
<220>
   <223> assembling oligo for CpG-Free Bsr
<400> 92
   ctgacagcca caatggtgtc aaagtccttc tgcccgttgc 40
<210> 93
   <211> 40
   <212> DNA
   <213> Artificial Sequence
<220>
   <223> assembling oligo for CpG-Free Bsr
<400> 93
   tcacagcaga cccaatggca atggcttcag cacagacagt 40
<210> 94
   <211> 40
   <212> DNA
   <213> Artificial Sequence
<220>
   <223> assembling oligo for CpG-Free Bsr
<400> 94
   gaccctgcca atgtaggcct caatgtggac agcagagatg 40
<210> 95
   <211> 40
   <212> DNA
   <213> Artificial Sequence
<220>
   <223> assembling oligo for CpG-Free Bsr
<400> 95
   atctccccag tcttggtcct gatggccgcc ccgacatggt 40
<210> 96
   <211> 40
   <212> DNA
   <213> Artificial Sequence
<220>
   <223> assembling oligo for CpG-Free Bsr
<400> 96
   gcttgttgtc ctcatagagc atggtgatct tctcagtggc 40
<210> 97
   <211> 40
   <212> DNA
   <213> Artificial Sequence
<220>
   <223> assembling oligo for cpG-Free Bsr
<400> 97
   gacctccacc agctccagat cctgctgaga gatgttgaag 40
<210> 98
   <211> 20
   <212> DNA
   <213> Artificial Sequence
<220>
   <223> assembling oligo for CpG-Free Bsr
<400> 98
   gtcttcatga tgtgcctcct 20
<210> 99
   <211> 60
   <212> DNA
   <213> Artificial Sequence
<220>
   <223> assembling oligo for cpG-Free Pac
<400> 99
   ctcactatag gaggaccatc atgactgagt acaaacccac agtgaggctg gcaaccagag 60

<210> 100
   <211> 60
   <212> DNA
   <213> Artificial Sequence
<220>
   <223> assembling oligo for CpG-Free Pac
<400> 100
   atgatgttcc aagagctgtg agaacactgg ctgctgcttt tgcagactac cctgcaacaa 60
<210> 101
   <211> 60
   <212> DNA
   <213> Artificial Sequence
<220>
   <223> assembling oligo for CpG-Free Pac
<400> 101
   ggcacacagt tgaccctgac aggcacattg agagggtgac agaactgcaa gaactcttcc 60
<210> 102
   <211> 60
   <212> DNA
   <213> Artificial Sequence
<220>
   <223> assembling oligo for CpG-Free Pac
<400> 102
   tcaccagagt gggactggac attggaaaag tttgggttgc agatgatgga gctgctgttg 60
<210> 103
   <211> 60
   <212> DNA
   <213> Artificial Sequence
<220>
   <223> assembling oligo for CpG-Free Pac
<400> 103
   cagtttggac aacacctgag tctgttgaag ctggtgctgt ttttgctgaa attggaccaa 60
<210> 104
   <211> 60
   <212> DNA
   <213> Artificial Sequence
<220>
   <223> assembling oligo for CpG-Free Pac
<400> 104
   gaatggctga gctctctgga agcaggctgg cagcacaaca acaaatggaa ggtctgctgg 60
<210> 105
   <211> 60
   <212> DNA
   <213> Artificial Sequence
<220>
   <223> assembling oligo for CpG-Free Pac
<400> 105
   caccacacag gccaaaagag ccagcttggt ttctggcaac tgttggagtg agccctgacc 60
<210> 106
   <211> 60
   <212> DNA
   <213> Artificial Sequence
<220>
   <223> assembling oligo for CpG-Free Pac
<400> 106
   accagggaaa gggtctggga tctgctgttg ttctgcctgg agttgaagct gctgaaaggg 60
<210> 107
   <211> 60
   <212> DNA
   <213> Artificial Sequence
<220>
   <223> assembling oligo for CpG-Free Pac
<400> 107
   ctggagttcc tgcctttctg gaaacttctg ctcccagaaa cctgcctttt tatgaaagac 60
<210> 108
   <211> 60
   <212> DNA
   <213> Artificial sequence
<220>
   <223> assembling oligo for CpG-Free Pac

<400> 108
   tgggattcac tgtgacagct gatgttgagg ttccagaagg cccaagaact tggtgcatga 60
<210> 109
   <211> 60
   <212> DNA
   <213> Artificial Sequence
<220>
   <223> assembling oligo for CpG-Free Pac
<400> 109
   caaggaagcc tggagcttaa acctgagcta gctcgacatg ataagataca ttgatgagtt 60
<210> 110
   <211> 30
   <212> DNA
   <213> Artificial Sequence
<220>
   <223> assembling oligo for CpG-Free Pac
<400> 110
   aactcatcaa tgtatcttat catgtcgagc 30
<210> 111
   <211> 60
   <212> DNA
   <213> Artificial Sequence
<220>
   <223> assembling oligo for CpG-Free Pac
<400> 111
   tagctcaggt ttaagctcca ggcttccttg tcatgcacca agttcttggg ccttctggaa 60
<210> 112
   <211> 60
   <212> DNA
   <213> Artificial Sequence
<220>
   <223> assembling oligo for CpG-Free Pac
<400> 112
   cctcaacatc agctgtcaca gtgaatccca gtctttcata aaaaggcagg tttctgggag 60
<210> 113
   <211> 60
   <212> DNA
   <213> Artificial Sequence
<220>
   <223> assembling oligo for CpG-Free Pac
<400> 113
   cagaagtttc cagaaaggca ggaactccag ccctttcagc agcttcaact ccaggcagaa 60
<210> 114
   <211> 60
   <212> DNA
   <213> Artificial Sequence
<220>
   <223> assembling oligo for CpG-Free Pac
<400> 114
   caacagcaga tcccagaccc tttccctggt ggtcagggct cactccaaca gttgccagaa 60
<210> 115
   <211> 60
   <212> DNA
   <213> Artificial Sequence
<220>
   <223> assembling oligo for CpG-Free Pac
<400> 115
   accaagctgg ctcttttggc ctgtgtggtg ccagcagacc ttccatttgt tgttgtgctg 60
<210> 116
   <211> 60
   <212> DNA
   <213> Artificial Sequence
<220>
   <223> assembling oligo for CpG-Free Pac
<400> 116
   ccagcctgct tccagagagc tcagccattc ttggtccaat ttcagcaaaa acagcaccag 60
<210> 117
   <211> 60
   <212> DNA
   <213> Artificial Sequence
<220>
   <223> assembling oligo for CpG-Free Pac
<400> 117
   cttcaacaga ctcaggtgtt gtccaaactg caacagcagc tccatcatct gcaacccaaa 60
<210> 118
   <211> 60
   <212> DNA
   <213> Artificial Sequence
<220>
   <223> assembling oligo for CpG-Free Pac

<400> 118
   cttttccaat gtccagtccc actctggtga ggaagagttc ttgcagttct gtcaccctct 60
<210> 119
   <211> 60
   <212> DNA
   <213> Artificial Sequence
<220>
   <223> assembling oligo for CpG-Free Pac
<400> 119
   caatgtgcct gtcagggtca actgtgtgcc ttgttgcagg gtagtctgca aaagcagcag 60
<210> 120
   <211> 60
   <212> DNA
   <213> Artificial Sequence
<220>
   <223> assembling oligo for CpG-Free Pac
<400> 120
   ccagtgttct cacagctctt ggaacatcat ctctggttgc cagcctcact gtgggtttgt 60
<210> 121
   <211> 30
   <212> DNA
   <213> Artificial Sequence
<220>
   <223> assembling oligo for CpG-Free Pac
<400> 121
   actcagtcat gatggtcctc ctatagtgag 30
<210> 122
   <211> 40
   <212> DNA
   <213> Artificial Sequence
<220>
   <223> assembling oligo for CpG-free LacZ
<400> 122
   atcactatag gagggccacc atggaccctg ttgtgctgca 40
<210> 123
   <211> 20
   <212> DNA
   <213> Artificial Sequence
<220>
   <223> assembling oligo for CpG-free LacZ
<400> 123
   ggtggccctc ctatagtgat 20
<210> 124
   <211> 40
   <212> DNA
   <213> Artificial Sequence
<220>
   <223> assembling oligo for CpG-free LacZ
<400> 124
   aaggagagac tgggagaacc ctggagtgac ccagctcaac 40
<210> 125
   <211> 40
   <212> DNA
   <213> Artificial Sequence
<220>
   <223> assembling oligo for CpG-free Lacz
<400> 125
   ggttctccca gtctctcctt tgcagcacaa cagggtccat 40
<210> 126
   <211> 40
   <212> DNA
   <213> Artificial Sequence
<220>
   <223> assembling oligo for CpG-free Lacz
<400> 126
   agactggctg cccaccctcc ctttgcctct tggaggaact 40
<210> 127
   <211> 40
   <212> DNA
   <213> Artificial Sequence
<220>
   <223> assembling oligo for CpG-free Lacz
<400> 127
   ggagggtggg cagccagtct gttgagctgg gtcactccag 40
<210> 128
   <211> 40
   <212> DNA
   <213> Artificial Sequence

<220>
   <223> assembling oligo for CpG-free Lacz
<400> 128
   ctgaggaagc caggacagac aggcccagcc agcagctcag 40
<210> 129
   <211> 40
   <212> DNA
   <213> Artificial Sequence
<220>
   <223> assembling oligo for cpG-free Lacz
<400> 129
   gtctgtcctg gcttcctcag agttcctcca agaggcaaag 40
<210> 130
   <211> 40
   <212> DNA
   <213> Artificial Sequence
<220>
   <223> assembling oligo for CpG-free LacZ
<400> 130
   gtctctcaat ggagagtgga ggtttgcctg gttccctgcc 40
<210> 131
   <211> 40
   <212> DNA
   <213> Artificial Sequence
<220>
   <223> assembling oligo for CpG-free LacZ
<400> 131
   tccactctcc attgagagac ctgagctgct ggctgggcct 40
<210> 132
   <211> 40
   <212> DNA
   <213> Artificial Sequence
<220>
   <223> assembling oligo for CpG-free LacZ
<400> 132
   cctgaagctg tgcctgagtc ttggctggag tgtgacctcc 40
<210> 133
   <211> 40
   <212> DNA
   <213> Artificial Sequence
<220>
   <223> assembling oligo for CpG-free LacZ
<400> 133
   gactcaggca cagcttcagg ggcagggaac caggcaaacc 40
<210> 134
   <211> 40
   <212> DNA
   <213> Artificial Sequence
<220>
   <223> assembling oligo for CpG-free LacZ
<400> 134
   cagaggctga cactgttgtg gtgcccagca actggcagat 40
<210> 135
   <211> 40
   <212> DNA
   <213> Artificial Sequence
<220>
   <223> assembling oligo for CpG-free Lacz
<400> 135
   cacaacagtg tcagcctctg ggaggtcaca ctccagccaa 40
<210> 136
   <211> 40
   <212> DNA
   <213> Artificial Sequence
<220>
   <223> assembling oligo for CpG-free Lacz
<400> 136
   gcatggctat gatgccccca tctacaccaa tgtcacctac 40
<210> 137
   <211> 40
   <212> DNA
   <213> Artificial Sequence
<220>
   <223> assembling oligo for CpG-free LacZ
<400> 137
   tgggggcatc atagccatgc atctgccagt tgctgggcac 40
<210> 138
   <211> 40
   <212> DNA
   <213> Artificial Sequence
<220>
   <223> assembling oligo for CpG-free LacZ
<400> 138
   cccatcactg tgaacccccc ttttgtgccc actgagaacc 40
<210> 139
   <211> 40
   <212> DNA
   <213> Artificial Sequence
<220>
   <223> assembling oligo for CpG-free Lacz
<400> 139
   ggggggttca cagtgatggg gtaggtgaca ttggtgtaga 40
<210> 140
   <211> 40
   <212> DNA
   <213> Artificial Sequence
<220>
   <223> assembling oligo for CpG-free Lacz
<400> 140
   ccactggctg ctacagcctg accttcaatg ttgatgagag 40

<210> 141
   <211> 40
   <212> DNA
   <213> Artificial Sequence
<220>
   <223> assembling oligo for cpG-free Lacz
<400> 141
   caggctgtag cagccagtgg ggttctcagt gggcacaaaa 40
<210> 142
   <211> 40
   <212> DNA
   <213> Artificial Sequence
<220>
   <223> assembling oligo for CpG-free LacZ
<400> 142
   ctggctgcaa gaaggccaga ccaggatcat ctttgatgga 40
<210> 143
   <211> 40
   <212> DNA
   <213> Artificial Sequence
<220>
   <223> assembling oligo for CpG-free Lacz
<400> 143
   tctggccttc ttgcagccag ctctcatcaa cattgaaggt 40
<210> 144
   <211> 40
   <212> DNA
   <213> Artificial Sequence
<220>
   <223> assembling oligo for CpG-free LacZ
<400> 144
   gtcaactctg ccttccacct ctggtgcaat ggcaggtggg 40
<210> 145
   <211> 40
   <212> DNA
   <213> Artificial Sequence
<220>
   <223> assembling oligo for CpG-free LacZ
<400> 145
   aggtggaagg cagagttgac tccatcaaag atgatcctgg 40
<210> 146
   <211> 40
   <212> DNA
   <213> Artificial Sequence
<220>
   <223> assembling oligo for CpG-free Lacz
<400> 146
   ttggctatgg ccaagacagc aggctgccct ctgagtttga 40
<210> 147
   <211> 40
   <212> DNA
   <213> Artificial Sequence
<220>
   <223> assembling oligo for CpG-free Lacz
<400> 147
   gctgtcttgg ccatagccaa cccacctgcc attgcaccag 40
<210> 148
   <211> 40
   <212> DNA
   <213> Artificial Sequence
<220>
   <223> assembling oligo for CpG-free Lacz
<400> 148
   cctctctgcc ttcctcagag ctggagagaa caggctggct 40
<210> 149
   <211> 40
   <212> DNA
   <213> Artificial Sequence
<220>
   <223> assembling oligo for CpG-free Lacz
<400> 149
   ctctgaggaa ggcagagagg tcaaactcag agggcagcct 40
<210> 150
   <211> 40
   <212> DNA
   <213> Artificial Sequence
<220>
   <223> assembling oligo for CpG-free Lacz
<400> 150
   gtcatggtgc tcaggtggtc tgatggcagc tacctggaag 40
<210> 151
   <211> 40
   <212> DNA
   <213> Artificial Sequence
<220>
   <223> assembling oligo for CpG-free Lacz
<400> 151
   gaccacctga gcaccatgac agccagcctg ttctctccag 40
<210> 152
   <211> 40
   <212> DNA
   <213> Artificial Sequence
<220>
   <223> assembling oligo for CpG-free LaCz
<400> 152
   accaagacat gtggaggatg tctggcatct tcagggatgt 40

<210> 153
   <211> 40
   <212> DNA
   <213> Artificial Sequence
<220>
   <223> assembling oligo for CpG-free Lacz
<400> 153
   catcctccac atgtcttggt cttccaggta gctgccatca 40
<210> 154
   <211> 40
   <212> DNA
   <213> Artificial Sequence
<220>
   <223> assembling oligo for CpG-free LacZ
<400> 154
   gagcctgctg cacaagccca ccacccagat ttctgacttc 40
<210> 155
   <211> 40
   <212> DNA
   <213> Artificial Sequence
<220>
   <223> assembling oligo for CpG-free Lacz
<400> 155
   tgggcttgtg cagcaggctc acatccctga agatgccaga 40
<210> 156
   <211> 40
   <212> DNA
   <213> Artificial Sequence
<220>
   <223> assembling oligo for CpG-free LacZ
<400> 156
   catgttgcca ccaggttcaa tgatgacttc agcagagctg 40
<210> 157
   <211> 40
   <212> DNA
   <213> Artificial Sequence
<220>
   <223> assembling oligo for CpG-free Lacz
<400> 157
   ttgaacctgg tggcaacatg gaagtcagaa atctgggtgg 40
<210> 158
   <211> 40
   <212> DNA
   <213> Artificial Sequence
<220>
   <223> assembling oligo for CpG-free Lacz
<400> 158
   tgctggaggc tgaggtgcag atgtgtggag aactcagaga 40
<210> 159
   <211> 40
   <212> DNA
   <213> Artificial Sequence
<220>
   <223> assembling oligo for CpG-free LacZ
<400> 159
   ctgcacctca gcctccagca cagctctgct gaagtcatca 40
<210> 160
   <211> 40
   <212> DNA
   <213> Artificial Sequence
<220>
   <223> assembling oligo for CpG-free Lacz
<400> 160
   ctacctgaga gtcacagtga gcctctggca aggtgagacc 40
<210> 161
   <211> 40
   <212> DNA
   <213> Artificial Sequence
<220>
   <223> assembling oligo for CpG-free LacZ
<400> 161
   tcactgtgac tctcaggtag tctctgagtt ctccacacat 40
<210> 162
   <211> 40
   <212> DNA
   <213> Artificial Sequence
<220>
   <223> assembling oligo for CpG-free Lacz
<400> 162
   caggtggcct ctggcacagc cccctttgga ggagagatca 40
<210> 163
   <211> 40
   <212> DNA
   <213> Artificial Sequence
<220>
   <223> assembling oligo for cpG-free Lacz

<400> 163
   gctgtgccag aggccacctg ggtctcacct tgccagaggc 40
<210> 164
   <211> 40
   <212> DNA
   <213> Artificial Sequence
<220>
   <223> assembling oligo for cpG-free Lacz
<400> 164
   ttgatgagag aggaggctat gctgacagag tcaccctgag 40
<210> 165
   <211> 40
   <212> DNA
   <213> Artificial Sequence
<220>
   <223> assembling oligo for CpG-free LacZ
<400> 165
   atagcctcct ctctcatcaa tgatctctcc tccaaagggg 40
<210> 166
   <211> 40
   <212> DNA
   <213> Artificial Sequence
<220>
   <223> assembling oligo for CpG-free Lacz
<400> 166
   gctcaatgtg gagaacccca agctgtggtc tgctgagatc 40
<210> 167
   <211> 40
   <212> DNA
   <213> Artificial sequence
<220>
   <223> assembling oligo for cpG-free LacZ
<400> 167
   tggggttctc cacattgagc ctcagggtga ctctgtcagc 40
<210> 168
   <211> 40
   <212> DNA
   <213> Artificial Sequence
<220>
   <223> assembling oligo for CpG-free Lacz
<400> 168
   cccaacctct acagggctgt tgtggagctg cacactgctg 40
<210> 169
   <211> 40
   <212> DNA
   <213> Artificial Sequence
<220>
   <223> assembling oligo for CpG-free Lacz
<400> 169
   acagccctgt agaggttggg gatctcagca gaccacagct 40
<210> 170
   <211> 40
   <212> DNA
   <213> Artificial Sequence
<220>
   <223> assembling oligo for CpG-free LacZ
<400> 170
   atggcaccct gattgaagct gaagcctgtg atgttggatt 40
<210> 171
   <211> 40
   <212> DNA
   <213> Artificial Sequence
<220>
   <223> assembling oligo for cpG-free Lacz
<400> 171
   agcttcaatc agggtgccat cagcagtgtg cagctccaca 40
<210> 172
   <211> 40
   <212> DNA
   <213> Artificial Sequence
<220>
   <223> assembling oligo for CpG-free Lacz
<400> 172
   cagagaagtc aggattgaga atggcctgct gctgctcaat 40
<210> 173
   <211> 40
   <212> DNA
   <213> Artificial Sequence
<220>
   <223> assembling oligo for cpG-free Lacz
<400> 173
   tctcaatcct gacttctctg aatccaacat cacaggcttc 40
<210> 174
   <211> 40
   <212> DNA
   <213> Artificial Sequence
<220>
   <223> assembling oligo for CpG-free Lacz
<400> 174
   ggcaagcctc tgctcatcag gggagtcaac aggcatgagc 40
<210> 175
   <211> 40
   <212> DNA
   <213> Artificial Sequence
<220>
   <223> assembling oligo for CpG-free LacZ
<400> 175
   ctgatgagca gaggcttgcc attgagcagc agcaggccat 40
<210> 176
   <211> 40
   <212> DNA
   <213> Artificial Sequence
<220>
   <223> assembling oligo for CpG-free LacZ
<400> 176
   accaccctct gcatggacaa gtgatggatg aacagacaat 40
<210> 177
   <211> 40
   <212> DNA
   <213> Artificial Sequence
<220>
   <223> assembling oligo for CpG-free Lacz
<400> 177
   ttgtccatgc agagggtggt gctcatgcct gttgactccc 40
<210> 178
   <211> 40
   <212> DNA
   <213> Artificial Sequence
<220>
   <223> assembling oligo for cpG-free Lacz
<400> 178
   ggtgcaagat atcctgctga tgaagcagaa ctccgcctac 40
<210> 179
   <211> 40
   <212> DNA
   <213> Artificial Sequence
<220>
   <223> assembling oligo for CpG-free LacZ
<400> 179
   tcagcaggat atcttgcacc attgtctgtt catccatcac 40
<210> 180
   <211> 20
   <212> DNA
   <213> Artificial Sequence
<220>
   <223> assembling oligo for CpG-free Lacz
<400> 180
   gtaggcggag ttctgcttca 20

<210> 181
   <211> 20
   <212> DNA
   <213> Artificial Sequence
<220>
   <223> assembling oligo for CpG-free Lacz
<400> 181
   tcattagcag gatatcttgc 20
<210> 182
   <211> 40
   <212> DNA
   <213> Artificial Sequence
<220>
   <223> assembling oligo for CpG-free LacZ
<400> 182
   gcaagatatc ctgctaatga agcagaacaa cttcaatgct 40
<210> 183
   <211> 40
   <212> DNA
   <213> Artificial Sequence
<220>
   <223> assembling oligo for CpG-free LacZ
<400> 183
   gggtagtgag agcacctgac agcattgaag ttgttctgct 40
<210> 184
   <211> 40
   <212> DNA
   <213> Artificial Sequence
<220>
   <223> assembling oligo for CpG-free Lacz
<400> 184
   gtcaggtgct ctcactaccc caaccaccct ctctggtaca 40
<210> 185
   <211> 40
   <212> DNA
   <213> Artificial Sequence
<220>
   <223> assembling oligo for CpG-free Lacz
<400> 185
   gccatacctg tcacacaggg tgtaccagag agggtggttg 40
<210> 186
   <211> 40
   <212> DNA
   <213> Artificial Sequence
<220>
   <223> assembling oligo for CpG-free Lacz
<400> 186
   ccctgtgtga caggtatggc ctgtatgttg ttgatgaagc 40
<210> 187
   <211> 40
   <212> DNA
   <213> Artificial Sequence
<220>
   <223> assembling oligo for CpG-free LacZ
<400> 187
   tgccatgtgt ctcaatgttg gcttcatcaa caacatacag 40
<210> 188
   <211> 40
   <212> DNA
   <213> Artificial Sequence
<220>
   <223> assembling oligo for CpG-free LacZ
<400> 188
   caacattgag acacatggca tggtgcccat gaacaggctc 40
<210> 189
   <211> 40
   <212> DNA
   <213> Artificial Sequence
<220>
   <223> assembling oligo for CpG-free Lacz
<400> 189
   agccacctgg ggtcatctgt gagcctgttc atgggcacca 40
<210> 190
   <211> 40
   <212> DNA
   <213> Artificial Sequence
<220>
   <223> assembling oligo for CpG-free Lacz
<400> 190
   acagatgacc ccaggtggct gcctgccatg tctgagagag 40
<210> 191
   <211> 40
   <212> DNA
   <213> Artificial Sequence
<220>
   <223> assembling oligo for CpG-free Lacz
<400> 191
   tctctgcacc atcctggtca ctctctcaga catggcaggc 40
<210> 192
   <211> 40
   <212> DNA
   <213> Artificial Sequence
<220>
   <223> assembling oligo for CpG-free Lacz
<400> 192
   tgaccaggat ggtgcagaga gacaggaacc acccctctgt 40
<210> 193
   <211> 40
   <212> DNA
   <213> Artificial Sequence
<220>
   <223> assembling oligo for CpG-free Lacz
<400> 193
   tgcccagaga ccagatgatc acagaggggt ggttcctgtc 40
<210> 194
   <211> 40
   <212> DNA
   <213> Artificial Sequence
<220>
   <223> assembling oligo for CpG-free LacZ
<400> 194
   gatcatctgg tctctgggca atgagtctgg acatggagcc 40
<210> 195
   <211> 40
   <212> DNA
   <213> Artificial Sequence
<220>
   <223> assembling oligo for CpG-free LacZ
<400> 195
   ctgtagagag catcatggtt ggctccatgt ccagactcat 40
<210> 196
   <211> 40
   <212> DNA
   <213> Artificial Sequence
<220>
   <223> assembling oligo for CpG-free Lacz
<400> 196
   aaccatgatg ctctctacag gtggatcaag tctgttgacc 40
<210> 197
   <211> 40
   <212> DNA
   <213> Artificial Sequence
<220>
   <223> assembling oligo for CpG-free LacZ
<400> 197
   atactgcaca ggtctgctgg ggtcaacaga cttgatccac 40
<210> 198
   <211> 40
   <212> DNA
   <213> Artificial Sequence
<220>
   <223> assembling oligo for CpG-free LacZ
<400> 198
   ccagcagacc tgtgcagtat gaaggaggtg gagcagacac 40
<210> 199
   <211> 40
   <212> DNA
   <213> Artificial Sequence
<220>
   <223> assembling oligo for CpG-free LacZ
<400> 199
   agatgatgtc tgtggctgtg gtgtctgctc cacctccttc 40
<210> 200
   <211> 40
   <212> DNA
   <213> Artificial Sequence
<220>
   <223> assembling oligo for CpG-free Lacz
<400> 200
   cacagccaca gacatcatct gccccatgta tgccagggtt 40
<210> 201
   <211> 40
   <212> DNA
   <213> Artificial Sequence
<220>
   <223> assembling oligo for CpG-free Lacz
<400> 201
   gggaagggct ggtcctcatc aaccctggca tacatggggc 40
<210> 202
   <211> 40
   <212> DNA
   <213> Artificial Sequence
<220>
   <223> assembling oligo for CpG-free Lacz
<400> 202
   gatgaggacc agcccttccc tgctgtgccc aagtggagca 40
<210> 203
   <211> 40
   <212> DNA
   <213> Artificial Sequence
<220>
   <223> assembling oligo for CpG-free LacZ
<400> 203
   cagagagagc cacttcttga tgctccactt gggcacagca 40
<210> 204
   <211> 40
   <212> DNA
   <213> Artificial Sequence
<220>
   <223> assembling oligo for CpG-free LacZ
<400> 204
   tcaagaagtg gctctctctg cctggagaga ccagacctct 40
<210> 205
   <211> 40
   <212> DNA
   <213> Artificial Sequence
<220>
   <223> assembling oligo for CpG-free LacZ
<400> 205
   gtgcatattc acacaggatc agaggtctgg tctctccagg 40
<210> 206
   <211> 40
   <212> DNA
   <213> Artificial Sequence
<220>
   <223> assembling oligo for CpG-free Lacz
<400> 206
   gatcctgtgt gaatatgcac atgcaatggg caactctctg 40
<210> 207
   <211> 40
   <212> DNA
   <213> Artificial Sequence
<220>
   <223> assembling oligo for CpG-free LacZ
<400> 207
   cagtacttgg caaagcctcc cagagagttg cccattgcat 40
<210> 208
   <211> 40
   <212> DNA
   <213> Artificial Sequence
<220>
   <223> assembling oligo for CpG-free Lacz
<400> 208
   ggaggctttg ccaagtactg gcaagccttc agacagtacc 40
<210> 209
   <211> 40
   <212> DNA
   <213> Artificial Sequence
<220>
   <223> assembling oligo for cpG-free Lacz
<400> 209
   aaatcctcct tgcagcctgg ggtactgtct gaaggcttgc 40
<210> 210
   <211> 40
   <212> DNA
   <213> Artificial Sequence
<220>
   <223> assembling oligo for cpG-free Lacz
<400> 210
   ccaggctgca aggaggattt gtgtgggact gggtggacca 40
<210> 211
   <211> 40
   <212> DNA
   <213> Artificial Sequence
<220>
   <223> assembling oligo for CpG-free LacZ
<400> 211
   catcatactt gatgagagat tggtccaccc agtcccacac 40

<210> 212
   <211> 40
   <212> DNA
   <213> Artificial Sequence
<220>
   <223> assembling oligo for CpG-free Lacz
<400> 212
   atctctcatc aagtatgatg agaatggcaa cccctggtct 40
<210> 213
   <211> 40
   <212> DNA
   <213> Artificial Sequence
<220>
   <223> assembling oligo for CpG-free Lacz
<400> 213
   ccaaagtctc ctccataggc agaccagggg ttgccattct 40
<210> 214
   <211> 40
   <212> DNA
   <213> Artificial Sequence
<220>
   <223> assembling oligo for CpG-free Lacz
<400> 214
   gcctatggag gagactttgg tgacaccccc aatgacaggc 40
<210> 215
   <211> 40
   <212> DNA
   <213> Artificial Sequence
<220>
   <223> assembling oligo for CpG-free LacZ
<400> 215
   caggccattc atgcagaact gcctgtcatt gggggtgtca 40
<210> 216
   <211> 40
   <212> DNA
   <213> Artificial sequence
<220>
   <223> assembling oligo for CpG-free Lacz
<400> 216
   agttctgcat gaatggcctg gtctttgcag acaggacccc 40
<210> 217
   <211> 40
   <212> DNA
   <213> Artificial Sequence
<220>
   <223> assembling oligo for CpG-free Lacz
<400> 217
   cctctgtgag ggcagggtga ggggtcctgt ctgcaaagac 40
<210> 218
   <211> 40
   <212> DNA
   <213> Artificial Sequence
<220>
   <223> assembling oligo for CpG-free Lacz
<400> 218
   tcaccctgcc ctcacagagg ccaagcacca gcaacagttc 40
<210> 219
   <211> 40
   <212> DNA
   <213> Artificial Sequence
<220>
   <223> assembling oligo for CpG-free LacZ
<400> 219
   ccagacagcc tgaactggaa gaactgttgc tggtgcttgg 40
<210> 220
   <211> 40
   <212> DNA
   <213> Artificial Sequence
<220>
   <223> assembling oligo for CpG-free Lacz
<400> 220
   ttccagttca ggctgtctgg acagaccatt gaggtgacat 40
<210> 221
   <211> 40
   <212> DNA
   <213> Artificial Sequence
<220>
   <223> assembling oligo for CpG-free LacZ
<400> 221
   gtgcctgaag aggtactcag atgtcacctc aatggtctgt 40
<210> 222
   <211> 40
   <212> DNA
   <213> Artificial Sequence
<220>
   <223> assembling oligo for CpG-free Lacz
<400> 222
   ctgagtacct cttcaggcac tctgacaatg agctcctgca 40
<210> 223
   <211> 20
   <212> DNA
   <213> Artificial Sequence
<220>
   <223> assembling oligo for CpG-free Lacz
<400> 223
   tgcaggagct cattgtcaga 20
<210> 224
   <211> 40
   <212> DNA
   <213> Artificial Sequence
<220>
   <223> assembling oligo for CpG-free Lacz
<400> 224
   gtaatttaac aatgagctcc tgcactggat ggtggccctg 40
<210> 225
   <211> 20
   <212> DNA
   <213> Artificial Sequence
<220>
   <223> assembling oligo for CpG-free LacZ
<400> 225
   ggagctcatt gttaaattac 20
<210> 226
   <211> 40
   <212> DNA
   <213> Artificial Sequence
<220>
   <223> assembling oligo for CpG-free LacZ
<400> 226
   gatggcaagc ctctggcttc tggtgaggtg cctctggatg 40
<210> 227
   <211> 40
   <212> DNA
   <213> Artificial Sequence
<220>
   <223> assembling oligo for cpG-free Lacz
<400> 227
   gaagccagag gcttgccatc cagggccacc atccagtgca 40
<210> 228
   <211> 40
   <212> DNA
   <213> Artificial Sequence
<220>
   <223> assembling oligo for cpG-free Lacz
<400> 228
   tggcccctca aggaaagcag ctgattgaac tgcctgagct 40
<210> 229
   <211> 40
   <212> DNA
   <213> Artificial Sequence
<220>
   <223> assembling oligo for CpG-free Lacz
<400> 229
   ctgctttcct tgaggggcca catccagagg cacctcacca 40
<210> 230
   <211> 40
   <212> DNA
   <213> Artificial Sequence
<220>
   <223> assembling oligo for CpG-free Lacz
<400> 230
   gcctcagcca gagtctgctg gacaactgtg gctaacagtg 40
<210> 231
   <211> 40
   <212> DNA
   <213> Artificial Sequence
<220>
   <223> assembling oligo for cpG-free LacZ
<400> 231
   cagcagactc tggctgaggc agctcaggca gttcaatcag 40
<210> 232
   <211> 40
   <212> DNA
   <213> Artificial Sequence
<220>
   <223> assembling oligo for CpG-free LacZ
<400> 232
   agggtggttc agcccaatgc aacagcttgg tctgaggcag 40
<210> 233
   <211> 40
   <212> DNA
   <213> Artificial Sequence
<220>
   <223> assembling oligo for cpG-free Lacz
<400> 233
   gcattgggct gaaccaccct cactgttagc cacagttgtc 40
<210> 234
   <211> 40
   <212> DNA
   <213> Artificial Sequence
<220>
   <223> assembling oligo for CpG-free Lacz
<400> 234
   gccacatctc tgcatggcag cagtggaggc tggctgagaa 40
<210> 235
   <211> 40
   <212> DNA
   <213> Artificial Sequence
<220>
   <223> assembling oligo for CpG-free Lacz
<400> 235
   ctgccatgca gagatgtggc ctgcctcaga ccaagctgtt 40
<210> 236
   <211> 40
   <212> DNA
   <213> Artificial Sequence
<220>
   <223> assembling oligo for CpG-free Lacz
<400> 236
   cctctctgtg accctgcctg ctgcctctca tgccatccct 40
<210> 237
   <211> 40
   <212> DNA
   <213> Artificial Sequence
<220>
   <223> assembling oligo for cpG-free Lacz
<400> 237
   caggcagggt cacagagagg ttctcagcca gcctccactg 40
<210> 238
   <211> 40
   <212> DNA
   <213> Artificial Sequence
<220>
   <223> assembling oligo for CpG-free Lacz
<400> 238
   cacctgacaa catctgaaat ggacttctgc attgagctgg 40
<210> 239
   <211> 40
   <212> DNA
   <213> Artificial Sequence
<220>
   <223> assembling oligo for cpG-free Lacz
<400> 239
   atttcagatg ttgtcaggtg agggatggca tgagaggcag 40

<210> 240
   <211> 40
   <212> DNA
   <213> Artificial Sequence
<220>
   <223> assembling oligo for CpG-free Lacz
<400> 240
   gcaacaagag atggcagttc aacaggcagt ctggcttcct 40
<210> 241
   <211> 40
   <212> DNA
   <213> Artificial Sequence
<220>
   <223> assembling oligo for CpG-free Lacz
<400> 241
   gaactgccat ctcttgttgc ccagctcaat gcagaagtcc 40
<210> 242
   <211> 40
   <212> DNA
   <213> Artificial Sequence
<220>
   <223> assembling oligo for CpG-free LacZ
<400> 242
   gtctcagatg tggattggag acaagaagca gctcctcacc 40
<210> 243
   <211> 40
   <212> DNA
   <213> Artificial Sequence
<220>
   <223> assembling oligo for CpG-free LacZ
<400> 243
   ctccaatcca catctgagac aggaagccag actgcctgtt 40
<210> 244
   <211> 40
   <212> DNA
   <213> Artificial Sequence
<220>
   <223> assembling oligo for CpG-free Lacz
<400> 244
   cctctcaggg accaattcac cagggctcct ctggacaatg 40
<210> 245
   <211> 40
   <212> DNA
   <213> Artificial Sequence
<220>
   <223> assembling oligo for CpG-free Lacz
<400> 245
   gtgaattggt ccctgagagg ggtgaggagc tgcttcttgt 40
<210> 246
   <211> 40
   <212> DNA
   <213> Artificial Sequence
<220>
   <223> assembling oligo for cpG-free Lacz
<400> 246
   acattggagt gtctgaggcc accaggattg acccaaatgc 40
<210> 247
   <211> 40
   <212> DNA
   <213> Artificial Sequence
<220>
   <223> assembling oligo for cpG-free LacZ
<400> 247
   ggcctcagac actccaatgt cattgtccag aggagccctg 40
<210> 248
   <211> 40
   <212> DNA
   <213> Artificial Sequence
<220>
   <223> assembling oligo for CpG-free Lacz
<400> 248
   ttgggtggag aggtggaagg ctgctggaca ctaccaggct 40
<210> 249
   <211> 40
   <212> DNA
   <213> Artificial Sequence
<220>
   <223> assembling oligo for CpG-free LacZ
<400> 249
   ccttccacct ctccacccaa gcatttgggt caatcctggt 40
<210> 250
   <211> 40
   <212> DNA
   <213> Artificial Sequence
<220>
   <223> assembling oligo for CpG-free Lacz
<400> 250
   gaggctgccc tgctccagtg cacagcagac accctggctg 40
<210> 251
   <211> 40
   <212> DNA
   <213> Artificial Sequence
<220>
   <223> assembling oligo for CpG-free LacZ
<400> 251
   cactggagca gggcagcctc agcctggtag tgtccagcag 40
<210> 252
   <211> 40
   <212> DNA
   <213> Artificial Sequence
<220>
   <223> assembling oligo for CpG-free Lacz
<400> 252
   atgctgttct gatcaccaca gcccatgctt ggcagcacca 40
<210> 253
   <211> 40
   <212> DNA
   <213> Artificial Sequence
<220>
   <223> assembling oligo for CpG-free Lacz
<400> 253
   tgtggtgatc agaacagcat cagccagggt gtctgctgtg 40
<210> 254
   <211> 40
   <212> DNA
   <213> Artificial Sequence
<220>
   <223> assembling oligo for cpG-free LacZ
<400> 254
   aggcaagacc ctgttcatca gcagaaagac ctacaggatt 40
<210> 255
   <211> 40
   <212> DNA
   <213> Artificial Sequence
<220>
   <223> assembling oligo for CpG-free LacZ
<400> 255
   tgatgaacag ggtcttgcct tggtgctgcc aagcatgggc 40
<210> 256
   <211> 40
   <212> DNA
   <213> Artificial Sequence
<220>
   <223> assembling oligo for cpG-free Lacz
<400> 256
   gatggctctg gacagatggc aatcacagtg gatgtggagg 40
<210> 257
   <211> 40
   <212> DNA
   <213> Artificial Sequence
<220>
   <223> assembling oligo for CpG-free Lacz
<400> 257
   gccatctgtc cagagccatc aatcctgtag gtctttctgc 40
<210> 258
   <211> 40
   <212> DNA
   <213> Artificial Sequence
<220>
   <223> assembling oligo for CpG-free Lacz
<400> 258
   ttgcctctga cacacctcac cctgcaagga ttggcctgaa 40
<210> 259
   <211> 40
   <212> DNA
   <213> Artificial Sequence
<220>
   <223> assembling oligo for CpG-free Lacz
<400> 259
   gtgaggtgtg tcagaggcaa cctccacatc cactgtgatt 40
<210> 260
   <211> 40
   <212> DNA
   <213> Artificial Sequence
<220>
   <223> assembling oligo for CpG-free Lacz
<400> 260
   ctgtcaactg gcacaggtgg ctgagagggt gaactggctg 40
<210> 261
   <211> 40
   <212> DNA
   <213> Artificial Sequence
<220>
   <223> assembling oligo for CpG-free Lacz
<400> 261
   ccacctgtgc cagttgacag ttcaggccaa tccttgcagg 40
<210> 262
   <211> 40
   <212> DNA
   <213> Artificial Sequence
<220>
   <223> assembling oligo for CpG-free LacZ
<400> 262
   ggcttaggcc ctcaggagaa ctaccctgac aggctgacag 40
<210> 263
   <211> 40
   <212> DNA
   <213> Artificial Sequence
<220>
   <223> assembling oligo for CpG-free LacZ
<400> 263
   ttctcctgag ggcctaagcc cagccagttc accctctcag 40
<210> 264
   <211> 40
   <212> DNA
   <213> Artificial Sequence
<220>
   <223> assembling oligo for CpG-free Lacz
<400> 264
   ctgcctgctt tgacaggtgg gacctgcctc tgtctgacat 40
<210> 265
   <211> 40
   <212> DNA
   <213> Artificial Sequence
<220>
   <223> assembling oligo for cpG-free Lacz
<400> 265
   ccacctgtca aagcaggcag ctgtcagcct gtcagggtag 40
<210> 266
   <211> 40
   <212> DNA
   <213> Artificial Sequence
<220>
   <223> assembling oligo for CpG-free Lacz
<400> 266
   gtacacccct tatgtgttcc cttctgagaa tggcctgagg 40
<210> 267
   <211> 40
   <212> DNA
   <213> Artificial Sequence
<220>
   <223> assembling oligo for CpG-free LacZ
<400> 267
   ggaacacata aggggtgtac atgtcagaca gaggcaggtc 40
<210> 268
   <211> 40
   <212> DNA
   <213> Artificial Sequence
<220>
   <223> assembling oligo for CpG-free Lacz
<400> 268
   tgtggcacca gggagctgaa ctatggtcct caccagtgga 40
<210> 269
   <211> 40
   <212> DNA
   <213> Artificial Sequence
<220>
   <223> assembling oligo for CpG-free LacZ
<400> 269
   ttcagctccc tggtgccaca cctcaggcca ttctcagaag 40
<210> 270
   <211> 40
   <212> DNA
   <213> Artificial Sequence
<220>
   <223> assembling oligo for CpG-free Lacz
<400> 270
   ggggagactt ccagttcaac atctccaggt actctcagca 40
<210> 271
   <211> 40
   <212> DNA
   <213> Artificial Sequence
<220>
   <223> assembling oligo for CpG-free Lacz
<400> 271
   gttgaactgg aagtctcccc tccactggtg aggaccatag 40
<210> 272
   <211> 40
   <212> DNA
   <213> Artificial Sequence
<220>
   <223> assembling oligo for CpG-free Lacz
<400> 272
   acagctcatg gaaacctctc acaggcacct gctccatgca 40
<210> 273
   <211> 40
   <212> DNA
   <213> Artificial Sequence
<220>
   <223> assembling oligo for CpG-free Lacz
<400> 273
   gagaggtttc catgagctgt tgctgagagt acctggagat 40

<210> 274
   <211> 40
   <212> DNA
   <213> Artificial Sequence
<220>
   <223> assembling oligo for cpG-free Lacz
<400> 274
   gaggagggaa cctggctgaa cattgatggc ttccacatgg 40
<210> 275
   <211> 40
   <212> DNA
   <213> Artificial Sequence
<220>
   <223> assembling oligo for CpG-free Lacz
<400> 275
   ttcagccagg ttccctcctc tgcatggagc aggtgcctgt 40
<210> 276
   <211> 40
   <212> DNA
   <213> Artificial Sequence
<220>
   <223> assembling oligo for CpG-free LacZ
<400> 276
   gcattggagg agatgactct tggtctcctt ctgtgtctgc 40
<210> 277
   <211> 40
   <212> DNA
   <213> Artificial Sequence
<220>
   <223> assembling oligo for CpG-free Lacz
<400> 277
   agagtcatct cctccaatgc ccatgtggaa gccatcaatg 40
<210> 278
   <211> 40
   <212> DNA
   <213> Artificial Sequence
<220>
   <223> assembling oligo for CpG-free LacZ
<400> 278
   tgagttccag ttatctgctg gcaggtacca ctatcagctg 40
<210> 279
   <211> 40
   <212> DNA
   <213> Artificial Sequence
<220>
   <223> assembling oligo for cpG-free LacZ
<400> 279
   cagcagataa ctggaactca gcagacacag aaggagacca 40
<210> 280
   <211> 40
   <212> DNA
   <213> Artificial Sequence
<220>
   <223> assembling oligo for cpG-free Lacz
<400> 280
   gtgtggtgcc agaagtaaac ctgagctagc agtccatgat 40
<210> 281
   <211> 40
   <212> DNA
   <213> Artificial Sequence
<220>
   <223> assembling oligo for CpG-free Lacz
<400> 281
   gtttacttct ggcaccacac cagctgatag tggtacctgc 40
<210> 282
   <211> 20
   <212> DNA
   <213> Artificial Sequence
<220>
   <223> assembling oligo for CpG-free Lacz
<400> 282
   atcatggact gctagctcag 20
<210> 283
   <211> 40
   <212> DNA
   <213> Artificial Sequence
<220>
   <223> assembling oligo for R6K gamma M2A replication origin
<400> 283
   gcaggactga ggcttaatta aaccttaaaa cctttaaaag 40
<210> 284
   <211> 40
   <212> DNA
   <213> Artificial Sequence
<220>
   <223> assembling oligo for R6K gamma M2A replication origin
<400> 284
   ccttatatat tctttttttt cttataaaac ttaaaacctt 40
<210> 285
   <211> 40
   <212> DNA
   <213> Artificial Sequence
<220>
   <223> assembling oligo for R6K gamma M2A replication origin
<400> 285
   agaggctatt taagttgctg atttatatta attttattgt 40
<210> 286
   <211> 50
   <212> DNA
   <213> Artificial Sequence
<220>
   <223> assembling oligo for R6K gamma M2A replication origin
<400> 286
   tcaaacatga gagcttagta catgaaacat gagagcttag tacattagcc 50
<210> 287
   <211> 60
   <212> DNA
   <213> Artificial Sequence
<220>
   <223> assembling oligo for R6K gamma M2A replication origin
<400> 287
   atgagagctt agtacattag ccatgagggt ttagttcatt aaacatgaga gcttagtaca 60
<210> 288
   <211> 60
   <212> DNA
   <213> Artificial Sequence
<220>
   <223> assembling oligo for R6K gamma M2A replication origin
<400> 288
   ttaaacatga gagcttagta catgaaacat gagagcttag tacatactat caacaggttg 60
<210> 289
   <211> 30
   <212> DNA
   <213> Artificial Sequence
<220>
   <223> assembling oligo for R6K gamma M2A replication origin
<400> 289
   aactgctgat cttaattaac ctggagactt 30
<210> 290
   <211> 60
   <212> DNA
   <213> Artificial Sequence
<220>
   <223> assembling oligo for R6K gamma M2A replication origin
<400> 290
   aagtctccag gttaattaag atcagcagtt caacctgttg atagtatgta ctaagctctc 60
<210> 291
   <211> 60
   <212> DNA
   <213> Artificial Sequence
<220>
   <223> assembling oligo for R6K gamma M2A replication origin
<400> 291
   atgtttcatg tactaagctc tcatgtttaa tgtactaagc tctcatgttt aatgaactaa 60
<210> 292
   <211> 60
   <212> DNA
   <213> Artificial Sequence
<220>
   <223> assembling oligo for R6K gamma M2A replication origin
<400> 292
   accctcatgg ctaatgtact aagctctcat ggctaatgta ctaagctctc atgtttcatg 60
<210> 293
   <211> 40
   <212> DNA
   <213> Artificial Sequence
<220>
   <223> assembling oligo for R6K gamma M2A replication origin
<400> 293
   tactaagctc tcatgtttga acaataaaat taatataaat 40
<210> 294
   <211> 40
   <212> DNA
   <213> Artificial Sequence
<220>
   <223> assembling oligo for R6K gamma M2A replication origin
<400> 294
   cagcaactta aatagcctct aaggttttaa gttttataag 40
<210> 295
   <211> 40
   <212> DNA
   <213> Artificial Sequence
<220>
   <223> assembling oligo for R6K gamma M2A repplication origin
<400> 295
   aaaaaaaaga atatataagg cttttaaagg ttttaaggtt 40
<210> 296
   <211> 20
   <212> DNA
   <213> Artificial Sequence
<220>
   <223> assembling oligo for R6K gamma M2A replication origin
<400> 296
   taattaagcc tcagtcctgc 20
<210> 297
   <211> 66
   <212> DNA
   <213> Artificial Sequence
<220>
   <223> EM7 promotor
   <400> 297
   caattaatca tcggcatagt atatcggcat agtataatac gactcactat aggagggcca 60
   ccatgg 66
<210> 298
   <211> 66
   <212> DNA
   <213> Artificial Sequence
<220>
   <223> degenerated oligo for assembling the GpG-Free EM2K promotor
<400> 298
   caattaawca tdggcatagt atatcwgcat agtataatac hactcactat aggagggcca 60
   ccatgg 66
<210> 299
   <211> 20
   <212> DNA
   <213> Artificial Sequence
<220>
   <223> sense oligo for the assembly of SEQ ID No: 38-74 oligos
<400> 299
   ttcagctgag gaggcacatc 20
<210> 300
   <211> 20
   <212> DNA
   <213> Artificial Sequence
<220>
   <223> reverse oligo for the assembly of SEQ ID NO: 38-74 oligos
<400> 300
   ctcaggatcc gctagctaat 20
<210> 301
   <211> 18
   <212> DNA
   <213> Artificial Sequence
<220>
   <223> sense primer pur 24
<400> 301
   aggaccatca tgactgag 18
<210> 302
   <211> 18
   <212> DNA
   <213> Artificial Sequence
<220>
   <223> reverse primer pur 25
<400> 302
   atcatgtcga gctagctc 18
<210> 303
   <211> 31
   <212> DNA
   <213> Artificial Sequence
<220>
   <223> RK15 primer
<400> 303
   gcaggactga ggcttaatta aaccttaaaa c 31
<210> 304
   <211> 31
   <212> DNA
   <213> Artificial Sequence
<220>
   <223> RK16 primer
<400> 304
   aagtctccag gttaattaag atcagcagtt c 31
<210> 305
   <211> 52
   <212> DNA
   <213> Artificial Sequence
<220>
   <223> rps0-1 linker oligo
<400> 305
   ctagctgagt ttcagaaaag ggggcctgag tggccccttt tttcaactta at 52
<210> 306
   <211> 46
   <212> DNA
   <213> Artificial Sequence
<220>
   <223> rps0-2 linker oligo
<400> 306
   taagttgaaa aaaggggcca ctcaggcccc cttttctgaa actcag 46
<210> 307
   <211> 39
   <212> DNA
   <213> Artificial Sequence
<220>
   <223> PACI-UP primer
<400> 307
   atcgttaatt aaaacagtag ttgacaatta aacattggc 39
<210> 308
   <211> 28
   <212> DNA
   <213> Artificial Sequence
<220>
   <223> PACI-DOWN primer
<400> 308
   atcgttaatt aagttgaaaa aaggggcc 28
<210> 309
   <211> 17
   <212> DNA
   <213> Artificial Sequence
<220>
   <223> rbs-1 linker oligo
<400> 309
   aattctgagg agaagct 17
<210> 310
   <211> 17
   <212> DNA
   <213> Artificial Sequence
<220>
   <223> rbs-2 linker oligo
<400> 310
   catgagcttc tcctcag 17
<210> 311
   <211> 36
   <212> DNA
   <213> Artificial Sequence
<220>
   <223> OL dcm AF primer
<400> 311
   ttttgcggcc gcttgctgcg ccagcaacta ataacg 36
<210> 312
   <211> 41
   <212> DNA
   <213> Artificial Sequence
<220>
   <223> OL dcm AR primer
<400> 312
   ccttggatcc tggtaaacac gcactgtccg ccaatcgatt c 41
<210> 313
   <211> 30
   <212> DNA
   <213> Artificial Sequence
<220>
   <223> OL dcm BF primer
<400> 313
   ttttggatcc tcagcaagag gcacaacatg 30
<210> 314
   <211> 35
   <212> DNA
   <213> Artificial sequence
<220>
   <223> OL dcm BR primer
<400> 314
   ttttctcgag aaacggcagc tctgatactt gcttc 35
<210> 315
   <211> 32
   <212> DNA
   <213> Artificial Sequence
<220>
   <223> OL dcm CF primer
<400> 315
   ttttgcggcc gcgttgcggt attacccttg tc 32
<210> 316
   <211> 795
   <212> DNA
   <213> Artificial Sequence
<220>
   <223> Neo DeltaCpG
   <220>
   <221> CDS
   <222> (1)..(795)
   <223> neo gene without CpG
<400> 316
   atg att gaa caa gat ggc cta cat gca ggt tct cca gct gcc tgg gtt      48
   Met Ile Glu Gln Asp Gly Leu His Ala Gly Ser Pro Ala Ala Trp Val
   1               5                   10                  15
   gag aga ctg ttt ggc tat gac tgg gca cag cag acc att ggt tgc tct      96
   Glu Arg Leu Phe Gly Tyr Asp Trp Ala Gln Gln Thr Ile Gly Cys Ser
               20                  25                  30
   gat gca gca gtt ttc aga ctt tca gcc caa ggc agg cca gtc ctt ttt     144
   Asp Ala Ala Val Phe Arg Leu Ser Ala Gln Gly Arg Pro Val Leu Phe
           35                  40                  45
   gta aag aca gac ctc agt ggg gct ctc aat gag ctc cag gat gag gct     192
   Val Lys Thr Asp Leu Ser Gly Ala Leu Asn Glu Leu Gln Asp Glu Ala
       50                  55                  60
   gcc aga ctc tcc tgg ttg gca aca act ggg gtc ccc tgt gca gct gtc     240
   Ala Arg Leu Ser Trp Leu Ala Thr Thr Gly Val Pro Cys Ala Ala Val
   65                  70                  75                  80
   ctt gat gtg gtc aca gaa gct gga agg gac tgg ctc cta cta ggt gag     288
   Leu Asp Val Val Thr Glu Ala Gly Arg Asp Trp Leu Leu Leu Gly Glu
                   85                  90                  95
   gtg cct ggg cag gac ctc ctt tcc tct cac cta gct cca gct gag aaa     336
   Val Pro Gly Gln Asp Leu Leu Ser Ser His Leu Ala Pro Ala Glu Lys
               100                 105                 110
   gtg tca atc atg gct gat gcc atg aga aga ctc cac acc ctt gac cca     384
   Val Ser Ile Met Ala Asp Ala Met Arg Arg Leu His Thr Leu Asp Pro
           115                 120                 125
   gcc acc tgc ccc ttt gac cac cag gcc aag cac agg ata gag agg gcc     432
   Ala Thr Cys Pro Phe Asp His Gln Ala Lys His Arg Ile Glu Arg Ala
       130                 135                 140
   aga acc agg atg gag gct ggc ctg gtg gac caa gat gac ttg gat gaa     480
   Arg Thr Arg Met Glu Ala Gly Leu Val Asp Gln Asp Asp Leu Asp Glu
   145                 150                 155                 160
   gaa cac cag ggc ctg gcc cct gct gaa cta ttt gcc agg ctc aag gca     528
   Glu His Gln Gly Leu Ala Pro Ala Glu Leu Phe Ala Arg Leu Lys Ala
                   165                 170                 175
   tcc atg cca gat ggt gag gac cta gtg gtg act cat ggg gat gcc tgc     576
   Ser Met Pro Asp Gly Glu Asp Leu Val Val Thr His Gly Asp Ala Cys
               180                 185                 190
   ctt ccc aac atc atg gtt gaa aat gga agg ttc tct ggc ttc ata gac     624
   Leu Pro Asn Ile Met Val Glu Asn Gly Arg Phe Ser Gly Phe Ile Asp
           195                 200                 205
   tgt ggc agg ctg gga gtg gct gac agg tac cag gac att gcc cta gca     672
   Cys Gly Arg Leu Gly Val Ala Asp Arg Tyr Gln Asp Ile Ala Leu Ala
       210                 215                 220
   acc agg gac ata gca gaa gag cta ggg gga gag tgg gca gac agg ttc     720
   Thr Arg Asp Ile Ala Glu Glu Leu Gly Gly Glu Trp Ala Asp Arg Phe
   225                 230                 235                 240
   cta gtg ctc tat ggc att gca gcc cct gac tcc cag aga att gcc ttc     768
   Leu Val Leu Tyr Gly Ile Ala Ala Pro Asp Ser Gln Arg Ile Ala Phe
                   245                 250                 255
   tac aga ctt ctt gat gag ttc ttc taa                                 795
   Tyr Arg Leu Leu Asp Glu Phe Phe
               260
<210> 317
   <211> 264
   <212> PRT
   <213> Artificial Sequence
<220>
   <223> Neo DeltaCpG
<400> 317
   Met Ile Glu Gln Asp Gly Leu His Ala Gly Ser Pro Ala Ala Trp Val
   1 5 10 15
   Glu Arg Leu Phe Gly Tyr Asp Trp Ala Gln Gln Thr Ile Gly Cys Ser
   20 25 30
   Asp Ala Ala Val Phe Arg Leu Ser Ala Gln Gly Arg Pro Val Leu Phe
   35 40 45
   Val Lys Thr Asp Leu Ser Gly Ala Leu Asn Glu Leu Gln Asp Glu Ala
   50 55 60
   Ala Arg Leu Ser Trp Leu Ala Thr Thr Gly Val Pro Cys Ala Ala Val
   65 70 75 80
   Leu Asp Val Val Thr Glu Ala Gly Arg Asp Trp Leu Leu Leu Gly Glu
   85 90 95
   Val Pro Gly Gln Asp Leu Leu Ser Ser His Leu Ala Pro Ala Glu Lys
   100 105 110
   Val Ser Ile Met Ala Asp Ala Met Arg Arg Leu His Thr Leu Asp Pro
   115 120 125
   Ala Thr Cys Pro Phe Asp His Gln Ala Lys His Arg Ile Glu Arg Ala
   130 135 140
   Arg Thr Arg Met Glu Ala Gly Leu Val Asp Gln Asp Asp Leu Asp Glu
   145 150 155 160
   Glu His Gln Gly Leu Ala Pro Ala Glu Leu Phe Ala Arg Leu Lys Ala
   165 170 175
   Ser Met Pro Asp Gly Glu Asp Leu Val Val Thr His Gly Asp Ala Cys
   180 185 190
   Leu Pro Asn Ile Met Val Glu Asn Gly Arg Phe Ser Gly Phe Ile Asp
   195 200 205
   Cys Gly Arg Leu Gly Val Ala Asp Arg Tyr Gln Asp Ile Ala Leu Ala
   210 215 220
   Thr Arg Asp Ile Ala Glu Glu Leu Gly Gly Glu Trp Ala Asp Arg Phe
   225 230 235 240
   Leu Val Leu Tyr Gly Ile Ala Ala Pro Asp Ser Gln Arg Ile Ala Phe
   245 250 255
   Tyr Arg Leu Leu Asp Glu Phe Phe
   260

<210> 318
   <211> 40
   <212> DNA
   <213> Artificial Sequence
<220>
   <223> oligo for construction of Neo DeltaCpG
<400> 318
   cattaccggt aggcacatca tgattgaaca agatggccta 40
<210> 319
   <211> 40
   <212> DNA
   <213> Artificial Sequence
<220>
   <223> oligo for construction of Neo DeltaCpG
<400> 319
   catgcaggtt ctccagctgc ctgggttgag agactgtttg 40
<210> 320
   <211> 40
   <212> DNA
   <213> Artificial Sequence
<220>
   <223> oligo for construction of Neo DeltacpG
<400> 320
   gctatgactg ggcacagcag accattggtt gctctgatgc 40
<210> 321
   <211> 40
   <212> DNA
   <213> Artificial Sequence
<220>
   <223> oligo for construction of Neo DeltacpG
<400> 321
   agcagttttc agactttcag cccaaggcag gccagtcctt 40
<210> 322
   <211> 40
   <212> DNA
   <213> Artificial Sequence
<220>
   <223> oligo for construction of Neo DeltaCpG
<400> 322
   tttgtaaaga cagacctcag tggggctctc aatgagctcc 40
<210> 323
   <211> 40
   <212> DNA
   <213> Artificial Sequence
<220>
   <223> oligo for construction of Neo DeltaCpG
<400> 323
   aggatgaggc tgccagactc tcctggttgg caacaactgg 40
<210> 324
   <211> 40
   <212> DNA
   <213> Artificial Sequence
<220>
   <223> oligo for construction of Neo DeltaCpG
<400> 324
   ggtcccctgt gcagctgtcc ttgatgtggt cacagaagct 40
<210> 325
   <211> 40
   <212> DNA
   <213> Artificial Sequence
<220>
   <223> oligo for construction of Neo DeltacpG
<400> 325
   ggaagggact ggctcctact aggtgaggtg cctgggcagg 40
<210> 326
   <211> 40
   <212> DNA
   <213> Artificial Sequence
<220>
   <223> oligo for construction of Neo DeltacpG
<400> 326
   acctcctttc ctctcaccta gctccagctg agaaagtgtc 40
<210> 327
   <211> 40
   <212> DNA
   <213> Artificial Sequence
<220>
   <223> oligo for construction of Neo DeTtacpG
<400> 327
   aatcatggct gatgccatga gaagactcca cacccttgac 40
<210> 328
   <211> 40
   <212> DNA
   <213> Artificial Sequence
<220>
   <223> oligo for construction of Neo DeltaCpG
<400> 328
   ccagccacct gcccctttga ccaccaggcc aagcacagga 40
<210> 329
   <211> 40
   <212> DNA
   <213> Artificial Sequence
<220>
   <223> oligo for construction of Neo DeltaCpG
<400> 329
   tagagagggc cagaaccagg atggaggctg gcctggtgga 40
<210> 330
   <211> 40
   <212> DNA
   <213> Artificial Sequence
<220>
   <223> oligo for construction of Neo DeltacpG
<400> 330
   ccaagatgac ttggatgaag aacaccaggg cctggcccct 40
<210> 331
   <211> 40
   <212> DNA
   <213> Artificial Sequence
<220>
   <223> oligo for construction of Neo DeltaCpG
<400> 331
   gctgaactat ttgccaggct caaggcatcc atgccagatg 40
<210> 332
   <211> 40
   <212> DNA
   <213> Artificial Sequence
<220>
   <223> oligo for construction of Neo DeltaCpG
<400> 332
   gtgaggacct agtggtgact catggggatg cctgccttcc 40
<210> 333
   <211> 40
   <212> DNA
   <213> Artificial Sequence
<220>
   <223> oligo for construction of Neo DeltaCpG
<400> 333
   caacatcatg gttgaaaatg gaaggttctc tggcttcata 40
<210> 334
   <211> 40
   <212> DNA
   <213> Artificial Sequence
<220>
   <223> oligo for construction of Neo DeltacpG
<400> 334
   gactgtggca ggctgggagt ggctgacagg taccaggaca 40
<210> 335
   <211> 40
   <212> DNA
   <213> Artificial Sequence
<220>
   <223> oligo for construction of Neo DeltacpG
<400> 335
   ttgccctagc aaccagggac atagcagaag agctaggggg 40
<210> 336
   <211> 40
   <212> DNA
   <213> Artificial Sequence
<220>
   <223> oligo for construction of Neo DeltaCpG
<400> 336
   agagtgggca gacaggttcc tagtgctcta tggcattgca 40
<210> 337
   <211> 40
   <212> DNA
   <213> Artificial Sequence
<220>
   <223> oligo for construction of Neo DeltaCpG
<400> 337
   gcccctgact cccagagaat tgccttctac agacttcttg 40
<210> 338
   <211> 40
   <212> DNA
   <213> Artificial Sequence
<220>
   <223> oligo for construction of Neo DeltaCpG
<400> 338
   atgagttctt ctaaagctag ctgatcctga tagctgttcg 40
<210> 339
   <211> 20
   <212> DNA
   <213> Artificial Sequence
<220>
   <223> oligo for construction of Neo DeltaCpG
<400> 339
   cgaacagcta tcaggatcag 20
<210> 340
   <211> 40
   <212> DNA
   <213> Artificial Sequence
<220>
   <223> oligo for construction of Neo DeltaCpG
<400> 340
   ctagctttag aagaactcat caagaagtct gtagaaggca 40
<210> 341
   <211> 40
   <212> DNA
   <213> Artificial Sequence
<220>
   <223> oligo for construction of Neo DeltaCpG
<400> 341
   attctctggg agtcaggggc tgcaatgcca tagagcacta 40
<210> 342
   <211> 40
   <212> DNA
   <213> Artificial Sequence
<220>
   <223> oligo for construction of Neo DeltacpG
<400> 342
   ggaacctgtc tgcccactct ccccctagct cttctgctat 40
<210> 343
   <211> 40
   <212> DNA
   <213> Artificial Sequence
<220>
   <223> oligo for construction of Neo DeltaCpG
<400> 343
   gtccctggtt gctagggcaa tgtcctggta cctgtcagcc 40
<210> 344
   <211> 40
   <212> DNA
   <213> Artificial Sequence
<220>
   <223> oligo for construction of Neo DeltacpG
<400> 344
   actcccagcc tgccacagtc tatgaagcca gagaaccttc 40
<210> 345
   <211> 40
   <212> DNA
   <213> Artificial Sequence
<220>
   <223> oligo for construction of Neo DeltacpG
<400> 345
   cattttcaac catgatgttg ggaaggcagg catccccatg 40
<210> 346
   <211> 40
   <212> DNA
   <213> Artificial Sequence
<220>
   <223> oligo for construction of Neo DeltaCpG
<400> 346
   agtcaccact aggtcctcac catctggcat ggatgccttg 40
<210> 347
   <211> 40
   <212> DNA
   <213> Artificial Sequence
<220>
   <223> oligo for construction of Neo DeltaCpG
<400> 347
   agcctggcaa atagttcagc aggggccagg ccctggtgtt 40
<210> 348
   <211> 40
   <212> DNA
   <213> Artificial Sequence
<220>
   <223> oligo for construction of Neo DeltaCpG
<400> 348
   cttcatccaa gtcatcttgg tccaccaggc cagcctccat 40
<210> 349
   <211> 40
   <212> DNA
   <213> Artificial Sequence
<220>
   <223> oligo for construction of Neo DeltacpG
<400> 349
   cctggttctg gccctctcta tcctgtgctt ggcctggtgg 40
<210> 350
   <211> 40
   <212> DNA
   <213> Artificial Sequence
<220>
   <223> oligo for construction of Neo DeltaCpG
<400> 350
   tcaaaggggc aggtggctgg gtcaagggtg tggagtcttc 40
<210> 351
   <211> 40
   <212> DNA
   <213> Artificial Sequence
<220>
   <223> oligo for construction of Neo DeltaCpG
<400> 351
   tcatggcatc agccatgatt gacactttct cagctggagc 40
<210> 352
   <211> 40
   <212> DNA
   <213> Artificial Sequence
<220>
   <223> oligo for construction of Neo DeltaCpG
<400> 352
   taggtgagag gaaaggaggt cctgcccagg cacctcacct 40
<210> 353
   <211> 40
   <212> DNA
   <213> Artificial Sequence
<220>
   <223> oligo for construction of Neo DeltacpG
<400> 353
   agtaggagcc agtcccttcc agcttctgtg accacatcaa 40
<210> 354
   <211> 40
   <212> DNA
   <213> Artificial Sequence
<220>
   <223> oligo for construction of Neo DeltaCpG
<400> 354
   ggacagctgc acaggggacc ccagttgttg ccaaccagga 40
<210> 355
   <211> 40
   <212> DNA
   <213> Artificial Sequence
<220>
   <223> oligo for construction of Neo DeltaCpG
<400> 355
   gagtctggca gcctcatcct ggagctcatt gagagcccca 40
<210> 356
   <211> 40
   <212> DNA
   <213> Artificial Sequence
<220>
   <223> oligo for construction of Neo DeltaCpG
<400> 356
   ctgaggtctg tctttacaaa aaggactggc ctgccttggg 40
<210> 357
   <211> 40
   <212> DNA
   <213> Artificial Sequence
<220>
   <223> oligo for construction of Neo DeltacpG
<400> 357
   ctgaaagtct gaaaactgct gcatcagagc aaccaatggt 40
<210> 358
   <211> 40
   <212> DNA
   <213> Artificial Sequence
<220>
   <223> oligo for construction of Neo DeltaCpG
<400> 358
   ctgctgtgcc cagtcatagc caaacagtct ctcaacccag 40
<210> 359
   <211> 40
   <212> DNA
   <213> Artificial Sequence
<220>
   <223> oligo for construction of Neo DeltaCpG
<400> 359
   gcagctggag aacctgcatg taggccatct tgttcaatca 40
<210> 360
   <211> 20
   <212> DNA
   <213> Artificial Sequence
<220>
   <223> oligo for construction of Neo DeltaCpG
<400> 360
   tgatgtgcct accggtaatg 20